Europäisches Patentamt

European Patent Office

Office européen des brevets

(11) Numéro de publication: **0 377 349 B1**

(12) **FASCICULE DE BREVET EUROPEEN**

(45) Date de publication de fascicule du brevet: **08.06.94**

(51) Int. Cl.5: **C12N 15/47**, A61K 39/205, C12N 1/21, C12Q 1/70, C12P 21/08

(21) Numéro de dépôt: **89402812.5**

(22) Date de dépôt: **11.10.89**

(54) Polypeptides recombinants du virus de la septicemie hemorragique du poisson.

(30) Priorité: **12.10.88 EP 88402587**

(43) Date de publication de la demande: **11.07.90 Bulletin 90/28**

(45) Mention de la délivrance du brevet: **08.06.94 Bulletin 94/23**

(84) Etats contractants désignés: **AT BE CH DE ES FR GB GR IT LI LU NL SE**

(56) Documents cités: **EP-A- 0 051 522**

**ANN. INST. PASTEUR/VIROL., vol. 136E, 1985, pages 13-26, Elsevier, Paris, FR; J. BERNARD et al.: "Viral haemorrhagic septicaemia in rainbow trout: attempt to relate interferon production, antibody synthesis and structure of the virus with the mechanism of virulence"**

**IDEM**

(73) Titulaire: **EUROGENTEC S.A. Campus du Sart-Tilman Allée du Six Août B6 Bldg B-4000 Liège(BE)**

(72) Inventeur: **Renard, André 37 avenue des Cerfs B-4900 Angleur(BE)** Inventeur: **Thiry, Michel Cul du Bief 166 B-4640 Nessonvaux(BE)**

(74) Mandataire: **Desaix, Anne et al Ernest Gutmann - Yves Plasseraud S.A. 3, rue Chauveau-Lagarde F-75008 Paris (FR)**

DISS. ABSTR. INT. B, vol. 48, no. 8, 1988, pages 2207-2208; J.M. FEYEREISEN-KOENER: "Cloning, sequence, and expression of the glycoprotein gene of infectious hematopoietic necrosis virus, a fish rhabdovirus"

INFECTION AND IMMUNITY, vol. 39, no. 1, janvier 1983, pages 7-14, American Society for Microbiology; J. BERNARD et al.: "Viral hemorrhagic septicemia of rainbow trout: relation between the G polypeptide and antibody production in protection of the fish after infection with the F25 attenuated variant"

CHEMICAL ABSTRACTS, vol. 82, no. 25, 23 juin 1975, page 152, résumé no. 166189m, Columbus, Ohio, US; P.E. McALLISTER et al.: "Structural proteins of two salmonid rhabdoviruses"

JOURNAL OF VIROLOGY, vol. 36, no. 3, décembre 1980, pages 652-658; P. DE KINKELIN et al.: "Viral hemorrhagic septicemia of rainbow trout: selection of a thermoresistant virus variant and comparison of polypeptide synthesis with the wild-type virus strain"

CHEMICAL ABSTRACTS, vol. 107, no. 7, 17 août 1987, page 192, résumé no. 53084q, Columbus, Ohio, US; J.F. KOENER et al.: "Nucleotide sequence of a cDNA clone carrying the glycoprotein gene of infectious hematopoietic necrosis virus, a fish rhabdovirus", & J. VIROL. 1987, 61(5), 1342-9

F. FENNER et al.: "Veterinary Virology", pages 546,547, Academic Press, Inc., Orlando, US

## Description

L'invention concerne des polypeptides recombinants immunogènes, dans un état de pureté permettant leur application à la production d'anticorps neutralisants contre le virus de la septicémie hémorragique virale (SHV). L'invention concerne également un procédé de préparation desdits polypeptides. Elle concerne également les acides nucléiques codant pour une partie ou la totalité des séquences d'acides aminés entrant dans la constitution de ces polypeptides. De plus, l'invention se rapporte aux vaccins contenant les susdits polypeptides immunogènes comme principe actif contre la septicémie hémorragique virale des poissons.

Par "polypeptide recombinant", il faut entendre dans ce qui suit toute molécule à ossature polypeptidique susceptible d'être produite par génie génétique, à l'occasion de la transcription et de la traduction d'une séquence d'ADN correspondante sous le contrôle d'éléments de régulation appropriés au sein d'un hôte cellulaire compétent. Par conséquent, l'expression "polypeptide recombinant" telle qu'elle est utilisée dans le contexte de la présente description n'exclut pas la possibilité qu'il porte d'autres groupes, par exemple des groupements glycosylés.

L'adjectif "recombinant" véhicule certes l'idée que le polypeptide en question a été fabriqué par génie génétique, notamment en tant qu'il résulte alors généralement de l'expression dans un hôte cellulaire de la séquence d'acide nucléique correspondante qui avait, auparavant, été introduite dans le vecteur d'expression utilisé dans cet hôte par recombinaison génétique. Néanmoins, il doit être entendu que l'expression n'exclut pas la possibilité que le polypeptide en question soit produit par un procédé différent, par exemple part synthèse chimique selon des méthodes appliquées dans la chimie des protéines.

Par état de "pureté biologique" ou "biologiquement pur" il faut entendre, d'une part un niveau de pureté tel que le polypeptide recombinant immunogène du genre en question puisse être appliqué à la production de compositions vaccinantes, d'autre part, l'absence de contaminants, plus particulièrement de contaminants naturels, issus du virus de la septicémie hémorragique virale.

Le virus de la septicémie hémorragique virale (également désigné par virus d'Egtved) infecte les poissons de la famille des Salmonidés, par exemple truites et saumons. Le virus est un virus à ARN, encapsidé, monosegmenté négatif, appartenant à la famille des Rhabdoviridae.

La septicémie hémorragique virale (également désignée par SHV) est une maladie typiquement européenne mais il existe aux Etats Unis et au Japon une maladie proche, la nécrose hématopoïétique infectieuse (IHN) causée par un virus de la même famille (rhabdoviridae) que celui de la SHV.

Jusqu'à présent, les études réalisées sur le virus d'Egtved (encore désigné dans la suite par l'expression "virus de la SHV") ont permis de mettre en évidence l'existence de plusieurs variants notamment 3, voire 5 types différents. Toutefois, si ces différents variants présentent des distinctions dans leurs propriétés antigéniques ou dans leur caractère pathogène vis-à-vis des poissons de la famille des Salmonidés, ils conservent une parenté importante au niveau de leurs séquences d'acides nucléiques, avec une homologie de séquence de leurs acides nucléiques pouvant atteindre 95% ou plus.

Certaines des propriétés physico-chimiques du virus ainsi que certains éléments liés à sa pathogénicité ont déjà été décrits. On peut citer à titre d'exemple l'article de Lenoir et de Kinkelin dans "Journal of Virology, Aug. 1975 p.259-262" décrivant des essais de fractionnement des protéines du virus de la SHV de la truite. L'article fait référence à l'existence de cinq protéines (L, G, N, M1, M2) parmi lesquelles l'existence d'une glycoprotéine désignée par protéine G. L'article mentionne que cette protéine G, détectée par gel d'électrophorèse pourrait être la composante des protubérances présentes sur la surface du virion.

Dans une autre publication, du "Journal Vet. Med. B, 33, 36-46 (1986)", Deuter et al. rapportent des résultats consécutifs à la détermination par plusieurs laboratoires des poids moléculaires des protéines des virus VHS-V et SVC-V indiqués ci-dessus. Deuter et al. soulignent la divergence des résultats observés et obtiennent quant à eux des poids moléculaires (PM) des protéines L, G, N, $M_1$ et $M_2$, encore différents de tous ceux obtenus par les laboratoires dont question précédemment, et donnent un poids moléculaire de 63000D pour la protéine G.

D'autres résultats sur le virus sont rapportés dans l'article de de Kinkelin publié dans "Antigens of fish pathogens" (Antigènes d'agents pathogènes pour le poisson) Collection Fondation Marcel Mérieux 1982 qui précise que les protéines G, M1 et M2 appartiennent à l'enveloppe du virus, et que la glycoprotéine G du virus est la composante des protubérances et serait porteur et l'antigénicité neutralisante.

Comme le montrent les divergences des résultats connus à ce jour, les caractéristiques physico-chimiques de la protéine G sont imprécises et de plus insuffisantes, pour permettre son identification de façon certaine et non ambiguë, ainsi que la caractérisation de ses éléments structuraux et fonctionnels.

L'article de Bernard et al., publié dans "Infection and Immunity" (39, 7-14) concerne un variant non pathogène du virus de la SHV, désigné par F25 et comparé sur le plan de son antigénicité neutralisante, avec une souche sauvage du virus de la SHV. Cet article indique que l'on n'a pas pu mettre en évidence le polypeptide G du variant F25 sur un gel d'électrophorèse SDS. Cet article mentionne aussi que par électrophorèse sur gel le polypeptide G de la souche sauvage "comigre" avec une autre bande.

De plus, les auteurs font remarquer que dans les expériences de séroneutralisation, chaque virus croise faiblement avec l'antisérum hétérologue, ce qui est en accord avec la différence dans la glycosylation de chacun des deux virus indiqués ci-dessus.

L'article de J. Bernard et al. (Ann. Inst. Pasteur/Virol., 1985, 136 E, p. 13-26) mentionne l'induction d'anticorps présentés comme neutralisants chez le poisson par de fortes doses d'une préparation à base de polypeptide G extrait d'une souche sauvage de SHV à l'aide de 3-octylglucopyranoside.

L'article de Robert D. Gilmore et al. (Biotechnology, vol. 6, March 1988, p. 295-300) fait état de l'induction d'anticorps neutralisants chez le poisson par le produit d'expression d'un fragment recombinant contenant un insérat constitué par un fragment de gène de glycoprotéine du virus IHNV responsable de la nécrose hématopoïétique.

Par ailleurs, jusqu'à ce jour, les vaccins utilisés pour la prévention de la septicémie hémorragique virale des poissons, consistent en des préparations enrichies en virus purifié atténué ou tué, administrables par voie intrapéritonéale. De tels vaccins présentent des inconvénients : en particulier, le vaccin atténué possède une innocuité insuffisante pour les poissons traités, chez lesquels on peut constater un certain niveau de mortalité lors de la vaccination. Il existe une possibilité de contamination de l'eau par les poissons vaccinés, avec les conséquences écologiques inquiétantes que l'on imagine. Le vaccin tué ne présente pas les mêmes inconvénients, mais son prix de revient est élevé et, partant, défavorable à son utilisation à grande échelle, dans les pisciculturas.

Par ailleurs, le mode d'administration des vaccins contre la SHV utilisés à ce jour chez les poissons tels que la truite, est peu pratique. Il s'agit de la voie intrapéritonéale ou de la balnéation. L'administration de vaccins par voie orale s'est montrée insatisfaisante, notamment en raison de l'acidité et des protéases de l'estomac (cf. conclusions générales du symposium sur la vaccination du poisson, Office International des Epizooties, Paris 22 Février 1984).

A ce jour, il est admis dans l'état de la technique que les possibilités de préparer et d'utiliser des vaccins efficaces par voie orale ne constituent que des perspectives hypothétiques très lointaines.

L'invention a pour objet des polypeptides recombinants utilisables comme principes actifs de vaccins biologiquement purs, étant reconnus par des anticorps dirigés contre le virus de la SHV et susceptibles d'induire in vivo la formation d'anticorps neutralisants vis-à-vis du virus de la SHV.

L'invention a également pour objet les acides nucléiques codant pour l'ossature peptidique des polypeptides recombinants biologiquement purs de l'invention permettant leur préparation à grande échelle.

L'invention a également pour but la production de vaccins efficaces contre la SHV, accessibles à faible coût, et, de préférence faciles à administrer. En particulier, l'invention a pour but l'obtention de compositions de vaccins, administrables par balnéation, douche ou par voie orale, notamment avec la nourriture des poissons. Il va sans dire que les autres voies d'administration, notamment par injection, ne sont pas exclues des compositions vaccinantes de l'invention.

Le polypeptide recombinant selon l'invention est caractérisé par la présence dans son ossature polypeptidique d'un enchaînement d'acides aminés, contenu dans la séquence suivante :

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-


Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

Un polypeptide avantageux de l'invention est caractérisé en ce qu'il est reconnu par des anticorps neutralisants formés au préalable contre le virus de la SHV et en outre éventuellement susceptible d'induire à son tour, in vivo, des anticorps neutralisants contre ce même virus.

L'ensemble des propriétés que possèdent les polypeptides de l'invention à savoir l'aptitude à être reconnus par des anticorps neutralisant le virus de la SHV et la capacité à induire in vivo la production d'anticorps neutralisant le virus sera dans ce qui suit, pour la commodité du langage, désigné sous l'expression "propriétés SHV-séroneutralisantes".

Dans la suite, on désigne le polypeptide caractérisé par la totalité de l'enchaînement d'amino-acides indiqué ci-dessus par "polypeptide I".

Un procédé pour la production de ces polypeptides immunogènes comprend :
- la culture d'un hôte cellulaire compétent, auparavant transformé avec un acide nucléique contenant une séquence de nucléotides codant pour le susdit polypeptide immunogène et dans laquelle cette séquence de nucléotides se trouve placée sous le contrôle d'éléments de régulation, dont notamment un promoteur reconnu par les polymérases de cet hôte cellulaire compétent, les éléments d'initiation et de terminaison de la traduction, et
- la récupération du polypeptide immunogène produit à partir des produits d'expression de cet hôte cellulaire.

Des séquences de nucléotides préférées, à mettre en oeuvre dans le procédé selon l'invention seront indiquées plus loin.

Un autre procédé de préparation des polypeptides de l'invention est caractérisé en ce que, partant de préférence de l'amino acide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura en soin de protéger au préalable toutes les fonctions réactives portées par ces aminoacyles ou fragments à l'exception des fonctions amines de l'un et carboxyle de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

Des variantes préférées du procédé de génie génétique qui vient d'être brièvement défini et dont l'originalité réside essentiellement dans la nature de la séquence de nucléotides dont l'expression est recherchée, peuvent être envisagées. Il permet la production d'un polypeptide recombinant "SHV-séroneutralisant" conforme à l'invention.

On conçoit que l'homme de métier dispose de la possibilité d'identifier, voire de sélectionner ceux des polypeptides "SHV-séroneutralisants" de séquences plus courtes qui entrent dans le champ de l'invention. A titre de l'un des moyens généraux lui permettant de procéder à cette identification on mentionnera, par exemple le traitement du polypeptide I avec une protéine spécifique clivant exclusivement le polypeptide I dans un site peptidique choisi soit dans une région N-terminale, soit dans une région C-terminale, suivi de la séparation du fragment N-terminal ou du fragment C-terminal du restant du polypeptide I, ce "restant" étant alors testé pour son activité SHV-séroneutralisante. En cas de réponse positive, l'on aura alors établi que le fragment N-terminal ou C-terminal ne jouait pas un rôle significatif, sinon essentiel pour la manifestation des propriétés SHV-séroneutralisantes du polypeptide I. L'opération peut éventuellement être répétée pour autant que l'on dispose d'une protéase susceptible de reconnaître spécifiquement et exclusivement un autre site spécifique proche d'une extrémité N-terminale ou C-terminale du polypeptide restant. La perte par le polypeptide plus petit reconnu des propriétés SHV-séroneutralisantes du fragment plus long dont il était issu, peut alors conduire à l'hypothèse que le dernier fragment séparé jouait un rôle significatif dans la manifestation des propriétés SHV-séroneutralisantes du polypeptide I et, qu'à ce titre, il appartient normalement, au moins en partie, à la séquence en acides aminés du polypeptide recombinant immunogène conforme à l'invention.

Une autre variante, plus simple que la précédente, de détection des régions du polypeptide I essentielles à la manifestation de leur activité SHV-séroneutralisante, peut être basée sur des traitements enzymatiques d'un acide nucléique codant pour le polypeptide I, avant l'incorporation de l'acide nucléique, encore présumé coder pour un polypeptide SHV-séroneutralisant, dans le vecteur d'expression utilisé pour la mise en oeuvre du procédé sus-indiqué de production d'un polypeptide immunogène recombinant, dans l'hôte cellulaire approprié. Ce traitement enzymatique peut alors consister soit en un émondage des extrémités de l'acide nucléique initial (codant par exemple pour le polypeptide I entier), par exemple par une enzyme exonucléolytique, telle que Bal31, soit par une ou plusieurs enzymes de restriction choisies selon leurs sites respectifs (le cas échéant aménagés par mutagénèse ponctuelle dirigée) dans la séquence de l'acide nucléique initial. L'acide nucléique tronqué obtenu peut alors être testé, après incorporation dans le vecteur choisi et transformation de l'hôte cellulaire avec le vecteur recombinant obtenu, pour sa capacité à exprimer un polypeptide tronqué correspondant, possédant encore les susdites propriétés SHV-séroneutralisantes - ou bien au contraire ne les possédant plus - d'où la possibilité, comme dans la variante précédente, d'identifier au sein du polypeptide I les séquences jouant un rôle important, sinon essentiel dans la manifestation de ses propriétés SHV-séroneutralisantes.

On indique ci-après - de façon bien entendu non limitative - un test de production d'anticorps polyclonaux et la mise en évidence des susdites propriétés SHV-séroneutralisantes des anticorps polyclonaux formés.

Ce test comprend l'immunisation d'un animal, par exemple un lapin, soumis à de multiples injections intradermiques, cinq à dix, de 100µg de virus purifié dans l'adjuvant complet de Freund, le traitement étant répété à deux reprises à un mois d'intervalle. L'apparition des anticorps anti-SHV est recherchée par la méthode ELISA.

Le caractère séroneutralisant des anticorps ainsi obtenus est mis en évidence de la manière suivante. Dans une plaque de culture cellulaire de 96 puits, on dispose dans chaque puits un nombre constant de particules virales (5 $10^5$ ufp/ml) et on ajoute des dilutions croissantes de sérum anti-SHV. Après une nuit à 4°C, on ajoute les cellules sensibles au virus (lignée cellulaire de truite RTG2), à la concentration de 2 $10^4$ cellules par ml et on incube trois jours à 15°C. On mesure l'inhibition de l'effet pathogène dû au virus, c'est-à-dire la séroneutralisation par coloration des cellules fixées au cristal violet.

Il va de soi que ce test peut être relayé par un ou plusieurs tests encore plus sensibles, mettant en oeuvre des anticorps monoclonaux spécifiques des épitopes contenus dans le polypeptide I et nécessaires à la manifestation des susdites propriétés SHV-séroneutralisantes.

L'invention fournit ainsi les moyens permettant l'identification des polypeptides directement dérivés du polypeptide I, tel qu'il a été défini dans ce qui précède.

L'invention concerne aussi les polypeptides dont les séquences se distinguent des précédentes par un ou plusieurs acides aminés, sans pour autant perdre la capacité de former des complexes antigène/anticorps avec des anticorps neutralisant le virus de la SHV et susceptibles le cas échéant d'induire in vivo des anticorps neutralisants contre ce virus.

Il résulte déjà de ce qui précède que les polypeptides recombinants entrant dans le champ de l'invention ne sauraient être limités au "polypeptide I"

Font en fait partie de l'invention tous les polypeptides plus courts, qui se distinguent du polypeptides I par l'absence d'une région N-terminale ou d'une région C-terminale ou des deux à la fois, sans avoir néanmoins perdu l'épitope (ou les épitopes) intervenant soit dans la reconnaissance antigène-anticorps neutralisant, soit dans l'induction in vivo d'anticorps neutralisants contre le virus de la SHV, dont il a été question plus haut.

Il en va de même des polypeptides dont le polypeptide I ou l'un des polypeptides plus courts susmentionnés n'entrent que pour partie dans leurs propres séquences. A titre d'exemples de polypeptides de ce type on peut mentionner des polypeptides hybrides, dans lesquels les polypeptides immunogènes précédents se retrouvent recombinés avec d'autres séquences d'amino acides, en particulier des séquences "porteuses" dont la présence peut résulter du type de procédé de génie génétique mis en oeuvre pour les produire. Ces séquences sont le plus souvent hétérologues, par exemple sont constituées de fragments de la séquence polypeptidique au sein de laquelle ces polypeptides immunogènes ont été exprimés : à titre d'exemple de telles séquences hétérolologues on mentionnera la bêta-galactosidase de E. coli ou des fragments de celle-ci, notamment lorsque la production du polypeptide immunogène a été effectuée par transformation de E. coli avec un vecteur, plasmide ou phage, comprenant une séquence Lac Z au sein de laquelle un acide nucléique correspondant au polypeptide immunogène avait auparavant été introduit par recombinaison génétique in vitro. Les séquences hétérologues du genre en question peuvent être autres : la seule condition qui leur est imposée est de ne pas interférer de façon substantielle avec les propriétés immunogènes sus-définies des polypeptides recombinants immunogènes de l'invention.

A titre d'exemples d'autres séquences polypeptidiques susceptibles d'être associées au polypeptide immunogène conforme à l'invention, on mentionne en particulier :
- lorsque la synthèse en est assurée par E. coli les produits d'expression du gène TrpE, la protéine de surface OmpA, la lipoprotéine de E. coli, ou
- lorsque la synthèse en est assurée par une levure, les produits d'expression du gène gal 1 ou gal 10 de la levure (notamment lorsque le vecteur utilisé est un plasmide 2µ modifié par ces gènes et l'hôte cellulaire du type Saccharomyces cerevisiae).

Il est à remarquer que le "polypeptide I, défini ci-dessus et les polypeptides II et III" dont question ci-après, sont dérivés de produits d'expression d'un cADN dérivé de la séquence de nucléotides codant pour une protéine G d'un virus précédemment isolé de la SHV, comme on l'indique dans les exemples ci-après.

Les polypeptides tels qu'ils ont été définis, sont dépourvus d'une séquence polypeptidique C-terminale correspondant à ce qui paraît correspondre à une séquence ou peptide d'ancrage de la protéine G dans l'enveloppe du virus.

Les polypeptides selon l'invention peuvent être glycosylés ou non, notamment en certains de leurs sites de glycosylation du type Asn-X-Ser ou Asn-X-Thr, X représentant un acide aminé quelconque.

Un polypeptide particulier entrant dans le cadre des définitions de l'invention, données précédemment, ci-après dénommé "Polypeptide II" comprend tout ou partie de l'enchaînement d'acides aminés suivant :

Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-

```
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

Ce polypeptide II diffère du polypeptide I en ce qu'il est dépourvu du peptide signal.

Un autre polypeptide particulier (polypeptide III) selon l'invention contient tout ou partie de l'enchaînement d'acides aminés suivant :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

L'invention concerne naturellement aussi les polypeptides I, II et III (ou les polypeptides qui en sont dérivés dans les conditions sus-indiquées) qui sont pourvus de (ou prolongé par) un "peptide d'ancrage" lequel présente en particulier la séquence suivante en acides aminés :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

La présence de ce "peptide d'ancrage" dans les peptides immunogènes recombinants obtenus présente un intérêt tout particulier dans le cas où l'on envisage de recourir, pour obtenir l'expression dudit polypeptide recombinant immunogène à un système vecteur-hôte cellulaire dont les composants, c'est-à-dire vecteur d'une part, hôte cellulaire d'autre part, et le cas échéant encore les séquences d'acides nucléiques associées au cADN codant pour le polypeptide immunogène et le peptide d'ancrage qui lui est associé sont choisis de façon à promouvoir le transport du produit d'expression de ce cADN vers la membrane externe de l'hôte cellulaire dans des conditions autorisant l'exposition de la séquence immunogène à la surface de la cellule hôte et son ancrage dans la membrane de celle-ci par l'intermédiaire du peptide d'ancrage. A titre d'exemple d'un tel système vecteur-hôte cellulaire, on peut mentionner celui dans lequel l'hôte cellulaire serait constitué par la levure Saccharomyces cerevisiae et le vecteur par un plasmide d'expression caractérisé en ce qu'il contient une origine de réplication fonctionnelle dans la levure ($2\mu$ ou ARS), un gène permettant sa sélection (Leu2, Ura3 ou Trp1) ainsi qu'un promoteur de levure (GAPDH, ADH1 ou 2, Gal1), par exemple pAAH5. En aval du promoteur, on insère la séquence nucléotidique correspondant au polypeptide I prolongé de la séquence d'ancrage. La séquence signal devrait guider le polypeptide synthétisé dans la cellule vers le réticulum endoplasmique, le golgi puis la membrane externe ou la séquence d'ancrage devra le maintenir fixé.

Dans un ordre d'idées semblable le peptide recombinant immunogène pourrait se trouver associé à une séquence peptidique signal endogène, notamment celle constituée par la séquence d'acides aminés IV suivante :

```
Met-Glu-Trp-Asn-Thr-Phe-Phe-Leu-Val-Ile-Leu-Ile-Ile-
Ile-Ile-Lys-Ser-Thr-Thr-Pro
```

L'invention concerne aussi des compositions de vaccin, contenant le susdit polypeptide recombinant immunogène, le cas échéant couplé à une molécule porteuse (protéine naturelle ou à un polypeptide synthétique) ayant un poids moléculaire suffisant pour que le conjugué soit capable d'induire in vivo la production d'anticorps neutralisants anti-SHV.

Dans le cas où le polypeptide recombinant présent un poids moléculaire suffisant pour induire in vivo la production d'anticorps neutralisants anti-SHV, il n'est pas nécessaire de le coupler à une molécule porteuse.

Selon un aspect particulièrement avantageux de l'invention, le principe actif de vaccin défini est administrable par voie orale, par voie intrapéritonéale ou par voie de balnéation.

Lorsqu'il est administrable par voie orale, ce principe actif contient de 10ng à 100$\mu$g/g nourriture vaccinante, notamment 1 à 5 $\mu$g/g de nourriture.

Lorsqu'il est administrable par voie intrapéritonéale il contient de 1 à 1000ng/g de poisson, notamment 10 à 50ng/g de poisson, et avantageusement de 7 à 10ng/g de poisson.

Enfin, il est administrable par voie de balnéation dans un milieu contenant de 10ng à 100$\mu$g/ml ou par douche (les solutions étant alors de préférence 10 fois plus concentrées en principe actif).

Pour l'administration par balnéation, la composition de vaccin comprend avantageusement le polypeptide I.

Selon un autre mode de caractérisation, le polypeptide de l'invention comprend dans son ossature peptidique tout ou partie d'un enchaînement d'acides aminés codé par l'acide nucléique (1) suivant :

TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG


CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT CCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA ATG

GAA TGG AAC ACT TTT TTC TTG GTG ATC TTG ATC ATC ATC ATA

AAG AGC ACC ACA CCA CAG ATC ACT CAA CGA CCT CCG GTC GAA

AAC ATC TCG ACG TAC CAT GCA GAC TGG GAC ACT CCG CTA TAC

ACT CAT CCC TCC AAC TGC AGG GAC GAT TCC TTT GTC CCG ATT

CGA CCA GCT CAA CTC AGG TGT CCT CAT GAA TTT GAA GAC ATA

AAC AAG GGA CTG GTT TCC GTC CCA ACC AGG ATC ATC CAT CTC

CCG CTA TCG GTC ACC AGC GTC TCC GCA GTA GCG AGT GGC CAC

TAC CTG CAC AGA GTG ACT TAT CGA GTC ACC TGT TCG ACC AGC

TTC TTT GGA GGG CAA ACC ATC GAA AAG ACC ATC TTG GAG GCG

AAA CTG TCT CGT CAG GAG GCC ACA GAC GAG GCA AGC AAG GAT

CAC GAG TAC CCG TTC TTC CCT GAA CCC TCC TGC ATC TGG ATG

AAA AAC AAT GTC CAT AAG GAC ATA ACT CAC TAT TAC AAG ACC

CCA AAA ACA GTA TCG GTG GAT CTC TAC AGC AGG AAA TTT CTC

AAC CCT GAT TTC ATA GAG GGG GTT TGC ACA ACC TCG CCC TGT

CAA ACT CAT TGG CAG GGA GTC TAT TGG GTC GGT GCC ACA CCT

AAA GCC CAT TGC CCC ACG TCG GAA ACA CTA GAA GGA CAC CTG

TTC ACC AGG ACC CAT GAT CAC AGG GTG GTC AAG GCA ATT GTG

GCA GGC CAT CAT CCC TGG GGA CTC ACA ATG GCA TGC ACA GTG

ACA TTC TGC GGG ACA GAA TGG ATC AAG ACT GAC CTG GGG GAC

CTG ATC CAG GTG ACA GGA CCG GGG GGC ACG AGG AAA CTG ACT

CCA AAC AAG TGT GTC AAT ACC GAT ATC CAG ATG AGG GGG GCA

ACA GAC GAC TTT TCT TAT CTC AAC CAT CTC ATC ACC AAC ATG

GCT CAA AGA ACC GAG TGC CTA GAT GCC CAT AGT GAT ATC ACC

```
GCT TCT GGG AAA GTA TCC TCA TTT CTC CTC TCA AAG TTT CGT

CCC AGC CAC CCT GGA CCT GGC AAG GCA CAC TAT CTT CTC GAC

GGT CAA ATC ATG CGA GGT GAC TGT GAC TAT GAG GCA GTA GTC

AGC ATC AAC TAC AAT CGC GCT CAA TAC AAG ACG ATG AAC AAC

ACA TGG AAA TCA TGG AAA CGG GTA GAC AAC AAC ACA GAC GGG

TAC GAT GGG ATG ATA TTT GGG GAC AAA TTG ATC ATC CCG GAC

ATC GAA AAG TAT CAG AGT GTC TAT GAC AGT GGA ATG CTC GTT

CAA AGA AAC CTT GTG GAA GTC CCT CAT CTG AGC ATT GTG TTT

GTC TCC AAC ACA TCT GAT CTT TCC ACT AAT CAC ATC CAC ACC

AAC CTA ATC CCT TCG GAT
```

L'invention concerne encore les acides nucléiques codant pour des polypeptides sus-indiqués, et en particulier concerne tout ou partie de l'acide nucléique (1) ci-dessus.

Cet acide nucléique peut être modifié dès lors que la séquence d'acides aminés qu'il code conserve les propriétés biologiques ci-dessus.

L'invention concerne également les acides nucléiques codant pour les polypeptides sus-indiqués, et plus particulièrement un acide nucléique (1) qui comprend tout ou partie de l'enchaînement de nucléotides suivant :

```
TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA
```

EP 0 377 349 B1

GGA CTG ACC CAG GTT TGA GAC CAC ACT CTC ATT AGA TAG AAA

AAA ATG GCA CAT TTG TGT ACA CAA CAA GCT AGA CCC ACA ATG

GAA TGG AAC ACT TTT TTC TTG GTG ATC TTG ATC ATC ATC ATA

AAG AGC ACC ACA CCA CAG ATC ACT CAA CGA CCT CCG GTC GAA

AAC ATC TCG ACG TAC CAT GCA GAC TGG GAC ACT CCG CTA TAC

ACT CAT CCC TCC AAC TGC AGG GAC GAT TCC TTT GTC CCG ATT

CGA CCA GCT CAA CTC AGG TGT CCT CAT GAA TTT GAA GAC ATA

AAC AAG GGA CTG GTT TCC GTC CCA ACC AGG ATC ATC CAT CTC

CCG CTA TCG GTC ACC AGC GTC TCC GCA GTA GCG AGT GGC CAC

TAC CTG CAC AGA GTG ACT TAT CGA GTC ACC TGT TCG ACC AGC

TTC TTT GGA GGG CAA ACC ATC GAA AAG ACC ATC TTG GAG GCG

AAA CTG TCT CGT CAG GAG GCC ACA GAC GAG GCA AGC AAG GAT

CAC GAG TAC CCG TTC TTC CCT GAA CCC TCC TGC ATC TGG ATG

AAA AAC AAT GTC CAT AAG GAC ATA ACT CAC TAT TAC AAG ACC

CCA AAA ACA GTA TCG GTG GAT CTC TAC AGC AGG AAA TTT CTC

AAC CCT GAT TTC ATA GAG GGG GTT TGC ACA ACC TCG CCC TGT

CAA ACT CAT TGG CAG GGA GTC TAT TGG GTC GGT GCC ACA CCT

AAA GCC CAT TGC CCC ACG TCG GAA ACA CTA GAA GGA CAC CTG

TTC ACC AGG ACC CAT GAT CAC AGG GTG GTC AAG GCA ATT GTG

GCA GGC CAT CAT CCC TGG GGA CTC ACA ATG GCA TGC ACA GTG

ACA TTC TGC GGG ACA GAA TGG ATC AAG ACT GAC CTG GGG GAC

CTG ATC CAG GTG ACA GGA CCG GGG GGC ACG AGG AAA CTG ACT

CCA AAC AAG TGT GTC AAT ACC GAT ATC CAG ATG AGG GGG GCA

ACA GAC GAC TTT TCT TAT CTC AAC CAT CTC ATC ACC AAC ATG

GCT CAA AGA ACC GAG TGC CTA GAT GCC CAT AGT GAT ATC ACC

GCT TCT GGG AAA GTA TCC TCA TTT CTC CTC TCA AAG TTT CGT

CCC AGC CAC CCT GGA CCT GGC AAG GCA CAC TAT CTT CTC GAC

GGT CAA ATC ATG CGA GGT GAC TGT GAC TAT GAG GCA GTA GTC

AGC ATC AAC TAC AAT CGC GCT CAA TAC AAG ACG ATG AAC AAC

ACA TGG AAA TCA TGG AAA CGG GTA GAC AAC AAC ACA GAC GGG

TAC GAT GGG ATG ATA TTT GGG GAC AAA TTG ATC ATC CCG GAC

ATC GAA AAG TAT CAG AGT GTC TAT GAC AGT GGA ATG CTC GTT

CAA AGA AAC CTT GTG GAA GTC CCT CAT CTG AGC ATT GTG TTT

GTC TCC AAC ACA TCT GAT CTT TCC ACT AAT CAC ATC CAC ACC

AAC CTA ATC CCT TCG GAT

13

ou encore l'acide nucléique (2) caractérisé par tout ou partie de l'enchaînement de nucléotides suivant :

```
                                    ATG GAA TGG AAC ACT TTT TTC TTG GTG
        ATC TTG ATC ATC ATC ATA AAG AGC ACC ACA CCA CAG ATC ACT
        CAA CGA CCT CCG GTC GAA AAC ATC TCG ACG TAC CAT GCA GAC
        TGG GAC ACT CCG CTA TAC ACT CAT CCC TCC AAC TGC AGG GAC
        GAT TCC TTT GTC CCG ATT CGA CCA GCT CAA CTC AGG TGT CCT
        CAT GAA TTT GAA GAC ATA AAC AAG GGA CTG GTT TCC GTC CCA
        ACC AGG ATC ATC CAT CTC CCG CTA TCG GTC ACC AGC GTC TCC
        GCA GTA GCG AGT GGC CAC TAC CTG CAC AGA GTG ACT TAT CGA
        GTC ACC TGT TCG ACC AGC TTC TTT GGA GGG CAA ACC ATC GAA
        AAG ACC ATC TTG GAG GCG AAA CTG TCT CGT CAG GAG GCC ACA
        GAC GAG GCA AGC AAG GAT CAC GAG TAC CCG TTC TTC CCT GAA
        CCC TCC TGC ATC TGG ATG AAA AAC AAT GTC CAT AAG GAC ATA
        ACT CAC TAT TAC AAG ACC CCA AAA ACA GTA TCG GTG GAT CTC
        TAC AGC AGG AAA TTT CTC AAC CCT GAT TTC ATA GAG GGG GTT
        TGC ACA ACC TCG CCC TGT CAA ACT CAT TGG CAG GGA GTC TAT
        TGG GTC GGT GCC ACA CCT AAA GCC CAT TGC CCC ACG TCG GAA
        ACA CTA GAA GGA CAC CTG TTC ACC AGG ACC CAT GAT CAC AGG
        GTG GTC AAG GCA ATT GTG GCA GGC CAT CAT CCC TGG GGA CTC
        ACA ATG GCA TGC ACA GTG ACA TTC TGC GGG ACA GAA TGG ATC
        AAG ACT GAC CTG GGG GAC CTG ATC CAG GTG ACA GGA CCG GGG
        GGC ACG AGG AAA CTG ACT CCA AAC AAG TGT GTC AAT ACC GAT
        ATC CAG ATG AGG GGG GCA ACA GAC GAC TTT TCT TAT CTC AAC
        CAT CTC ATC ACC AAC ATG GCT CAA AGA ACC GAG TGC CTA GAT
        GCC CAT AGT GAT ATC ACC GCT TCT GGG AAA GTA TCC TCA TTT
        CTC CTC TCA AAG TTT CGT CCC AGC CAC CCT GGA CCT GGC AAG


        GCA CAC TAT CTT CTC GAC GGT CAA ATC ATG CGA GGT GAC TGT
        GAC TAT GAG GCA GTA GTC AGC ATC AAC TAC AAT CGC GCT CAA
        TAC AAG ACG ATG AAC AAC ACA TGG AAA TCA TGG AAA CGG GTA
        GAC AAC AAC ACA GAC GGG TAC GAT GGG ATG ATA TTT GGG GAC
        AAA TTG ATC ATC CCG GAC ATC GAA AAG TAT CAG AGT GTC TAT
        GAC AGT GGA ATG CTC GTT CAA AGA AAC CTT GTG GAA GTC CCT
        CAT CTG AGC ATT GTG TTT GTC TCC AAC ACA TCT GAT CTT TCC
        ACT AAT CAC ATC CAC ACC AAC CTA ATC CCT TCG GAT
```

EP 0 377 349 B1

ou encore l'acide nucléique (3) caractérisé par tout ou partie de l'enchaînement de nucléotides suivant :

```
CAG ATC ACT CAA CGA CCT CCG GTC GAA AAC ATC TCG ACG TAC
CAT GCA GAC TGG GAC ACT CCG CTA TAC ACT CAT CCC TCC AAC
TGC AGG GAC GAT TCC TTT GTC CCG ATT CGA CCA GCT CAA CTC
AGG TGT CCT CAT GAA TTT GAA GAC ATA AAC AAG GGA CTG GTT
TCC GTC CCA ACC AGG ATC ATC CAT CTC CCG CTA TCG GTC ACC
AGC GTC TCC GCA GTA GCG AGT GGC CAC TAC CTG CAC AGA GTG
ACT TAT CGA GTC ACC TGT TCG ACC AGC TTC TTT GGA GGG CAA
ACC ATC GAA AAG ACC ATC TTG GAG GCG AAA CTG TCT CGT CAG
GAG GCC ACA GAC GAG GCA AGC AAG GAT CAC CAG TAC CCG TTC
TTC CCT GAA CCC TCC TGC ATC TGG ATG AAA AAC AAT GTC CAT
AAG GAC ATA ACT CAC TAT TAC AAG ACC CCA AAA ACA GTA TCG
GTG GAT CTC TAC AGC AGG AAA TTT CTC AAC CCT GAT TTC ATA
GAG GGG GTT TGC ACA ACC TCG CCC TGT CAA ACT CAT TGG CAG
GGA GTC TAT TGG GTC GGT GCC ACA CCT AAA GCC CAT TGC CCC
ACG TCG GAA ACA CTA GAA GGA CAC CTG TTC ACC AGG ACC CAT
GAT CAC AGG GTG GTC AAG GCA ATT GTG GCA GGC CAT CAT CCC
TGG GGA CTC ACA ATG GCA TGC ACA GTG ACA TTC TGC GGG ACA
GAA TGG ATC AAG ACT GAC CTG GGG GAC CTG ATC CAG GTG ACA
GGA CCG GGG GGC ACG AGG AAA CTG ACT CCA AAC AAG TGT GTC
AAT ACC GAT ATC CAG ATG AGG GGG GCA ACA GAC GAC TTT TCT
TAT CTC AAC CAT CTC ATC ACC AAC ATG GCT CAA AGA ACC GAG
```

```
TGC CTA GAT GCC CAT AGT GAT ATC ACC GCT TCT GGG AAA GTA
TCC TCA TTT CTC CTC TCA AAG TTT CGT CCC AGC CAC CCT GGA
CCT GGC AAG GCA CAC TAT CTT CTC GAC GGT CAA ATC ATG CGA
GGT GAC TGT GAC TAT GAG GCA GTA GTC AGC ATC AAC TAC AAT
CGC GCT CAA TAC AAG ACG ATG AAC AAC ACA TGG AAA TCA TGG
AAA CGG GTA GAC AAC AAC ACA GAC GGG TAC GAT GGG ATG ATA
TTT GGG GAC AAA TTG ATC ATC CCG GAC ATC GAA AAG TAT CAG
AGT GTC TAT GAC AGT GGA ATG CTC GTT CAA AGA AAC CTT GTG
GAA GTC CCT CAT CTG AGC ATT GTG TTT GTC TCC AAC ACA TCT
GAT CTT TCC ACT AAT CAC ATC CAC ACC AAC CTA ATC CCT TCG
GAT
```

15

ou encore l'acide nucléique (4) caractérisé par tout ou partie de l'enchaînement de nucléotides suivant :

```
ATG CGA GGT GAC TGT GAC TAT GAG GCA GTA GTC AGC ATC AAC
TAC AAT CGC GCT CAA TAC AAG ACG ATG AAC AAC ACA TGG AAA
TCA TGG AAA CGG GTA GAC AAC AAC ACA GAC GGG TAC GAT GGG
ATG ATA TTT GGG GAC AAA TTG ATC ATC CCG GAC ATC GAA AAG
TAT CAG AGT GTC TAT GAC AGT GGA ATG CTC GTT CAA AGA AAC
CTT GTG GAA GTC CCT CAT
```

ou encore l'acide nucléique (5) qui comprend tout ou partie de l'enchaînement de nucléotides suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
```

```
GGA CTG ACC CAG GTT TGA GAC CAC ACT CTC ATT AGA TAG AAA
AAA ATG GCA CAT TTG TGT ACA CAA CAA GCT AGA CCC ACA
```

L'invention concerne aussi une séquence de nucléotides codant pour le peptide d'ancrage défini dans les pages précédentes. Cette séquence comprend tout ou partie de l'enchaînement nucléotidique suivant (6):

```
TGG TCA TTC AAC TGG AGT CTT TGG CCA TCA TTA TCT
GGG ATG GGG GTT GTG GGA GGG GCC TTC CTT CTA CTG GTA CTC
TGC TGT TGC TGC AAG GCG TCC CCT CCC ATT CCA AAT TAC GGG
ATT CCG ATG CAG CAG TTC TCC AGA AGT CAG ACG GTC TGA GCA
CAC CTG TCC GAA TGA CCA CAA TTC CTC TCT TAG GTA GAT AGA
AAA AAA AAA AAA AAA AAA AAA AAA A
```

L'utilisation d'un acide nucléique associant des séquences codant pour un peptide immunogène conforme à l'invention, associé à une séquence signal telle que celle indiquée ci-dessus ; ou encore associé à une séquence signal hétérologue vis-à-vis du polypeptide immunogène de l'invention, peut être envisagée dans tout système vecteur-hôte cellulaire mis en oeuvre pour l'expression du peptide immunogène associé au peptide signal (sous réserve que celui-ci ait été convenablement choisi) met à profit la capacité de l'hôte cellulaire, de préférence eucaryote, (en particulier levure ou cellule de mammifère) à exploiter l'information susceptible de lui être fourni par la séquence d'ADN signal pour assurer le transport du polypeptide immunogène vers le périplasme, voire son excrétion dans le milieu de culture de l'hôte cellulaire, après clivage du peptide signal.

A titre d'exemples encore d'un tel système vecteur-hôte cellulaire compétent, susceptible de coopérer dans les conditions sus-indiquées, on mentionne,

- au titre du peptide signal, celui de Suc2 (l'invertase),
- au titre de vecteur, un vecteur d'expression par exemple pAAH5,
- au titre de l'hôte cellulaire, la levure Saccharomyces cerevisiae.

Appartiennent également à l'invention, les acides nucléiques décrits ci-dessus, précédés d'une séquence nucléotidique comprenant les éléments de régulation y inclus le promoteur sous le contrôle duquel s'effectue la transcription des éléments initiateurs et terminateurs de la traduction qui lui sont normalement associés dans le gène isolé à partir du virus.

Les acides nucléiques de l'invention peuvent être préparés soit par un procédé chimique, soit par d'autres procédés.

Un mode de préparation approprié des acides nucléiques (comportant au maximum 200 nucléotides - ou pb, lorsqu'il s'agit d'acides nucléiques bicaténaires) de l'invention par voie chimique comprend les étapes suivantes:

- la synthèse d'ADN en utilisant la méthode automatisée des $\beta$-cyanethyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée.

Un mode de préparation, par voie chimique, d'acides nucléiques de longueur supérieure à 200 nucléotides - ou pb (lorsqu'il s'agit d'acides nucléiques bicaténaires) comprend les étapes suivantes:

- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

Les acides nucléiques de l'invention peuvent également être préparés à partir de l'ARN génomique du virus par un procédé comprenant les étapes suivantes:

- la centrifugation du surnageant d'une culture de cellules infectées par un isolat de la SHV du type I et la récupération du culot de centrifugation;
- le cas échéant la centrifugation du culot récupéré sur un gradient de sucrose par la méthode décrite dans le brevet EP-A-0138667,
- la récupération de la portion du gradient contenant le virus et la resédimentation du virus par centrifugation,
- la resuspension du culot de virus et l'extraction de l'ARN génomique selon la technique décrite par Chomzynski et al. (Anal. Biochem. 162, 156, 1987),
- la synthèse d'un brin d'ADNc au contact de l'ARN génomique du virus par une transcriptase réverse en présence d'initiateurs oligomériques dont les séquences sont aléatoires selon la technique décrite dans DNA 4:429-438, 1985,
- le cas échéant, la purification des hybrides ARN/ADNc par extraction par un mélange phénol/chloroforme et précipitation à l'éthanol,
- la fabrication d'un second brin d'ADNc en présence d'ADN polymérase I, de RNAse H et de 4 désoxynucléotides selon la méthode décrite dans Gene, 25:263-269 (1983), si besoin est après extraction des protéines présentes dans le milieu de réaction,
- le clonage des acides nucléiques ainsi obtenus dans un vecteur plasmidien approprié et la récupération de l'acide nucléique recherché à l'aide d'une sonde appropriée.

Un autre procédé de préparation des acides nucléiques de l'invention, à partir de l'ARNm comprend les étapes suivantes:

- la préparation des ARN cellulaires et viraux à partir d'une culture de cellules infectées depuis 16 à 20 heures par le virus de la SHV de type 1, à une multiplicité de 10 ufp par cellule, selon la technique décrite par Chomzynski et al. (Anal. Biochem 162, 156, 1987),
- la récupération et purification des ARNm cellulaires et viraux par passage des ARN cellulaires et viraux totaux par chromatographie avec un oligo(dT) immobilisé,
- la synthèse d'un brin d'ADNc à partir des ARNm purifiés selon la technique décrite dans Gene 25:263, 1983,
- le clonage des acides nucléiques ainsi obtenus dans un vecteur plasmidien approprié et la récupération de la séquence nucléique recherchée à l'aide d'une sonde d'hybridation appropriée.

# EP 0 377 349 B1

Pour préparer les acides nucléiques (1), (2), (3), (4), (5) et (6) ci-dessus définis, on peut avoir recours aux sondes d'hybridation suivantes:

sonde 1: ACG TTG GAG GGA AAG GCC

sonde 2: TTC TTG GTG ATC TTG ATC

sonde 3: AAG ACC ATC TTG GAG GCG

sonde 4: GAC GGG TAC GAT GGG ATG

sonde 5: GGG GTT GTG GGA GGG GCC dans les conditions d'hybridation semblables à celles indiquées à propos des sondes décrites ci-après.

Le caractérisation des séquences (1) à (6) par les sondes ci-dessus indiquées se fait selon les indications données dans le Tableau I ci-après:

TABLEAU I

| séquences | 1 | 2 | 3 | 4 | 5 | 6 |
|-----------|---|---|---|---|---|---|
| sondes |   |   |   |   |   |   |
| 1 | + | - | - | - | + | - |
| 2 | + | + | - | - | - | - |
| 3 | + | + | + | - | - | - |
| 4 | + | + | + | + | - | - |
| 5 | - | - | - | - | - | + |

Dans ce tableau, le signe "+" indique l'existence d'une réaction d'hybridation entre une sonde et une séquence déterminée et le signe "-" indiqué l'absence de réaction d'hybridation.

Dans le cadre de la préparation d'acides nucléiques à partir de l'ARN génomique, l'étape consistant à synthétiser un brin d'ADNc peut être effectuée de la façon suivante:

On part de l'ARN génomique et on utilise la méthode décrite par Saiki et al. Science 239, 487, 1988.

L'utilisation d'amorces spécifiques permet de synthétiser spécifiquement les séquences d'acides nucléiques recherchées. La première étape est la synthèse d'un premier brin d'ADN complémentaire par une transcriptase réverse en présence des amplimères spécifiques. Ensuite cet ADNc simple brin est amplifié par des cycles successifs avec une DNA polymérase par la technique PCR (polymerase chain reaction) décrite par exemple dans les brevets US n° 4,683,202 et n° 4,683,195 et le brevet européen n° 200362.

L'obtention d'une séquence particulière est garantie par l'utilisation des amplimères ci-dessous. L'amplimère "a" est celui qui sert d'amorce à la transcriptase réverse. Toutes les séquences sont données dans le sens 5'-->3'.

Séquence 1:

amplimère 1a: TCT TTG GAG AAA CCA GTG AGA

amplimère 1b: ATC CGA AGG GAT TAG GTT GGT

Séquence 2:

amplimère 2a: ATG GAA TGG AAC ACT TTT TTC

amplimère 1b: ATC CGA AGG GAT TAG GTT GGT

Séquence 3:

amplimère 3a: GAG ATC ACT CAA CGA CCT CCG

amplimère 1b:ATC CGA AGG GAT TAG GTT GGT

Séquence 4:

amplimère 4a: ATG CGA GGT GAC TGT GAC TAT

amplimère 4b: ATG AGG GAC TTC CAC AAG GTT

18

Séquence 5:

    amplimère 1a: TCT TTG GAG AAA CCA GTG AGA
    amplimère 5b: TGT GGG TCT AGC TTG TTG TGT

Séquence 6:

    amplimère 6a: TGG TCA TTC AAC TGG AGT CTT
    amplimère 6b (g): TCT ATC TAC CTA AGA GAG GAA

L'amplimère 6b n'est pas un polyT car l'ARN génomique n'est pas polyadénylé.

Les séquences ainsi obtenues sont examinées en électrophorèse sur gel d'agarose afin de s'assurer qu'elles possèdent la taille attendue. Elles sont ensuite clonées dans un vecteur plasmidien approprié.

Dans la préparation d'acides nucléiques à partir de l'ARN messager décrite ci-dessus, la synthèse d'ADNc peut être effectuée de la façon suivante:

On part d'ARN messager et on utilise la méthode décrite par Saiki et al. Science 239, 487, 1988.

L'utilisation d'amorces spécifiques permet de synthétiser spécifiquement les séquences décrites. La première étape est la synthèse d'un premier brin d'ADN complémentaire par une transcriptase réverse en présence des amplimères spécifiques. Ensuite cet ADNc simple brin est amplifié par des cycles successifs avec une DNA polymérase par la technique PCR.

L'obtention d'une séquence particulière est garantie par l'utilisation des amplimères décrits ci-dessus. L'amplimère "b" défini ci-dessus est celui qui sert d'amorce à la transcriptase réverse. Toutes les séquences sont données dans le sens 5′-->3′. Mais puisque les ARN messager sont polyadénylés on peut remplacer l'amplimère 6b(g) par l'amplimère 6b(m): TTT TTT TTT TTT TTT TTT TTT

Les acides nucléiques 1 à 6 de l'invention peuvent également être repérés dans le cadre de la préparation des acides nucléiques (à partir de l'ARN génomique ou à partir de l'ARN messager), à l'aide des séquences amplimères décrites ci-dessus, utilisées comme sondes, à condition que les séquences d'acides nucléiques à déterminer ne connaissent pas de délétion interne, sinon il faut séquencer, la mesure de la taille par électrophorèse pouvant être indicative.

Pour reconnaître l'une des séquences d'acides nucléiques 1 à 6, on procède à 3 ou 4 hybridations avec les amplimères flanquants utilisés comme sondes, mais il est également nécessaire de définir trois sondes pour couvrir les limites non utilisées par les amplimères et qui sont les suivantes:

    sonde 7: ATA AAG AGC ACC ACA CCA
    sonde 8: CTT CTC GAC GGT CAA ATC
    sonde 9: CTG AGC ATT GTG TTT GTC

La reconnaissance des séquences 1 à 6 par les susdits amplimères se fait selon les indications données dans le Tableau II ci-après:

TABLEAU II

| hybridation | + | + | - | - |
|-------------|-----|-----|-----|-----|
| séquence |  |  |  |  |
| 1 | 1a | 1b | 6a |  |
| 2 | 2a | 1b | 5b | 6a |
| 3 | 3a | 1b | 7 | 6a |
| 4 | 4a | 4b | 8 | 9 |
| 5 | 1a | 5b | 2a |  |
| 6 | 6a | 6b | 1b |  |

Dans ce tableau, le signe "+" indique l'existence d'une réaction d'hybridation entre une sonde et une séquence déterminée et le signe "-" indiqué l'absence de réaction d'hybridation.

Font également partie de l'invention les acides nucléiques recombinants dans lesquels les séquences d'acides nucléiques codant pour le polypeptide immunogène, le cas échéant aussi le peptide signal et/ou le peptide d'ancrage sont recombinés avec des éléments de régulation hétérologues vis-à-vis de ceux avec lesquels ils sont normalement associés au sein du gène viral, et plus particulièrement des éléments de régulation adaptés à réguler leur expression dans l'hôte cellulaire choisi pour assurer leur production.

En particulier l'invention concerne des vecteurs recombinants de ce type, plasmides, cosmides ou phages modifiés par un acide nucléique codant pour le polypeptide immunogène recombinant en l'un de ses sites non essentiels pour sa réplication.

Ce vecteur recombinant peut ainsi être formé par l'ADN hétérologue précédent dans la mesure où celui-ci constitue un système autonome de réplication.

Un vecteur recombinant particulier est caractérisé en ce qu'il est constitué par le plasmide PSHV-G1 déposé à la Collection Nationale de Culture de Microorganisme de l'Institut Pasteur de Paris (CNCM) le 8 Juillet 1988 sous le n° I 778.

Dans un mode de réalisation particulier de l'invention, le vecteur recombinant ci-dessus est un vecteur d'expression contenant en l'un de ses sites non essentiels pour sa réplication, des éléments nécessaires pour promouvoir l'expression, dans un hôte cellulaire, d'un acide nucléique codant pour une séquence d'acides aminés de l'invention, un promoteur compatible avec ledit hôte cellulaire, en particulier un promoteur inductible, et éventuellement une séquence signal et/ou une séquence d'ancrage membranaire.

Le choix du promoteur, de la séquence signal et de la séquence d'ancrage est déterminé en fonction de la nature du système d'expression choisi. Un vecteur recombinant particulier approprié à l'expression des séquences d'acides aminés selon l'invention dans E. Coli, est caractérisé en ce qu'il comprend, en un site non essentiel pour sa réplication, un ADN recombinant résultant lui-même de l'insertion d'un acide nucléique décrit ci-dessus dans un site du gène codant pour tout ou partie de la β-galactosidase et qui contient en outre les éléments permettant l'expression de l'acide nucléique inséré dans le vecteur par E. coli et notamment, les éléments permettant l'expression de tout ou partie du gène de la β-galactosidase.

Un autre vecteur recombinant est caractérisé en ce qu'il contient les éléments permettant d'insérer un acide nucléique, décrit plus haut, dans une levure et l'expression de cet acide nucléique dans cette levure.

D'autres cellules eucaryotes pourront être choisies pour insérer le vecteur recombinant contenant l'acide nucléique de l'invention, ce choix étant orienté par la capacité de ladite cellule eucaryote à organiser l'expression dudit acide nucléique.

L'invention se rapporte encore aux hôtes cellulaires transformés par un vecteur recombinant, tels que décrits plus haut, vecteur capable de se répliquer dans ledit microorganisme et comprenant les éléments de régulation permettant l'expression de la séquence nucléique codant pour le peptide immunogène recombinant de l'invention dans cet hôte.

Un premier hôte cellulaire préféré est constitué par E. coli transformé par un vecteur recombinant tel que décrit précédemment, et avantageusement par un vecteur recombinant contenant l'ADN codant pour l'une des protéines de fusion définies précédemment, et en particulier codant pour la β-galactosidase ou un fragment de la β-galactosidase associé à l'une des séquences d'acides aminés ci-dessus ou l'une des séquences la comprenant décrite plus haut.

D'autres hôtes cellulaires entrant dans le cadre de l'invention sont des levures transformées par les vecteurs recombinants appropriés correspondants, par exemple Saccharomyces cerevisiae transformée par un plasmide recombinant dérivé du plasmide 2μ ou Hansennulla polymorpha transformé par un plasmide recombinant dérivé de 2μ.

D'autres hôtes cellulaires peuvent encore être mis en oeuvre, notamment des champignons filamenteux par exemple Aspergillus niger transformé par un vecteur recombinant dérivé de 2μ.

L'invention vise également les sondes utilisables pour détecter des ARN viraux dans un échantillon biologique notamment de cellules, telles que des cellules isolées de rein ou d'un autre organe provenant des poissons suspects d'être porteurs d'une infection virale due à SHV, par hybridation avec un brin d'ADN complémentaire généralement marqué et dont la séquence nucléique est contenue dans l'un des brins des ADNs (1) à (6) ci-dessus.

Ces sondes peuvent être marquées de toute façon en soi connue (marquage radioactif, enzymatique, fluorescent, etc...).

A titre d'exemple de sondes susceptibles d'être mises en oeuvre pour détecter l'ARN viral, on mentionne les séquences nucléiques suivantes :

ACG TTG GAG GGA AAG GCC
GAC TGG GAC ACT CCG CTA
CCC TCC TGC ATC TGG ATG
AAG ACT GAC CTG GGG GAC
GAC AAC AAC ACA GAC GGG
TTC CTT CTA CTG GTA CTC

Les sondes de l'invention peuvent être préparées par voie chimique, telle qu'indiquée à propos de la préparation des acides nucléiques de l'invention comportant au maximum 200pb.

Ces sondes pour détecter l'ARN viral peuvent notamment être utilisées comme suit.

Des cellules de rein ou d'un autre organe provenant du poisson éventuellement infecté ou les virus précipités au polyéthylène-glycol provenant d'une eau suspecte, sont préalablement traités, par exemple comme indiqué dans l'exemple I ci-après pour rendre accessible l'ARN viral à la sonde utilisée.

La préparation contenant l'ARN cible ainsi dégagé est déposée par exemple sur une membrane de nitrocellulose, dans du 2 x SSC et fixée sur la membrane par chauffage 2 heures à 80°C sous vide.

Une pré-hybridation est effectuée dans les conditions suivantes : traitement à 16 heures à 56°C dans le milieu de pré-hybridation contenant le tampon suivant : 3M chlorure de tétraméthylammonium, 50mM Tris-HC1, pH 8.0, 2mM EDTA, 100µg/ml DNA soniqué, dénaturé de thymus de veau, 5X Denhardt,

- l'hybridation proprement dite est effectuée dans le même tampon, dans les conditions suivantes : traitement pendant 1 heure à la même température avec environ 100 pg d'une des sondes ou d'un mélange des sondes indiquées ci-dessus.

On effectue ensuite un lavage dans les conditions suivantes :

. 5 minutes dans 2X SSC, 0,1% SDS à température ambiante,

. 5 minutes dans 5X SSC, 0,1% SDS à 59°C,

. deux passages dans le tampon d'hybridation ci-dessus, sans Denhardt ni ADN de veau pendant 1 h, à 59°C.

On peut également utiliser la méthode récemment écrite (Saiki et al Science 239, 487, 1988) d'amplification d'ARN pour détecter l'ARN viral. Dans ce cas les amorces d'ADN simple chaîne (20-mer) sont de deux signes différents. La première sera complémentaire de l'ARN viral, de manière à amorcer la synthèse d'un brin complémentaire d'ADN par la transcriptase réverse. La deuxième amorce est de même signe que l'ARN viral et par conséquent complémentaire de l'ADN synthétisé. Idéalement les deux sondes sont distantes d'approximativement 100 paires de bases. Voici deux couples d'amorces qui répondent à ces exigences:

$$I \quad 5' \; TTG \; GTG \; ATC \; TTG \; ATC \; ATC \; A \; 3'$$

$$5'A \; GGA \; ATC \; GTC \; CCT \; GCA \; GTT \; 3'$$

$$II \; 5' \; GAC \; TAT \; GAG \; GCA \; GTA \; GTC \; A \; 3'$$

$$5' \; G \; GAT \; GAT \; CAA \; TTT \; GTC \; CCC \; 3'$$

Les séquences nucléiques complémentaires respectivement de chacune des susdites séquences peuvent être utilisées comme sondes pour la détection d'ARNm dans un échantillon biologique.

On peut également rechercher la présence de virus dans une eau suspecte. Par exemple 10 litres d'eau sont concentrés à 100ml par ultrafiltration, puis les virus sont précipités par le polyéthylèneglycol.

Les sondes elles-mêmes peuvent naturellement donner lieu à des mutations localisées, sinon ponctuelles. Font donc partie de l'invention les sondes ainsi modifiées dès lors qu'elles hybrident avec l'un au moins des cADNs qui ont été définis plus haut, notamment dans les conditions explicitées plus loin dans l'exemple I, sous le sous-titre :"4/Identification des clones recombinants porteurs de l'ADNc du virus de la SHV".

L'invention se rapporte également à des anticorps dirigés contre les polypeptides recombinants immunogènes, en particulier, des anticorps monoclonaux.

Un procédé de préparation d'anticorps comprend:

- l'immunisation d'un animal à l'aide d'un polypeptide ci-dessus défini, et
- la récupération des anticorps formés selon les techniques classiques.

Les anticorps monoclonaux secrétés par des hybridomes sont obtenus par des techniques devenues classiques et sélectionnés d'après l'aptitude des anticorps monoclonaux produits à reconnaître des épitopes dont aura été reconnue, par exemple comme indiquée plus haut, la présence nécessaire dans le polypeptide I, pour que se manifeste les propriétés SHV séroneutralisantes de ce dernier.

D'autres caractéristiques et avantages de l'invention apparaîtront dans les exemples qui suivent et dans les figures illustrant ces exemples.

EXEMPLE I :PREPARATION DE L'ADN COMPLEMENTAIRE (ADNc OU DNAc), DE L'ARN MESSAGER (ENCORE DESIGNE PAR ARNm OU RNAm) DU VIRUS DE LA SEPTICEMIE HEMORRAGIQUE VIRALE :

1) Culture cellulaire :

Une culture à 30°C de cellules de la lignée EPC (Epithelioma papulosum cyprini) (décrite par Fijan, N. et al. dans Ann. Virol.134E, 207 1983) est réalisée selon la méthode de de Kinkelin, P. et al. (rapportée dans Develop. Biol. Stand. 42, 99, 1978) dans le MEM (Milieu Essentiel Minimum) (Gibco 0721500) complémenté en acides aminés, vitamines et phosphate de tryptose, jusqu'à une densité cellulaire de 300.000/cm². Cette culture est infectée par des particules virales de la SHV du type I (Le Berre, M. et al., Bull. Off. Int. Epiz. 87, 391, 1975) à un taux de 10 virions par cellules. Dans ces conditions, le cours normal de l'infection virale est caractérisé par la lyse des cellules et le relargage des particules virales au bout de 48 heures. Afin d'obtenir une quantité importante d'ARN messager caractéristique du virus de la SHV, l'infection est interrompue après 20 heures. Les ARNs sont préparés à partir de 135.000.000 de cellules par la méthode de Chomzynski, P. et al. (Anal. Biochem. 162, 156, 1987).

2)Préparation d'ARNm :

Le tapis formé par la culture de cellules est rincé à l'aide de 15 ml de PBS, le surnageant est aspiré, puis les cellules sont lysées à l'aide de 15ml de solution dénaturante (solution D, thiocyanate de guanidium 4M, citrate de Na 25 mM, sarcosyl 0,5%, $\beta$-mercaptoethanol 0,1M). Le mélange est transféré dans un tube stérile. On y ajoute alors, successivement et en agitant doucement par inversion après l'addition de chaque réactif, 1,5 ml d'acétate de sodium 2M, 15 ml de phénol, et 3 ml de chloroforme/alcool iso-amylique. Le mélange est alors refroidi sur la glace pendant 15 minutes, puis placé dans une centrifugeuse pendant 20 minutes à 10000 g à 4°C.

La phase aqueuse (phase supérieure) est séparée de la phase inférieure et de l'interphase, dans lesquelles se trouvent l'ADN et les protéines. 1 volume d'isopropanol est ensuite ajouté, et on laisse reposer 1 heure au moins à -20°C. Puis, on centrifuge pendant 20 minutes à 10000 g à 4°C. Le surnageant est ensuite éliminé et le culot d'ARN est resuspendu dans 0,7 ml de solution D, puis transféré dans un tube Eppendorf stérile, dans lequel il est précipité à l'aide de 1 volume d'isopropanol pendant 1 heure à -20°C.

Après centrifugation, les ARN sont dissous dans un volume minimum d'eau.

Les ARNm ont été purifiés sur une colonne de cellulose oligo dT (Pharmacia PL7). La solution d'ARN cellulaires totaux est passée sur la colonne dans le tampon suivant : 0,5M NaCl 20mM Tris-HCl pH8 et 10 mM EDTA. La colonne est ensuite rincée avec le même tampon puis par un mélange de 0,1M NaCl, 20mM Tris-HCl à pH8 et 10mM EDTA. Enfin les ARN polyadénylés sont décrochés dans de l'eau pure. Le rendement de la purification des ARNm cellulaires et viraux à partir de 135.000.000 de cellules infectées est de 35$\mu$g.

3)Synthèse de l'ADNc :

Les ADNc ont été synthétisés à partir de 8$\mu$g d'ARNm purifiés par la méthode de Gubler, U. et al. (Gene 25, 263, 1983) en utilisant le kit Boerhinger mannheim (cDNA synthesis kit cat. n° 1013883).

Les ADNc obtenus ont été chromatographiés sur une colonne de Sepharose 4B (Pharmacia), les fractions contenant des molécules d'une longueur supérieure à 1kb ont été insérées au site Sma1 du plasmide pUC13 (Pharmacia pUC13 Sma1 Bap) dans 10$\mu$l de tampon de ligation (Tri-HCl20mM pH 7,6 MgCl$_2$10mM, dithiothreitol 10mM, ATP1mM) en présence d'une unité de ligase de T4, pendant 16 heures à 14°C. Les plasmides ont été introduits par transformation (Maniatis, T. et al., Molecular cloning, Cold Sring Harbor laboratory, p. 250, 1982) dans E. coli JM83 (Vieira J. et Messing, J., Gene 19, 259, 1982), afin d'obtenir des clones ApR (résistants à l'ampicilline), car le plasmide pUC13 porte les déterminants de la résistance à l'ampicilline.

4)Identification des clones recombinants porteurs de l'ADNc du virus de la SHV :

La recherche des clones recombinants porteurs d'ADNc viraux s'est faite par hybridation sur colonies ApR répliquées sur filtres de nitrocellulose (Maniatis op. cit. 313, 315, 326, 328).

Une réplique est hybridée avec une sonde ADN faite à partir d'ARN de cellules infectées par le virus de la SHV, marquée au 32P. 1 à 2$\mu$g d'ARN dans 8$\mu$l d'eau sont chauffés 3 minutes à 100°C puis refroidis

EP 0 377 349 B1

sur de la glace.

Les conditions d'hybridation relatives à cette étape sont les suivantes :
- on réalise dans un premier temps une pré-hybridation pendant 4 à 24 heures, entre 37°C et 68°C dans un tampon constitué par 5XSSC, 5X Denhardt, 0,5 % SDS, 100$\mu$g/ml d'ADN de sperme de saumon. A titre de rappel, 1XSSC comprend 0.15 M de NaCl, 0,015 M de citrate de Na à pH 7,0.

1X Denhardt comprend du Ficoll (1‰), de la polyvinylpyrrolidone (1‰) et de la sérumalbumine de boeuf (1‰).

Après la pré-hybridation, on réalise une hybridation dans le même milieu en ajoutant la sonde marquée radioactivement. On réalise l'hybridation pendant un temps allant de 30 minutes à 24 heures.

On procède ensuite à deux lavages de 15 minutes à température ambiante dans 2X SSC, 0,1 % SDS, puis à un lavage d'une heure à 65°C dans 1X SSC, 0,1 % SDS.

Ensuite, on réalise une exposition des filtres dans une cassette avec écran amplificateur, de 2 heures à 15 jours à -70°C.

Le marquage de la sonde est fait dans un volume de 20$\mu$l de Tris-HCl 50 mM, pH8, MgCl$_2$ 6 mM, KCl 40mM et dGTP, dATP, dTTP 1 mM, RNasin 10 U., 2$\mu$g hexamère statistique et 50 microCie de dCTP 32P à 400 Cie/mM et 10 unités de transcriptase reverse pendant 30 minutes à 42°C. La réaction est arrêtée avec 1$\mu$l d'EDTA 0,25M. Le dCPT 32P non incorporé est éliminé par chromatographie sur colonne G50 (Pharmacia).

Au mélange contenant l'ADN marqué, on ajoute une quantité d'EDTA (5mM) et de NaOH (0,1M), puis on chauffe 40 minutes à 65°C pour éliminer l'ARN. On ajoute ensuite au mélange 0,2M de tris et on neutralise avec HCl1M. L'hybridation se fait avec 50.000 à 1.000.000 de dpm/ml de sonde en présence de 70$\mu$g/ml d'ARN de cellules de manière à limiter l'hybridation avec les ADNc provenant de l'ARN cellulaire.

Une seconde réplique est hybridée de la même manière avec une sonde faite à partir d'ADN de cellules non infectées. Les clones recombinants qui contiennent des ADNc d'origine virale ne seront marqués que par la sonde provenant de l'ARN des cellules infectées. A l'opposé, les clones qui contiennent des ADNc d'origine cellulaire seront marqués sur les deux répliques. Au total 1632 clones ont été examinés de cette manière, ce qui a permis de distinguer 164 clones suspectés d'être d'origine virale.

<u>5)Identification d'un recombinant porteur de l'ADNc de la protéine G du virus de la SHV</u>

Des sous-populations d'ADNc ont été identifiées en réalisant une hybridation entre les inserts de certains clones recombinants marqués au 32P et tous les autres recombinants ainsi qu'avec des Northern Blot d'ARN de cellules infectées ou témoins.

Une première population est caractérisée par le recombinant n° 54 qui reconnaît un ARN messager d'une longueur de 1,6 kb présent uniquement dans les cellules infectées. Une deuxième population, en quantité inférieure à celle de la première population est caractérisée par le recombinant n° 77 (caractérisé par le plasmide pSHVG1) qui reconnaît un messager de 2 kb présent lui aussi dans les cellules infectées.

Dans une étape suivante, les plasmides de 164 clones recombinants ont été purifiés (Maniatis op. cit. 368) et les inserts ont été libérés par deux enzymes de restriction dont les sites se trouvent de part et d'autre du site de clonage Sma1 du plasmide pUC13. 10$\mu$l (0,1 à 2$\mu$g) ont été digérés pendant 2 heures à 37°C dans le tampon Med (Tris-HCl10mM, pH 7,5 MgCl$_2$ 10 mM, NaCl 50 mM) avec 5 unités de Hind III et 5 unités de EcoR1.

Les fragments ont ensuite été séparés par électrophorèse en gel d'agarose. Les inserts de quelques recombinants dont la taille est égale ou supérieure à 1,5 kb ont été découpés de l'agarose et élués à travers une membrane Genescreen (NEN-DuPont) comme décrit par Zhu, J. et al. (Biotechnology <u>3</u>, 1014, 1985) et marqués au 32P suivant le protocole du kit Boerhinger (Random Priming DNA labeling kit 1004760).

De 20 à 500 ng de chacun des 164 plasmides recombinants ont été déposés sur une membrane Genescreen (NEN-DuPont) dans NaOH 0,4M, NaCl 0,6M.

Les membranes ont été neutralisées dans un mélange Tris-HCl pH70,5M, NaCl 0,6M, séchées 1 heure à température ambiante puis hybridées comme ci-dessus.

En parallèle, 10$\mu$g d'ARN de cellules infectées ou témoins ont été séparés sur gel d'agarose après dénaturation au glyoxal et transférés sur membranes de nitrocellulose (Maniatis et al. op. cit 200-201).

Les membranes ont été hybridées avec les sondes produites à partir des inserts d'ADN recombinants.

La figure I montre l'hybridation obtenue avec les inserts du clone 54 (premier cadre) et du clone 77 (deuxième cadre) et des clones 54 et 77 (troisième cadre). L'hybridation en présence d'ARN provenant d'une part de cellules infectées (i), d'autre part de cellules témoins (c) n'a lieu qu'avec les cellules infectées. Le clone 54 donne une bande de 1,6 kb et le clone 77 (correspondant au plasmide pSHVG1) donne une bande de 2kb.

EXEMPLE II :SEQUENCAGE DE L'ADNc de pSHVG1 :

Le séquençage a été réalisé sur les deux brins de l'ADN par la méthode de Sanger et al. (Proc. Nat. Acad. Sci. USA 74, 5463, 1977), (Step-by-step protocole for DNA sequencing with Sequenase United States Biochemical Corporation) après sous-clonage dans M13mp18 et M13mp19 (Yanisch-Perron et al. Gene 33, 103, 1985). Le principe du séquençage est présenté sur la figure II, dans laquelle les flèches avec une astérisque représentent l'amorce universelle de M13 et les flèches simples représentent les oligomères synthétiques utilisés comme amorce. Les premières séquences ont été obtenues à partir de l'amorce universelle de M13, ensuite des amorces spécifiques (constituées de 18 résidus) ont été synthétisées (méthode phosphoramidite, synthétiseur d'ADN Cyclone) afin de progresser de proche en proche dans la séquence.

La séquence nucléotidique obtenue est donnée sur la figure III, dans laquelle les acides nucléiques sont mis en correspondance avec les acides aminés. Elle présente une phase ouverte de lecture de 1556pb qui commence par le codon ATG au résidu 426. Un second ATG, en phase, au résidu 460 pourrait être le codon d'initiation fonctionnel in vivo car son environnement ACAATGG est plus proche de l'idéal théorique ACCATGG. D'autre part, l'étude du profil d'hydrophobicité (figure IV) révèle l'existence d'une séquence signal qui commence directement après ce deuxième ATG et est clivée après le résidu Pro en position 520. Plus précisément, la figure IV représente le profil hydrophobe-hydrophile de la séquence d'acides aminés codé par la susdite séquence nucléotidique. Les parties hydrophobes sont situées au-dessus de la ligne horizontale représentée sur la figure IV et les parties hydrophiles sont situées au-dessus de cette ligne. On remarque la présence de deux régions particulièrement hydrophobes. La première correspond au peptide signal qui est clivé à la maturation de la protéine. On observe également la présence d'une région hydrophile.

On a représenté par des flèches les cinq sites de glycosylation potentiels, dont un site est à proximité de la séquence signal et les 4 autres du côté carboxyterminal.

On rappelle que des sites de glycosylation sont soit Asn-X-Thr ou Asn-X-Ser, dans lesquels X représente un acide amine quelconque.

EXEMPLE III :EXPRESSION SOUS FORME DE PROTEINE FUSION DANS E. COLI :

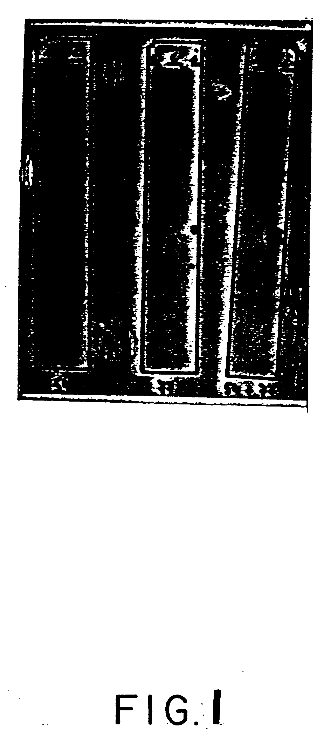

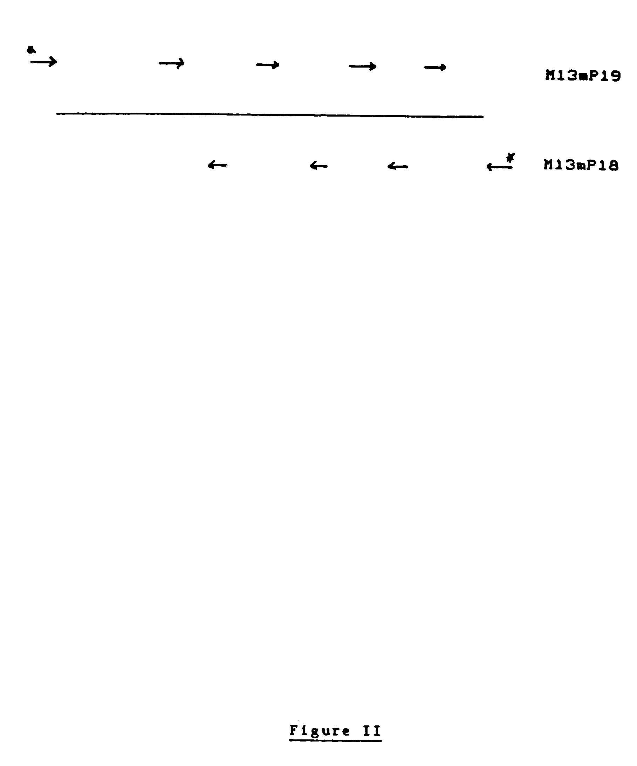

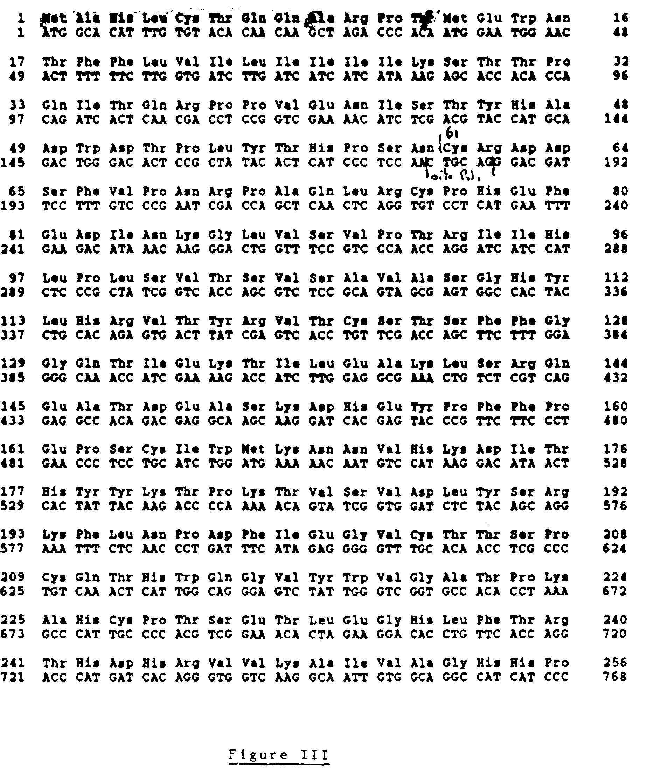

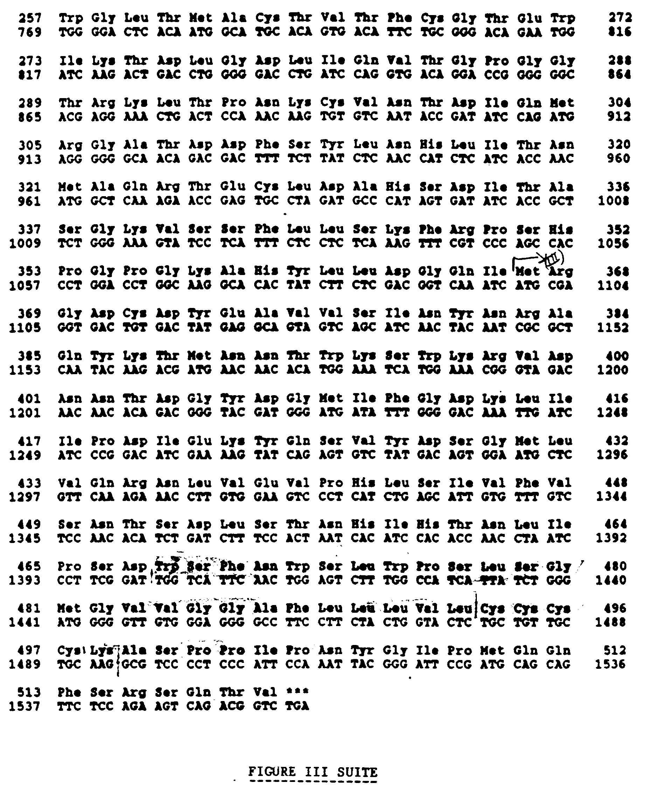

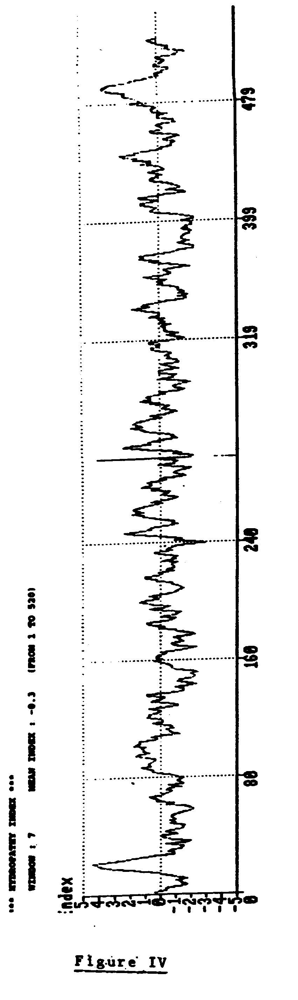

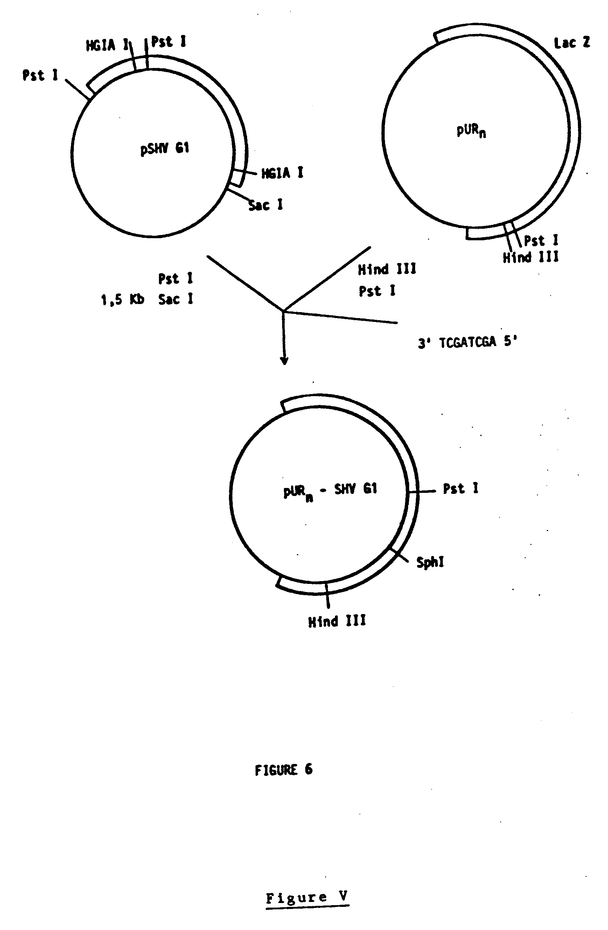

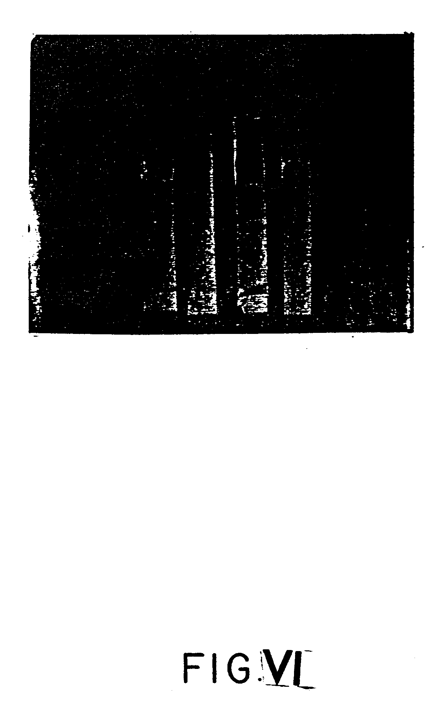

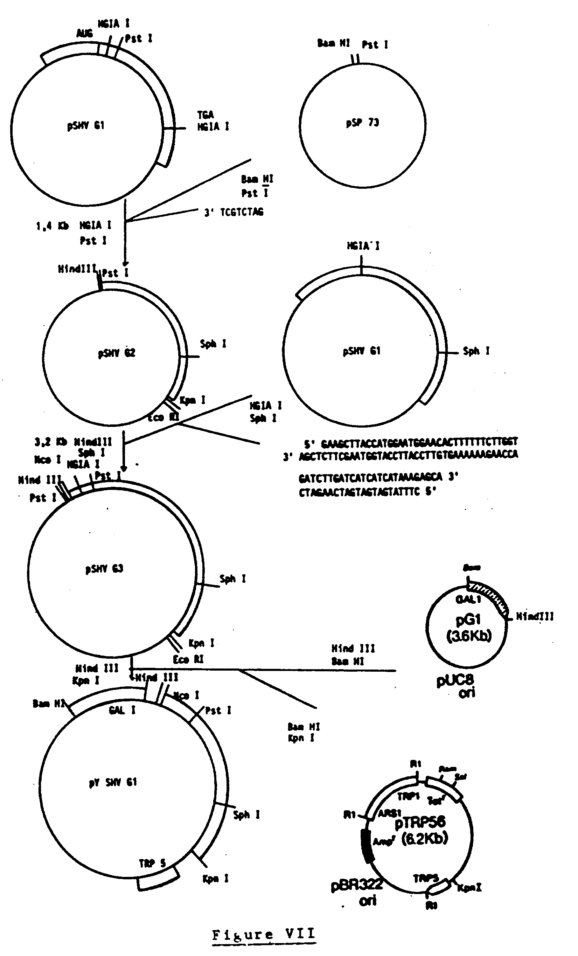

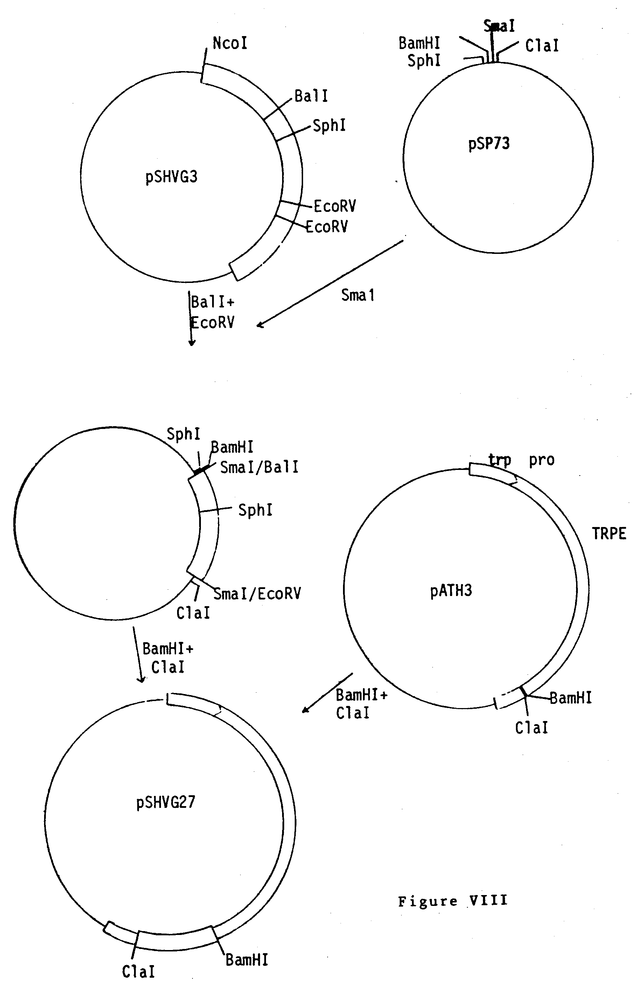

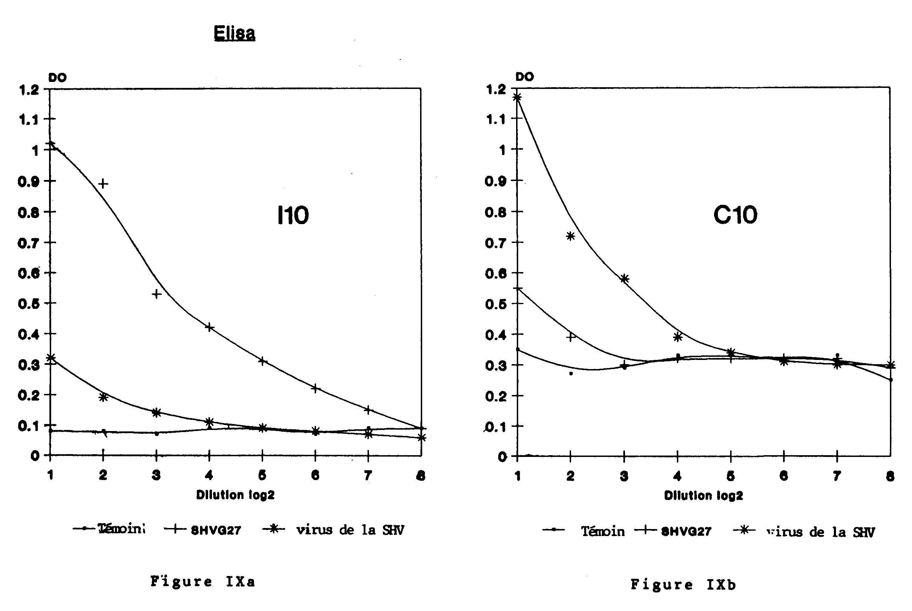

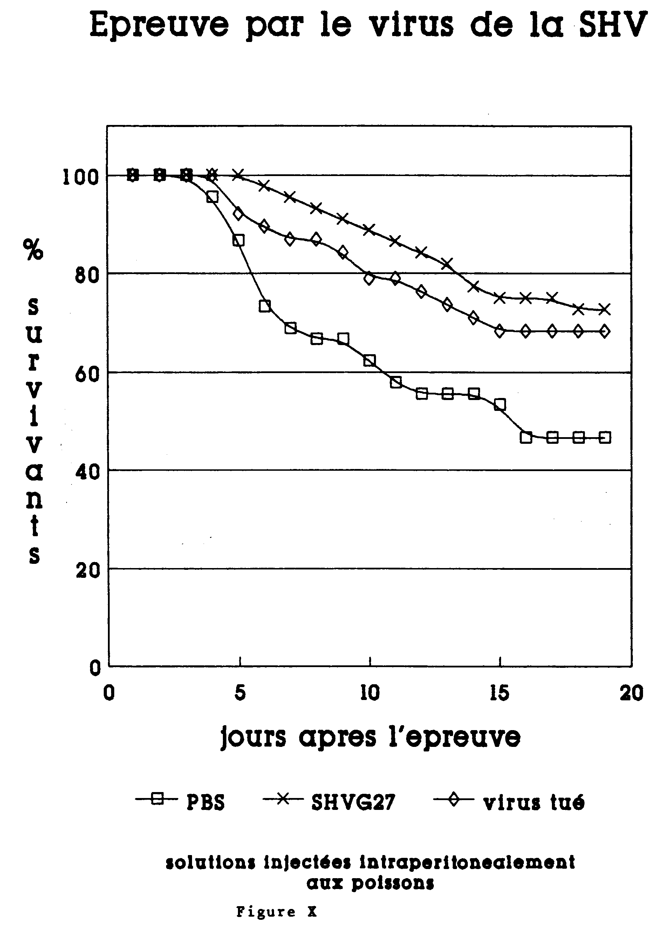

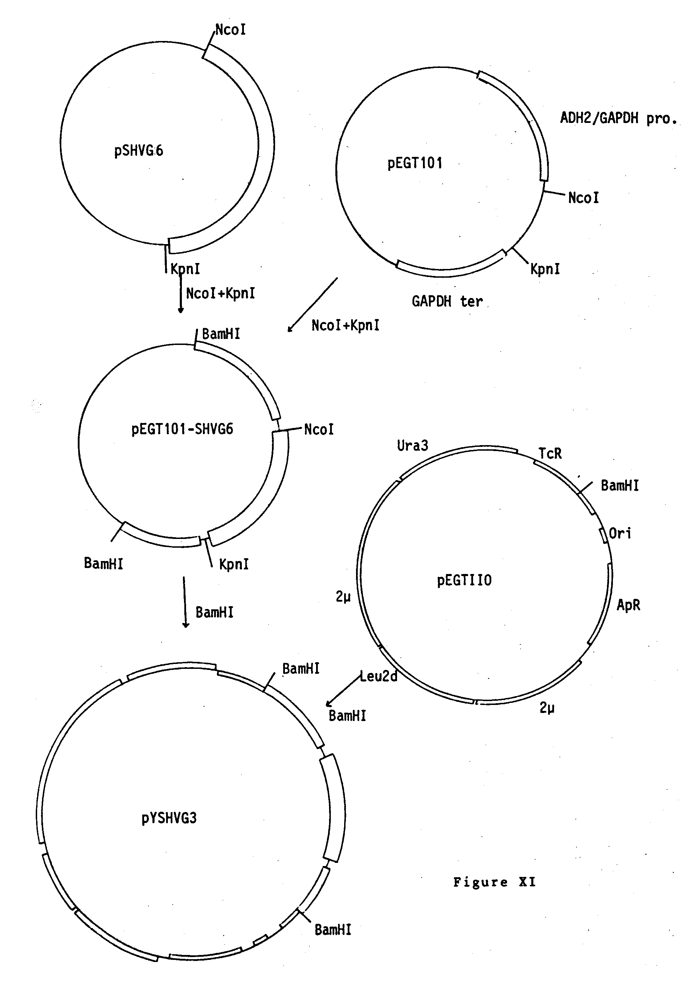

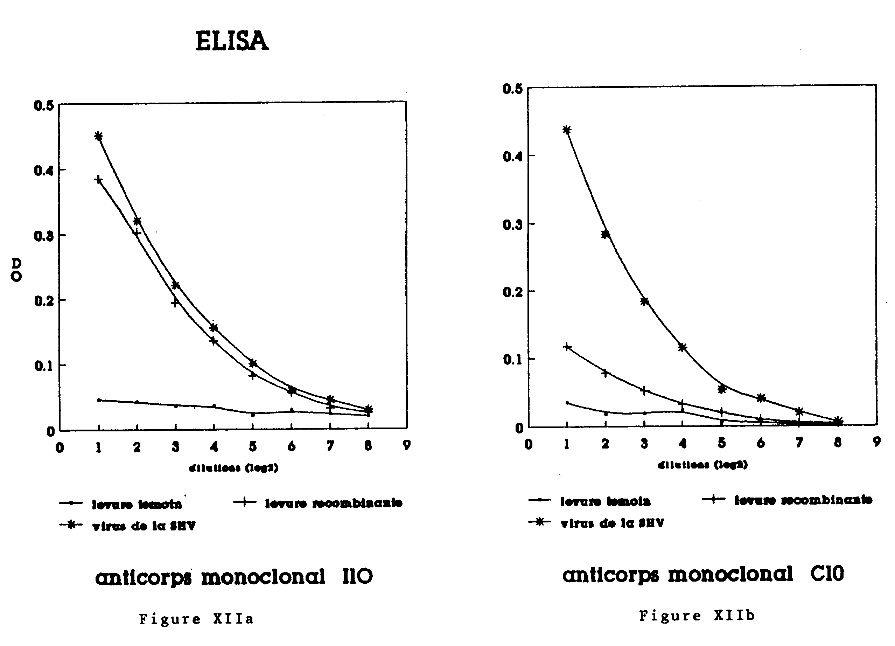

Les plasmides de la série des pUR 290-291 (désignés de façon générale par $pUR_n$) de Ruther et Muller-Hill (EMBO jour. 2, 1791, 1983) ont été utilisés. Ils permettent d'insérer l'ADN à exprimer dans la partie terminale du gène de la $\beta$-galactosidase de E. coli grâce à des sites de restriction multiples (figure 7).

La phase ouverte de lecture de pSHVG1 comprend un site Pst1 en position 604, soit 48 acides amines après le codon d'initiation probable. L'insertion dans pUR290 fournit une protéine fusion en phase, tandis que dans pUR291 un codon stop apparaît 17 acides aminés plus loin.

2µg de pSHVG1 sont digérés pendant 2 heures à 37°C dans 20µl du tampon low (Tris-HCl 10 mM, pH7,5, $MgCl_2$ 10 mM et dithiothréitol 1 mM) par 5 unités de Sac1 et 5 unités de Hind3. Les fragments sont séparés sur gel d'agarose et élués comme décrit plus haut. Parallèlement 1µg respectivement de pUR290 et de pUR291 sont digérés 2 heures à 37°C dans 20µl de tampon Med par 5 unités de Pst1 et 5 unités de Hind3. La réaction est arrêtée par une extraction au chloroforme.

20 ng de pUR coupé par Pst1 et Hind3, 10 ng du fragment Pst1 Sac1 de pSHVG1 et 1 ng de l'oligonucléotide ci-dessous :

```
        Sac1                        Hind

  ...............             ...............
5' G   A   G   C   T       A   G   C   T   T   3'
          C                              A
3'T   C   G   A            T   C   G   A   5'oligonculéotide
```

sont incubés pendant 2 heures à température ambiante dans 10µl du tampon de ligation, en présence d'une unité de ligase de T4 et de 5 unités de polynucléotide kinase. La construction de ces plasmides pURn-SHVG1 est rapportée sur la figure V.

Les deux mélanges de ligation sont utilisés tels quels pour transformer E. coli JM83 pour obtenir des colonnes ApR.

Les structures de pUR290-SHVG1 et pUR291-SHVG1 sont authentifiées après minipréparation à partir d'un clone purifié par la digestion par les enzymes Hind3, Pst1, EcoR1 et Sph1 et électrophorèse en gel d'agarose.

Un clone contenant pUR290-SHVG1 est mis en présence de 5 ml de milieu LB avec agitation à 37°C jusqu'à la densité optique de 0,6 à 650 nm. La culture est alors ramenée à 1mM en isopropyl-$\beta$-D-thiogalactopyranoside (IPTG) et cultivée de la même manière pendant 20 heures. Les bactéries sont ensuite centrifugées et lysées 10 minutes à 100°C dans le tampon de chargement (Tris HCl 60 mM pH6,8 SDS 2% glycérol 10% 2-mercapto-éthanol 3%) et soumises à l'électrophorèse sur gel de polyacrylamide SDS (Laemli Nature 227, 680, 1970). Les protéines sont ensuite transférées sur une membrane de nitrocellulose. Les membranes sont traitées successivement par deux anticorps monoclonaux anti-protéine G du virus d'Egtved reconnaissant la protéine G respectivement dans sa conformation naturelle (C10) et dans sa structure primaire (I10), puis par un anticorps antisouris couplé à la peroxidase. Les anticorps monoclonaux anti-protéine G, désignés par C10 et I10, ont été décrits par Bearzotti-Le Berre et de Kindelin en 1987 dans "European association of fish pathologists" 3nd international conference abstracts, suite au Congrès de Bergen (Norvège) d'Août 1987. Les complexes antigène-anticorps formés sont révélés par une réaction colorée (Towbin H. et al. Proc. Nat. Acad. Sci. USA 76, 4350, 1979) (BioRad Immunoblot Detection kit technical litterature n° 1216).

La même procédure est appliquée à un clone contenant le plasmide pUR291-SHVG1.

La présence de deux bandes colorées uniquement dans les canaux contenant les protéines produites par le clone pUR290-SHVG1 (figure VI) prouve que ce plasmide contient un ADNc codant pour une séquence d'acides aminés reconnue par des anticorps monoclonaux dirigés contre la protéine G du virus de la SHV.

EXEMPLE IV :CONSTRUCTION D'UN PLASMIDE RECOMBINANT pYSHVG1 CODANT POUR UNE SEQUENCE D'ACIDES AMINES RECONNUE PAR LES ANTICORPS MONOCLONAUX DIRIGES CONTRE LA PROTEINE G DU VIRUS DE LA SHV :

Comme vecteur de l'ADN dans la levure n'importe quel plasmide navette (obtenu de E. coli, d'une levure) peut être utilisé pour autant que l'ADN qu'il contient soit exprimé dans la levure. Le vecteur comprendra un élément susceptible d'assurer son maintien dans la levure comme l'origine de réplication du plasmide 2$\mu$ ou une Ars comme celle du gène Trp1. Il portera en outre le gène de la biosynthèse d'un acide aminé susceptible de complémenter une mutation dans la souche réceptrice et d'assurer la sélection positive du plasmide transformant, par exemple Leu2 ou Trp1. Enfin, il portera un promoteur inductible en aval duquel sera inséré le gène à exprimer par exemple le promoteur de la galactokinase (pG1) celui de l'alcool déshydrogénase (pA1) Miyajima, A. et al. Nuc. Ac. Res. 12, 6397 (1984) ou celui de la phosphatase acide (pPho5, Richard, A. et al. Proc. Nath. Acad. Sci. USA 81, 367 (1984).

1$\mu$g de plasmide pSHVG1 est digéré pendant 2 heures à 37°C dans 20$\mu$l de tampon high (Tris-HCl, 50mM pH7,5, MgCl$_2$ 10 mM, NaCl 100 mM, dithiothréitol 1mM) avec 5 unités de Pst1 et 5 unités de HgiA1. Les fragments sont séparés sur gel d'agarose et le fragment d'approximativement 1,5 kb Pst1-HgiA1 est récupéré par élution sur Genescreen comme décrit plus haut.

1$\mu$g de plasmide vecteur pSP73 (Promega) est digéré dans 20$\mu$l de tampon high ci-dessus, avec 5 unités de Pst1 et 5 unité de BamH1 pendant 2 heures à 37°C. La réaction est arrêtée par une extraction au chloroforme.

100ng du fragment Pst1-HgiA1 sont ligués avec 200ng du plasmide pSP73 ouvert par BamH1 et Pst1 dans 10$\mu$l du tampon de ligation avec 1 unité de ligase et 6 unités de polynucléotide kinase pendant 2 heures à température ambiante en présence de l'oligonucléotide suivant destiné à relier les sites de clivage HgiA1 et BamH1.

```
3'  T  C  G  T    C  T  A  G  5' oligonucléotide

    C                       G

5' G  A  G  C  A   G  A  T  C  C  3'

.................    ................
      HgiA1                BamH1
```

Le plasmide pSHVG2 est introduit par transformation dans E. coli HB101 (J. Molec. Biol. 41 459 1969) afin d'obtenir des colonies ApR.

Un plasmide pSHVG2 a été produit à partir d'une des colonies et sa structure a été confirmée par la digestion par les enzymes EcoR1, Sph1, Hind3 et Pst1.

Un µg du plasmide pSHVG2 est digéré 2 heures à 37°C dans 20µl de tampon Med en présence de 5 unités de Sph1 et de 5 unités de Pst1. Les fragments ont été séparés sur gel d'agarose et le fragment de 3,2 kb est récupéré comme ci-dessus.

10µg du plasmide pSHVG1 sont digérés 2 heures à 37°C dans 100µl de tampon high avec 5 unités de Sph1 et 5 unités de HgiA1. Les fragments sont séparés sur gel d'agarose et le fragment de 700pb Sph1-HgiA1 est récupéré.

1µg de ce fragment est incubé avec 0,1µg du linker ci-dessous :

```
5'GAAGCTTACCATGGAATGGAACACTTTTTTCTTGGTGATCTTGATCATCATCAT

3'AGCTCTTCGAATGGTACCTTACCTTGTGAAAAAAGAACCACTAGAACTAGTAGTAGTA

......../....../......
 Pst1   Hind3  Nco1

   AAAGAGCA  3'
   TTTC      5'

   .....
   HgiA1
```

qui reprend la séquence naturelle du gène de la protéine G du virus d'Egtved depuis le site HgiA1 jusqu'à l'ATG d'initiation. Trois sites de restriction ont été ajoutés, 1 site Nco1 qui recouvre le 1er ATG, 1 site HindIII et enfin l'extrémité cohésive de Pst1.

Le fragment et le linker sont incubés en présence de 1 unité de ligase et de 6 unités de polynucléotide kinase 2 heures à température ambiante.

Le produit de la ligation est digéré à 37°C dans le tampon med par 5 unités de HindIII et 5 unités de Sph1, soumis à l'électrophorèse en gel d'agarose et la bande de 760pb est récupérée. 100 ng de ce fragment Sph1-Pst1 sont incubés avec 200 ng du fragment de 3,2 kb HindIII-Sph1 de pSHVG1 dans 10µl du tampon de ligation avec 1 unité de ligase 2 heures à température ambiante. Le mélange de ligation est utilisé ensuite pour transformer E. coli HB101 afin d'obtenir des colonies ApR. Le plasmide pSHVG3 de telles colonies est extrait et digéré par EcoR1, HindIII, Nco1, HgiA1, Sph1 et Pst1 afin de vérifier l'exactitude de la construction. La région contenant le linker est séquencée.

2µg du plasmide PSHVG3 sont digérés dans le tampon low par 5 unités de Kpn1 et 5 unités de HindIII.

Les produits de la digestion sont séparés par électrophorèse sur gel d'agarose et le fragment d'approximativement 1,5 kb est récupéré.

2µg du plasmide pTRP56 (Miyajima, A. op. cit.), sont digérés pendant 2 heures à 37°C dans le tampon low par 5 unités de Kpn1 et 5 unités de BamH1. Le fragment de 4,5 kb est récupéré d'un gel d'agarose.

2µg du plasmide pG1 (Miyajima, A. op. cit.), dans le tampon low par 5 unités de BamH1 et 5 unités de Hind3. Le fragment de 800 pb est récupéré d'un gel d'agarose.

100ng respectivement des 3 fragments ci-dessus sont incubés 2 heures à température ambiante dans le tampon de ligation avec 1 unité de ligase. Le produit de la ligation est introduit dans E. coli HB101 par transformation. L'exactitude de la construction du plasmide résultant SHVG1 a été contrôlée par la digestion par Kpn1, Sph1, Pst1, HindIII et BamH1 et le séquençage.

La construction du plasmide pYSHVG1 est rapportée sur la figure VII.

Les cellules de levure W303-1B ont été transformées par la méthode au LiCl (Ito, H. et al. J. Bact. 153, 163 (1983) et sélectionnées pour la complémentation par le plasmide pY-SHVG1 de la mutation TRP1 de cette souche. Les levures transformées se sont montrées capables de pousser sur le milieu minimal yeast Nitrogene base sans acides aminés (commercialisé par DIFCO) supplémenté avec de l'uracile, de la leucine, de l'uridine et de l'adénine à $20\mu g/ml$.

Un clone de la levure porteur du plasmide pY-SHVG1 a été cultivé dans 20 ml de milieu minimal avec addition de 20 g/l de glucose pendant 24 heures à 30°C avec agitation. La culture a ensuite été centrifugée aseptiquement et resuspendue dans 20 ml du milieu minimal avec addition de 20 g/l de galactose afin d'induire l'opérateur Gall. Après 6 heures de culture avec agitation à 30°C les levures ont été centrifugées et resuspendues dans 2 ml de tampon phosphate 40 mM pH7. Elles ont été brisées dans un disrupteur à bille de verre pendant 5 minutes. Une partie aliquote des cellules a alors été resuspendue dans le tampon de chargement et soumise à une électrophorèse en gel de polyacrylamide-SDS. Après transfert sur membrane de nitrocellulose la présence d'une séquence d'acides aminés reconnue par les mêmes anticorps monoclonaux que ceux définis précédemment a été mise en évidence.

On peut également cultiver la levure à une température inférieure, comprise entre 0 et 30°C, avantageusement de 14 à 17°C pour favoriser la stabilité de la protéine G. On peut aussi cultiver la levure à 30°C jusqu'à l'introduction de manière à accumuler aisément la biomasse et abaisser la température lors de l'induction.

EXEMPLE V: CONSTRUCTION D'UN PLASMIDE RECOMBINANT pSHVG27 A PARTIR DES PLASMIDES pATH (DIECKMAN & TZAGAZLOFF, 1985, J. Bio. Chem. 260, 1513-1520) CODANT POUR UNE PROTEINE FUSION (SHVG27) CONTENANT UNE SEQUENCE D'ACIDES AMINES RECONNUE PAR LES ANTICORPS MONOCLONAUX DIRIGES CONTRE LA PROTEINE G DU VIRUS DE LA SHV:

réplicon: Co1E1, multicopie
résistance: Ampicilline
promoteur: trp
inducteur: AIA (acide indoleacrylique) ou carence en tryptophane
milieu de culture: M9 dont la composition par litre est la suivante:
6 g $Na_2HPO_4$ - $H_2O$
3 g $KH_2PO_4$
0.5 g NaCl
1g $NH_4Cl$
5 g casamino acids
1 ml 1 M $MgSO_4$
0.2 ml 0.5 M $CaCl_2$
5 ml 40% glucose
10 ml 1 mg/ml thiamine B1
avec ou sans tryptophane, à raison de
2 ml 10 mg/ml trytophane
protéine fusion avec: anthranylate synthetase (TRPE)

La construction du plasmide pSHVG27 est représentée sur la figure VIII.

Dans ce cas, une séquence d'acides aminés reconnue par les anticorps monoclonaux dirigés contre la protéine G du virus de la SHV est fusionnée avec les 37KD de l'anthranylate synthetase de E. coli. Une séquence située à la fin du cadre de lecture de la susdite séquence d'acides aminés contient de multiples sites de restriction qui permettent d'insérer une séquence d'ADN à exprimer sous forme de protéine fusion avec l'anthranylate synthétase. Trois plasmides différents pATH1, 2 et 3 permettent de choisir entre les trois phases de lecture possibles.Dans l'exemple présent, le domaine de la protéine G qui est inséré ne comprend ni peptide signal ni région transmembranaire. Cette région s'étend depuis l'acide aminé 111 de la séquence 1, jusqu'à l'acide aminé 302. En pratique, le fragment d'ADN découpé de la séquence codante sur le plasmide pSHVG3 par l'action des enzymes de restriction Bal1 et EcoRV, qui code pour la partie de la protéine comprise entre les résidus 111 et 302, a été inséré par ligation au site Sma1 du plasmide pSP73 (Promega). Ces trois enzymes de restriction produisent des extrémités franches compatibles entre elles. Les plasmides recombinants ont été introduits dans E. coli HB101 par transformation. Le recombinant pSHVG26 a été sélectionné parmi les plasmides recombinants issus de cette transformation parce qu'il portait l'insert dans l'orientation BamH1 -->Cla1 par rapport à ces sites présents dans le plasmide pSP73. La découpe de ce plasmide par l'enzyme Sph1 permet d'orienter l'insert. En effet il y a deux sites Sph1, le premier se trouve dans le plasmide pSP73 à proximité du site BamH1, le second se trouve dans l'insert à environ 220 paires de bases du site Bal1, qui se trouve au début de l'insert. pSHVG26 se caractérise à la digestion par Sph1 par l'apparition d'un fragment d'environ 200 pb. L'orientation inverse se caractériserait par un fragment de 360 pb. L'insert de pSHVG26 a été découpé par les enzymes BamH1 et Cal1 et inséré dans le plasmide pATH3 ouvert par les mêmes enzymes afin de mettre l'insert en phase avec le gène de l'anthranylate synthétase. Le signal stop de la protéine est fourni par la séquence terminale de l'anthranylate synthétase. Le plasmide recombinant pSHVG27 ainsi caractérisé a été introduit dans E. coli par transformation, le plasmide a été vérifié par la digestion par les enzymes BamH1 et Cla1. La protéine fusion, dénommée SHVG27, codée par ce plasmide est en théorie de 59kD.

La production se fait après culture de nuit dans le milieu minimal M9 complémenté avec du Casamino acid (0,5%), (un hydrolysat acide de caséine qui contient tous les acides aminés à l'exception du tryptophane), plus du tryptophane destiné à réprimer le promoteur. La culture est ensuite diluée 20 fois dans du milieu frais, carencé en tryptophane. Après une heure de culture, le promoteur est induit avec 5mg/l d'AIA. La production est maximale deux heures plus tard. La protéine fusion SHVG27 se présente sous la forme de corps d'inclusion insolubles. En électrophorèse sur gel de polyacrylamide, la protéine induite possède une taille apparente de 58kD.

Les rendements de production sont satisfaisants, la protéine fusion représente plus de 10% des protéines totales. Il est relativement aisé de purifier les corps d'inclusion; après avoir cassé les cellules à la presse de French, les corps d'inclusion sont centrifugés à basse vitesse (3000g, 10 min) et resuspendus dans du PBS. On procède de la sorte à 6 lavages puis on solubilise les corps d'inclusion dans l'urée 6M. Les protéines sont dialysées contre des concentrations décroissantes d'urée et enfin contre du PBS. A la fin de ce processus de dénaturation-renaturation, la protéine SHVG27 représente 35% des protéines en solution. Le rendement, pour une fermentation non optimisée est de 20mg/l.

La protéine SHVG27 renaturée est reconnue en ELISA par l'anticorps monoclonal C10 (fig. IXb), qui reconnaît la conformation naturelle de la protéine G du virus de la SHV et par l'anticorps monoclonal I10 (fig.IXa), qui reconnaît la structure primaire. La protéine témoin est le partenaire bactérien de la protéine fusion, l'anthranylate synthétase. La technique utilisée pour l'ELISA est classique:

1) l'anticorps monoclonal est adsorbé sur la plaque de titrage ELISA pendant une nuit à la dilution 1/200 dans un tampon contenant pour un litre 1,56g de $Na_2CO_3$, 2,93g de $NaHCO_3$ et 0,2g de $NaN_3$.

2) des dilutions de 2 en 2 de la solution contenant soit la protéine fusion (200 microg/ml) soit de l'anthranylate synthétase (200 microg/ml) soit du virus (c'est-à-dire 600 ng/ml de protéine G) en solution dans le PBS + 0,05% de Tween 20 + 2% de sérum de cheval sont déposées dans les plaques et incubées deux heures à température ambiante; on procède à 5 rinçages avec du PBS + 0,05% de Tween 20.

3) un anticorps polyclonal de lapin dirigé contre le virus de la SHV est ensuite disposé dans chaque puits de la plaque à la dilution 1/200 dans le PBS + 0,05% de Tween 20 + 2% de sérum de cheval, après 2 heures on rince comme ci-dessus.

4) un anticorps commercial dirigé contre les immunoglobulines de lapin, couplé à la peroxydase est ensuite ajouté dans le tampon ci-dessus à la dilution 1/500; après une heure on rince.

5) on procède à une réaction colorée de l'activité de la peroxydase dans 50 microl de 0,4% de dihydrochlorure d'o-phenylenediamine (Sigma 8787), 0,012% d'$H_2O_2$ dans un tampon phosphate-citrate pH5, après 15 minute on arrête la réaction avec 150 microl d'$H_2SO_4$ 2M et on mesure la densité optique à 402nm.

Sur les Figures IXa et IXb, l'axe des abscisses correspond au log 2 de la dilution et l'axe des ordonnées à la densité optique (DO). La courbe représentée par des croix (+) correspond à la réponse obtenue avec la protéine fusion (SHVG27) de l'invention.

La courbe représentée par des étoiles (*) correspond à la réponse obtenue avec le virus de la SHV (600mg/ml de protéine G).

La courbe représentée par des points (.) correspond à la réponse obtenue avec le témoin (anthranylate synthétase).

Le résultat indique en outre que des épitopes importants pour la neutralisation du virus, puisque reconnus par l'anticorps monoclonal neutralisant C10 sont contenus dans la séquence peptidique comprise entre les résidus 111 et 302, et que la glycosylation n'est pas indispensable pour cet épitope.

EXEMPLE VI: TEST DE VACCINATION A L'AIDE DE LA PROTEINE SHVG27:

Un essai de vaccination a été effectué avec la protéine SHVG27. Des lots de 45 alevins de truites arc-en-ciel, pesant approximativement 1g, ont été injectés intrapéritonéalement avec 20 microl des solutions suivantes:

1) du PBS + 10% d'adjuvant complet de Freund (ACF),

2) le virus de la SHV inactivé par la propionolactone, à raison de 1,6microg/ml c'est-à-dire 10ng de protéine G + 10% ACF,

3) la protéine SHVG27 à raison de 108 microg/ml c'est-à-dire une quantité de protéine reconnue par l'anticorps monoclonal C10 en ELISA évaluée à 7,65ng/alevin.

L'épreuve est faite trois semaines plus tard en ajoutant 50.000 unités formant plage de virus de la SHV de type I par ml de l'eau de l'aquarium. L'alimentation en eau fraîche est coupée pendant 1 heure pour permettre l'infection. L'alimentation est ensuite rétablie et les particules virales non adsorbées sont progressivement diluées. Les mortalités sont enregistrées dans les trois semaines qui suivent.

Le niveau de mortalité atteint par les poissons témoins, injectés par le PBS, varie d'une expérience à l'autre en fonction de la sensibilité des différents lots d'alevins. Dans cette expérience la mortalité des témoins atteint 56%, alors que le virus tué, le meilleur vaccin actuellement disponible, protège avec seulement 32% de morts, soit un coefficient de protection de 43%. Les alevins injectés avec la protéine SHVG27 enregistrent seulement 27% de morts, soit un coefficient de protection de 52%. Il faut aussi noter le retard à la mortalité enregistré dans les deux lots vaccinés par rapport au lot témoin.

La fig. X représente le pourcentage de poissons survivants qui ont été mis en contact par balnéation avec le virus de la SHV, 3 semaines après qu'on leur ait injecté,

- du virus de la SHV tué (courbe représentée par des losanges (◊),
- de PBS (courbe représentée par des carrés (□),
- de la protéine de l'invention (SHVG27):(courbe représentée par des croix (x).

Ce pourcentage est exprimé en fonction des jours écoulés depuis la balnéation, considéré comme jour 0.

## EXEMPLE VII: EXPRESSION EN LEVURE:

On a préféré un vecteur d'expression inductible pour la levure. Les caractéristiques de ce vecteur sont les suivantes:

- la réplication et le maintient dans la bactérie sont assurés par pBR322, et la résistance à l'ampicilline,
- la réplication dans la levure est assurée par les fonctions du 2µ,
- la sélection dans la levure se fait par complémentation d'une auxotrophie de la souche hôte par les gènes ura3 ou leu 2d (d pour déficient); ces deux gènes permettent d'étudier l'influence du nombre de copies sur la production de la protéine d'intérêt, car ura3 est un allèle sauvage du gène muté, la complémentation est efficace, le nombre de copies du vecteur est minimal, le gène leu 2d est très peu traduit, pour assurer la biosynthèse de leucine indispensable pour sa croissance la cellule n'a qu'une possibilité augmenter le nombre de copies du gène leu2d et par conséquent le nombre de copies du vecteur,
- le promoteur résulte de la fusion des séquences régulatrices (URS, Upstream Regulating Sequences) de ADH2, la gène de l'alcool déshydrogenase 2 qui est inductible par l'alcool, et le promoteur GAPDH, glyceraldehyde phosphate deshydrogenase, qui est un des promoteurs constitutifs les plus puissants de la levure, ce promoteur hybride combine la force de GAPDH avec l'inductibilité de ADH2,
- le terminateur du gène GAPDH.

Le cDNA retravaillé de la séquence d'acides aminés reconnue par les anticorps monoclonaux dirigés contre la protéine G du virus de la SHV présent sur le plasmide pSHVG3 a été inséré entre le promoteur ADH2-GAPDH, grâce au site Nco1 placé sur l'ATG initiateur, et le terminateur du plasmide intermédiaire pEGT101 grâce au site Kpn1 situé après le codon stop et introduit par transformation dans E. coli. Les caractéristiques des plasmides obtenus pEGT101-SHV ont été vérifiées par la digestion par les enzymes de restriction Nco1 et Kpn1 qui libère le cDNA de la susdite séquence d'acides aminés sous la forme d'une bande de 1,5kb. La cassette BamH1 contenant le promoteur, le gène de la protéine G et le terminateur est à son tour introduite au site unique BamH1 du vecteur navette E. coli-levure pEGT110, les plasmides sont introduits par transformation et la présence de la cassette dans le vecteur navette est vérifiée par la digestion BamH1 qui fait apparaître une bande de 3,7kb. La plasmide ainsi obtenu, pYSHVG3 est introduit par transformation au PEG dans la souche classique de levure DCO4 (a ade1 leu2-04 cir°).

La construction du vecteur pYSHVG3 est représentée sous la fig. XI. Le protocole de la fermentation et de l'induction est particulier car l'induction de ADH2 est sensible à la répression catabolique. La première phase de la fermentation vise à obtenir une biomasse en maintenant le plasmide. La sélection se fait par complémentation de la mutation leu2, par conséquent le milieu est le YNB (Yeast nitrogen base without amino acids Difco 0919-15), avec 3% de glucose. Le milieu est rendu aussi riche que possible en ajoutant tous les acides aminés à l'exception de la leucine, et les bases azotées. Le temps de génération de la souche dans ce milieu est proche de 4 heures, contre 2 heures seulement dans un milieu complexe. La densité optique atteinte est de 2,5. Les cellules sont ensuite transférées dans un milieu identique, pendant une nuit, où le glucose a été remplacé par du glycérol pour lever la répression catabolique. L'induction se fait dans le milieu riche YM (Yeast Broth Difco 07711-01), en présence de 3% d'éthanol.

Bien que ce milieu contienne tous les nutriments nécessaires à la levure, on observe qu'une faible croissance, même après une incubation de 24 heures. Après 5 heures d'incubation les cellules ont été récoltées par centrifugation et resuspendues dans le tampon Tris-HCl 20mM, EDTA 10mM, PMSF (fluorure de tampon Tris-HCl 20mM, EDTA 10mM, PMSF (fluorure de méthyl-phényl-sulfonyl) 1mM, pH8,0. Les cellules sont alors brisées par trois passages à la presse de French. Après une centrifugation de 30 min. à 15.000g le surnageant constitue un extrait brut de la séquence d'acides aminés sur lequel est réalisé l'ELISA, de manière identique à celle utilisée pour la protéine fusion SHVG27. La concentration du virus est de 600ng/ml dans le premier puits, suivent des dilutions de 2 en 2. La concentration initiale des protéines de levure est de 500 microg/ml. Le témoin de protéine de levure est la souche non transformée.

On constate que la protéine obtenue par expression dans la levure est reconnue par des anticorps monoclonaux dirigés contre la protéine G du virus de la SHV et notamment par les anticorps monoclonaux C10 et I10 définis précédemment.

Les Figures XIIa et XIIb représentent les résultats du test ELISA (respectivement avec l'anticorps monoclonal I10 et C10), obtenus respectivement avec:
- la levure témoin (courbe comportant les points (.)),
- le virus de la SHV (courbe comportant des étoiles (*)),
- la protéine de l'invention exprimée par la levure (courbe comportant des croix (+) et désignée par "levure recombinante" sur la légende des fig. XIIa et XIIb).

L'axe des ordonnées représente la densité optique et celui des abscisses le log 2 de la dilution.

**Revendications**
**Revendications pour les Etats contractants suivants : AT, BE, CH, DE, FR, GB, IT, LI, LU, NL, SE**

1. Polypeptide recombinant caractérisé par la présence dans son ossature polypeptidique d'un enchaînement (I) d'acides aminés contenu dans la séquence suivante :

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-


Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

31

et par l'absence de contaminants naturels, issus du virus de la septicémie hémorragique virale.

2. Polypeptide selon la revendication 1, caractérisé en ce qu'il est reconnu par des anticorps neutralisants formés au préalable contre le virus de la SHV.

3. Polypeptide recombinant selon la revendication 2, caractérisé en ce qu'il est susceptible d'induire in vivo, des anticorps neutralisants contre le virus de la SHV.

4. Polypeptide recombinant selon l'une quelconque des revendications 1 à 3, caractérisé par la présence dans son ossature peptidique d'un enchaînement (II) d'acides aminés suivant :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
```

```
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

et par l'absence de contaminants naturels, issus du virus de la septicémie hémorragique virale.

**5.** Polypeptide selon l'une quelconque des revendications 1 à 3, caractérisé en ce qu'il contient tout ou partie de l'enchaînement (III) d'acides aminés suivant :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

**6.** Polypeptide selon l'une quelconque des revendications 1 à 3, caractérisé en ce qu'il contient tout ou partie de l'enchaînement d'acides aminés suivants :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

**7.** Polypeptide selon l'une quelconque des revendications 1 à 3, caractérisé en ce qu'il est constitué par l'enchaînement d'acides aminés I suivant:

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-


His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

34

8. Polypeptide selon l'une quelconque des revendications 1 à 3, caractérisé en ce qu'il est constitué par l'enchaînement (II) d'acides aminés suivant :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
```

```
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

9. Polypeptide selon l'une quelconque des revendications 1 à 3, caractérisé en ce qu'il est constitué par l'enchaînement (III) d'acides aminés suivant :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

10. Polypeptide selon l'une quelconque des revendications 1 à 3, caractérisé en ce qu'il est constitué par la séquence d'acides aminés IV suivante :

```
Met-Glu-Trp-Asn-Thr-Phe-Phe-Leu-Val-Ile-Leu-Ile-Ile-
Ile-Ile-Lys-Ser-Thr-Thr-Pro
```

11. Séquence d'acides aminés, selon les revendications 1 à 9, caractérisée en ce qu'elle est constituée par un enchaînement d'acides aminés selon les revendications 1 à 9 et une protéine ou une séquence d'acides aminés hétérologue par rapport audit enchaînement comportant d'environ 8 à environ 800 acides aminés.

12. Séquence d'acides aminés, selon la revendication 11, caractérisée en ce que la séquence d'acides aminés étrangère est la $\beta$-galactosidase.

13. Polypeptide caractérisé par la présence d'une ossature peptidique comprenant tout ou partie d'un enchaînement d'acides aminés, codé par l'acide nucléique (1) suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
```

```
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT CCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC GAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG TCG GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
```

```
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**14.** Acide nucléique caractérisé en ce qu'il comprend un enchaînement de nucléotides codant pour les polypeptides selon les revendications 1 à 13.

**15.** Acide nucléique selon la revendication 14, caractérisé en ce qu'il comprend tout ou partie de l'enchaînement de nucléotides (2) suivant :

```
                        ATG GAA TGG AAC ACT TTT TTC TTG GTG
ATC TTG ATC ATC ATC ATA AAG AGC ACC ACA CCA CAG ATC ACT
CAA CGA CCT CCG GTC GAA AAC ATC TCG ACG TAC CAT GCA GAC
TGG GAC ACT CCG CTA TAC ACT CAT CCC TCC AAC TGC AGG GAC
GAT TCC TTT GTC CCG ATT CGA CCA GCT CAA CTC AGG TGT CCT
CAT GAA TTT GAA GAC ATA AAC AAG GGA CTG GTT TCC GTC CCA
ACC AGG ATC ATC CAT CTC CCG CTA TCG GTC ACC AGC GTC TCC
GCA GTA GCG AGT GGC CAC TAC CTG CAC AGA GTG ACT TAT CGA
GTC ACC TGT TCG ACC AGC TTC TTT GGA GGG CAA ACC ATC GAA
AAG ACC ATC TTG GAG GCG AAA CTG TCT CGT CAG GAG GCC ACA
GAC GAG GCA AGC AAG GAT CAC GAG TAC CCG TTC TTC CCT GAA
CCC TCC TGC ATC TGG ATG AAA AAC AAT GTC CAT AAG GAC ATA
ACT CAC TAT TAC AAG ACC CCA AAA ACA GTA TCG GTG GAT CTC
TAC AGC AGG AAA TTT CTC AAC CCT GAT TTC ATA GAG GGG GTT
TGC ACA ACC TCG CCC TGT CAA ACT CAT TGG CAG GGA GTC TAT
TGG GTC GGT GCC ACA CCT AAA GCC CAT TGC CCC ACG TCG GAA
ACA CTA GAA GGA CAC CTG TTC ACC AGG ACC CAT GAT CAC AGG
GTG GTC AAG GCA ATT GTG GCA GGC CAT CAT CCC TGG GGA CTC
ACA ATG GCA TGC ACA GTG ACA TTC TGC GGG ACA GAA TGG ATC
AAG ACT GAC CTG GGG GAC CTG ATC CAG GTG ACA GGA CCG GGG
GGC ACG AGG AAA CTG ACT CCA AAC AAG TGT GTC AAT ACC GAT
ATC CAG ATG AGG GGG GCA ACA GAC GAC TTT TCT TAT CTC AAC
CAT CTC ATC ACC AAC ATG GCT CAA AGA ACC GAG TGC CTA GAT
GCC CAT AGT GAT ATC ACC GCT TCT GGG AAA GTA TCC TCA TTT
CTC CTC TCA AAG TTT CGT CCC AGC CAC CCT GGA CCT GGC AAG
```

```
GCA CAC TAT CTT CTC GAC GGT CAA ATC ATG CGA GGT GAC TGT
GAC TAT GAG GCA GTA GTC AGC ATC AAC TAC AAT CGC GCT CAA
TAC AAG ACG ATG AAC AAC ACA TGG AAA TCA TGG AAA CGG GTA
GAC AAC AAC ACA GAC GGG TAC GAT GGG ATG ATA TTT GGG GAC
AAA TTG ATC ATC CCG GAC ATC GAA AAG TAT CAG AGT GTC TAT
GAC AGT GGA ATG CTC GTT CAA AGA AAC CTT GTG GAA GTC CCT
CAT CTG AGC ATT GTG TTT GTC TCC AAC ACA TCT GAT CTT TCC
ACT AAT CAC ATC CAC ACC AAC CTA ATC CCT TCG GAT
```

16. Acide nucléique caractérisé en ce qu'il comprend tout ou partie de l'enchaînement de nucléotides (3) suivant :

```
CAG ATC ACT CAA CGA CCT CCG GTC GAA AAC ATC TCG ACG TAC
CAT GCA GAC TGG GAC ACT CCG CTA TAC ACT CAT CCC TCC AAC
TGC AGG GAC GAT TCC TTT GTC CCG ATT CGA CCA GCT CAA CTC
AGG TGT CCT CAT GAA TTT GAA GAC ATA AAC AAG GGA CTG GTT
TCC GTC CCA ACC AGG ATC ATC CAT CTC CCG CTA TCG GTC ACC
AGC GTC TCC GCA GTA GCG AGT GGC CAC TAC CTG CAC AGA GTG
ACT TAT CGA GTC ACC TGT TCG ACC AGC TTC TTT GGA GGG CAA
ACC ATC GAA AAG ACC ATC TTG GAG GCG AAA CTG TCT CGT CAG
GAG GCC ACA GAC GAG GCA AGC AAG GAT CAC CAG TAC CCG TTC
TTC CCT GAA CCC TCC TGC ATC TGG ATG AAA AAC AAT GTC CAT
AAG GAC ATA ACT CAC TAT TAC AAG ACC CCA AAA ACA GTA TCG
GTG GAT CTC TAC AGC AGG AAA TTT CTC AAC CCT GAT TTC ATA
GAG GGG GTT TGC ACA ACC TCG CCC TGT CAA ACT CAT TGG CAG
GGA GTC TAT TGG GTC GGT GCC ACA CCT AAA GCC CAT TGC CCC
ACG TCG GAA ACA CTA GAA GGA CAC CTG TTC ACC AGG ACC CAT
GAT CAC AGG GTG GTC AAG GCA ATT GTG GCA GGC CAT CAT CCC
TGG GGA CTC ACA ATG GCA TGC ACA GTG ACA TTC TGC GGG ACA
GAA TGG ATC AAG ACT GAC CTG GGG GAC CTG ATC CAG GTG ACA
GGA CCG GGG GGC ACG AGG AAA CTG ACT CCA AAC AAG TGT GTC
AAT ACC GAT ATC CAG ATG AGG GGG GCA ACA GAC GAC TTT TCT
TAT CTC AAC CAT CTC ATC ACC AAC ATG GCT CAA AGA ACC GAG
TGC CTA GAT GCC CAT AGT GAT ATC ACC GCT TCT GGG AAA GTA
TCC TCA TTT CTC CTC TCA AAG TTT CGT CCC AGC CAC CCT GGA
CCT GGC AAG GCA CAC TAT CTT CTC GAC GGT CAA ATC ATG CGA
GGT GAC TGT GAC TAT GAG GCA GTA GTC AGC ATC AAC TAC AAT
```

```
CGC GCT CAA TAC AAG ACG ATG AAC AAC ACA TGG AAA TCA TGG
AAA CGG GTA GAC AAC AAC ACA GAC GGG TAC GAT GGG ATG ATA
TTT GGG GAC AAA TTG ATC ATC CCG GAC ATC GAA AAG TAT CAG
AGT GTC TAT GAC AGT GGA ATG CTC GTT CAA AGA AAC CTT GTG
GAA GTC CCT CAT CTG AGC ATT GTG TTT GTC TCC AAC ACA TCT
GAT CTT TCC ACT AAT CAC ATC CAC ACC AAC CTA ATC CCT TCG
GAT
```

**17.** Acide nucléique caractérisé en ce qu'il comprend tout ou partie de l'enchaînement de nucléotides (4) suivant :

```
ATG CGA GGT GAC TGT GAC TAT GAG GCA GTA GTC AGC ATC AAC
TAC AAT CGC GCT CAA TAC AAG ACG ATG AAC AAC ACA TGG AAA
TCA TGG AAA CGG GTA GAC AAC AAC ACA GAC GGG TAC GAT GGG
ATG ATA TTT GGG GAC AAA TTG ATC ATC CCG GAC ATC GAA AAG
TAT CAG AGT GTC TAT GAC AGT GGA ATG CTC GTT CAA AGA AAC
CTT GTG GAA GTC CCT CAT
```

**18.** Acide nucléique caractérisé en ce qu'il comprend tout ou partie de l'enchaînement de nucléotides (5) suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA
```

**19.** Acide nucléique caractérisé en ce qu'il comprend tout ou partie de l'enchaînement de nucléotides (6) suivant :

```
TGG TCA TTC AAC TGG AGT CTT TGG CCA TCA TTA TCT
GGG ATG GGG GTT GTG GGA GGG GCC TTC CTT CTA CTG GTA CTC
TGC TGT TGC TGC AAG GCG TCC CCT CCC ATT CCA AAT TAC GGG
```

```
ATT CCG ATG CAG CAG TTC TCC AGA AGT CAG ACG GTC TGA GCA
CAC CTG TCC GAA TGA CCA CAA TTC CTC TCT TAG GTA GAT AGA
AAA AAA AAA AAA AAA AAA AAA AAA A
```

20. Acide nucléique selon la revendication 15, caractérisé en ce qu'il est précédé d'une séquence nucléotidique comprenant les éléments de régulation de l'expression de ce dernier.

21. Acide nucléique selon les revendications 15 et 20, caractérisé en ce qu'il comprend les éléments terminateurs de la transcription de ce dernier.

22. Acide nucléique caractérisé en ce qu'il comprend tout ou partie de l'enchaînement de nucléotides (1) suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
```

41

```
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**23.** Acide nucléique selon la revendication 22, caractérisé en ce qu'il est constitué par l'enchaînement (1) de nucléotides suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
```

```
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

24. Acide nucléique recombinant, caractérisé en ce qu'il contient l'acide nucléique selon l'une quelconque des revendications 14 à 23, inséré dans un acide nucléique hétérologue vis-à-vis du susdit fragment.

25. Vecteur recombinant, en particulier pour le clonage et/ou l'expression notamment du type plasmide, cosmide ou phage, caractérisé en ce qu'il contient un acide nucléique recombinant selon l'une des revendications 14 à 23, en l'un des sites non essentiels pour sa réplication.

26. Vecteur recombinant selon la revendication 25, caractérisé en ce qu'il s'agit du plasmide PSHV-G1 déposé à la CNCM sous le n° I 778.

27. Vecteur recombinant selon la revendication 25, caractérisé en ce qu'il contient en l'un de ses sites non essentiels pour sa réplication, des éléments nécessaires pour promouvoir l'expression d'une séquence d'acides aminés selon les revendications 1 à 13 dans un hôte cellulaire, et éventuellement un promoteur reconnu par les polymérases de l'hôte cellulaire, en particulier un promoteur inductible et éventuellement une séquence signal et/ou une séquence d'ancrage.

28. Vecteur recombinant selon la revendication 27, caractérisé en ce qu'il contient
   - les éléments permettant l'expression de cet acide nucléique selon les revendications 11 à 20 inséré dans le vecteur par E. Coli, et notamment
   - les éléments permettant l'expression de tout ou partie du gène de la $\beta$-galactosidase.

29. Vecteur recombinant selon la revendication 27, caractérisé en ce qu'il contient les éléments permettant d'insérer l'acide nucléique selon les revendications 14 à 23 dans la levure et l'expression de cet acide nucléique dans la levure.

30. Hôte cellulaire transformé par un vecteur recombinant selon l'une quelconque des revendications 25 à 29 et comprenant les éléments de régulation permettant l'expression de la séquence nucléique codant pour le polypeptide selon l'une des revendications 1 à 13 dans cet hôte.

31. Hôte cellulaire selon la revendication 30, caractérisé en qu'il s'agit de E. Coli transformé par le vecteur selon la revendication 28.

32. Hôte cellulaire transformé selon la revendication 30, caractérisé en ce qu'il s'agit de levure transformée par le vecteur selon la revendication 29.

33. Produit d'expression d'un acide nucléique exprimé par un hôte cellulaire transformé selon les revendications 30 à 32.

34. Principe actif de vaccin caractérisé en ce qu'il comprend le produit d'expression de la revendication 33, éventuellement couplé à une protéine naturelle ou à un polypeptide synthétique ayant un poids moléculaire suffisant pour que le conjugué soit capable d'induire in vivo la production d'anticorps neutralisants anti-SHV.

35. Principe actif de vaccin, selon la revendication 34, administrable par voie orale, caractérisé en ce qu'il contient de 10 ng à 100 $\mu$g de principe actif par gramme de nourriture vaccinante.

36. Principe actif de vaccin, selon la revendication 34, administrable par voie intrapéritonéale, caractérisé en ce qu'il contient de 1 à 1000 ng/g de poisson

37. Principe actif de vaccin, selon la revendication 34, administrable par balnéation dans un milieu contenant de 10 ng à 100 $\mu$g/ml.

38. Sonde nucléotidique caractérisée en ce qu'elle hybride avec l'un des acides nucléiques selon les revendications 14 à 23 ou avec leur séquence complémentaire,
   et, en particulier, les sondes choisies parmi les séquences nucléotidiques suivantes :
   ACG TTG GAG GGA AAG GCC
   GAC TGG GAC ACT CCG CTA
   CCC TCC TGC ATC TGG ATG
   AAG ACT GAC CTG GGG GAC
   GAC AAC AAC ACA GAC GGG
   TTC CTT CTA CTG GTA CTC
   ou leurs séquences nucléotidiques complémentaires.

39. Procédé de préparation d'un polypeptide recombinant selon l'une quelconque des revendications 1 à 13, caractérisé en ce que :

44

- on cultive, dans un milieu de culture approprié, un hôte cellulaire préalablement transformé par un vecteur approprié contenant un acide nucléique selon l'une des revendications 14 à 23 et
- on récupère à partir du susdit milieu de culture le polypeptide produit par ledit hôte cellulaire transformé.

**40.** Procédé, selon la revendication 40, de préparation d'un polypeptide recombinant déterminé, caractérisé par la mise en culture de E. Coli selon la revendication 31 et par l'arrêt de la culture en fin de phase exponentielle de croissance dudit E. Coli, et récupération de la séquence d'acides aminés déterminée à partir du milieu de culture exempt des autres protéines et enzymes exocellulaires susceptibles d'être secrétées par E. Coli.

**41.** Procédé, selon la revendication 40, de préparation d'un polypeptide déterminé, caractérisé par la mise en culture de levure selon la revendication 32, et par l'arrêt de la culture en fin de phase exponentielle de croissance de ladite levure, et récupération du polypeptide micro-encapsulé dans la levure.

**42.** Polypeptide selon l'une des revendications 1 ou 3, caractérisé en ce qu'il est constitué par la séquence d'acides aminés définie entre les résidus 111 à 302, contenue dans la séquence d'acides aminés représentée sur la figure 3.

**43.** Acide nucléique, notamment fragment d'ADN, codant pour le polypeptide défini dans la revendication 42.

**44.** Anticorps monoclonal préparé à partir d'un polypeptide recombinant immunogène selon l'une quelconque des revendications 1 à 13, et caractérisé en ce qu'il est dirigé contre l'un quelconque de ces polypeptides.

**45.** Anticorps monoclonal selon la revendication 44, caractérisé en ce qu'il reconnaît spécifiquement le polypeptide de la revendication 42.

**Revendications pour l'Etat contractant suivant : ES**

**1.** Procédé de préparation d'un polypeptide contenant dans son ossature polypeptidique d'un enchaînement (I) d'acides aminés contenu dans la séquence suivante :

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-


Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

46

caractérisé en ce que, partant de préférence de l'amino acide C-terminal, l'on condense successivement deux à deux les aminoacyles successifs dans l'ordre requis, ou des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments à l'exception des fonctions amine de l'un et carboxyle de l'autre ou vice versa, qui doivent normalement intervenir dans la formation des liaisons peptidiques, notamment après activation de la fonction carboxyle, selon les méthodes connues dans la synthèse des peptides et ainsi de suite, de proche en proche, jusqu'à l'acide aminé N-terminal.

2. Procédé de préparation, selon la revendication 1, d'un polypeptide reconnu par des anticorps neutralisants formés au préalable contre le virus de la SHV.

3. Procédé de préparation, selon la revendication 1, d'un polypeptide susceptible d'induire in vivo, des anticorps neutralisants contre le virus de la SHV.

4. Procédé de préparation, selon la revendication 1, d'un polypeptide contenant dans son ossature peptidique d'un enchaînement (II) d'acides aminés suivant :

Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-


Ser-Asn-Thr-Ser-Asp-Leu-Ser-Thr-Asn-His-Ile-His-Thr-
Asn-Leu-Ile-Pro-Ser-Asp

48

5. Procédé de préparation, selon la revendication 1, d'un polypeptide contenant tout ou partie de l'enchaînement (III) d'acides aminés suivant :

```
Met Arg Gly Asp Cys Asp Tyr Glu Ala Val Val Ser Ile
Asn Tyr Asn Arg Ala Gln Tyr Lys Thr Met Asn Asn Thr
Trp Lys Ser Trp Lys Arg Val Asp Asn Asn Thr Asp Gly
Tyr Asp Gly Met Ile Phe Gly Asp Lys Leu Ile Ile Pro
Asp Ile Glu Lys Tyr Gln Ser Val Tyr Asp Ser Gly Met
Leu Val Gln Arg Asn Leu Val Glu Val Pro His
```

6. Procédé de préparation, selon la revendication 1, d'un polypeptide contenant tout ou partie de l'enchaînement d'acides aminés suivants :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

7. Procédé de préparation, selon la revendication 1, d'un polypeptide constitué par l'enchaînement d'acides aminés I suivant:

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
```

```
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

**8.** Procédé de préparation, selon la revendication 1, d'un polypeptide constitué par l'enchaînement (II) d'acides aminés suivant :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
```

```
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

**9.** Procédé de préparation, selon la revendication 1, d'un polypeptide constitué par l'enchaînement (III) d'acides aminés suivant :

```
Met Arg Gly Asp Cys Asp Tyr Glu Ala Val Val Ser Ile
Asn Tyr Asn Arg Ala Gln Tyr Lys Thr Met Asn Asn Thr
Trp Lys Ser Trp Lys Arg Val Asp Asn Asn Thr Asp Gly
Tyr Asp Gly Met Ile Phe Gly Asp Lys Leu Ile Ile Pro
Asp Ile Glu Lys Tyr Gln Ser Val Tyr Asp Ser Gly Met
Leu Val Gln Arg Asn Leu Val Glu Val Pro His
```

**10.** Procédé de préparation, selon la revendication 1, d'un polypeptide constitué par la séquence d'acides aminés IV suivante :

```
Met-Glu-Trp-Asn-Thr-Phe-Phe-Leu-Val-Ile-Leu-Ile-Ile-
Ile-Ile-Lys-Ser-Thr-Thr-Pro
```

11. Procédé de préparation, selon la revendication 1, d'une séquence d'acides aminés constituée par un enchaînement d'acides aminés défini dans les revendications 1 à 9 et une protéine ou une séquence d'acides aminés hétérologue par rapport audit enchaînement comportant d'environ 8 à environ 800 acides aminés.

12. Procédé de préparation, selon la revendication 1, d'une séquence d'acides aminés définie dans la revendication 11, dans laquelle la séquence d'acides aminés étrangère est la β-galactosidase.

13. Procédé de préparation d'un acide nucléique comprenant tout ou partie de l'enchaînement de nucléotides (1) suivant :

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG GTC GAA
```

52

```
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GGC CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

comprenant les étapes suivantes:
- la centrifugation du surnageant d'une culture de cellules infectées par un isolat de la SHV du type I et la récupération du culot de centrifugation;
- le cas échéant la centrifugation du culot récupéré sur un gradient de sucrose par la méthode décrite dans le brevet EP-A-0138667,

- la récupération de la portion du gradient contenant le virus et la resédimentation du virus par centrifugation,
- la resuspension du culot de virus et l'extraction de l'ARN génomique selon la technique décrite par Chomzynski et al. (Anal. Biochem. 162, 156, 1987),
- la synthèse d'un brin d'ADNc au contact de l'ARN génomique du virus par une transcriptase réverse en présence d'initiateurs oligomériques dont les séquences sont aléatoires selon la technique décrite dans DNA 4:429-438, 1985,
- le cas échéant, la purification des hybrides ARN/ADNc par extraction par un mélange phénol/chloroforme et précipitation à l'éthanol,
- la fabrication d'un second brin d'ADNc en présence d'ADN polymérase I, de RNAse H et de 4 désoxynucléotides selon la méthode décrite dans Gene, 25:263-269 (1983), si besoin est après extraction des protéines présentes dans le milieu de réaction,
- le clonage des acides nucléiques ainsi obtenus dans un vecteur plasmidien approprié et la récupération de l'acide nucléique recherché à l'aide d'une sonde appropriée.

14. Procédé de préparation d'un acide nucléique comprenant tout ou partie de l'enchaînement de nucléotides (1) suivant :

```
TCT TTG GAG AAA CCA GTG AGA AGA TTG ACT TCG GGA CTC

GCA GTA AGA CCC TGA TAA CAA GCT TCA AGA TAG CTG AAG

CAA AGG CCA TCT ACC TGG ATT CAA GTC CGG TGA GAT CTC

GCA TAG AGG CCA AGA AGT ACA CCA CTC CCA TTA GAC ATG

GGA GTG TGA CTT ACT ATG GTC CGT TCA TAT TTG CAG ATG

ACC ACG TTG GAG GGA AAG GCC ACC GCG AGA AGC TAG GGG

CAC TAT GTG GAT TCC TCC AGT CTG GAC CTT ACG GGC AGG

CGA AGG ACT ACT ACA ATC GTG CCG TCG AAG AAG AGA TAG

GCA TTC CCC CAA GGG ACC CGA AGC GCA GAT CTG GAA CCT

CCT CTG TCC GAC CTT GGT AGA CCG AAA GGA CTG ACC CAG

GTT TGA GAC CAC ACT CTC ATT AGA TAG AAA AAA ATG GCA

CAT TTG TGT ACA CAA CAA GCT AGA CCC ACA ATG GAA TGG

AAC ACT TTT TTC TTG GTG ATC TTG ATC ATC ATC ATA AAG

AGC ACC ACA CCA CAG ATC ACT CAA CGA CCT CCG GTC GAA

AAC ATC TCG ACG TAC CAT GCA GAC TGG GAC ACT CCG CTA

TAC ACT CAT CCC TCC AAC TGC AGG GAC GAT TCC TTT GTC

CCG ATT CGA CCA GCT CAA CTC AGG TGT CCT CAT GAA TTT

GAA GAC ATA AAC AAG GGA CTG GTT TCC GTC CCA ACC AGG

ATC ATC CAT CTC CCG CTA TCG GTC ACC AGC GTC TCC GCA

GTA GCG AGT GGC CAC TAC CTG CAC AGA GTG ACT TAT CGA

GTC ACC TGT TCG ACC AGC TTC TTT GGA GGG CAA ACC ATC

GAA AAG ACC ATC TTG GAG GCG AAA CTG TCT CGT CAG GAG

GCC ACA GAC GAG GCA AGC AAG GAT CAC CAG TAC CCG TTC

TTC CCT GAA CCC TCC TGC ATC TGG ATG AAA AAC AAT GTC

CAT AAG GAC ATA ACT CAC TAT TAC AAG ACC CCA AAA ACA

GTA TCG GTG GAT CTC TAC AGC AGG AAA TTT CTC AAC CCT

GAT TTC ATA GAG GGG GTT TGC ACA ACC TCG CCC TGT CAA

ACT CAT TGG CAG GGA GTC TAT TGG GTC GGT GCC ACA CCT

AAA GCC CAT TGC CCC ACG TCG GAA ACA CTA GAA GGA CAC

CTG TTC ACC AGG ACC CAT GAT CAC AGG GTG GTC AAG GCA

ATT GTG GCA GGC CAT CAT CCC TGG GGA CTC ACA ATG GCA

TGC ACA GTG ACA TTC TGC GGG ACA GAA TGG ATC AAG ACT

GAC CTG GGG GAC CTG ATC CAG GTG ACA GGA CCG GGG GGC

ACG AGG AAA CTG ACT CCA AAC AAG TGT GTC AAT ACC GAT
```

55

```
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

comprenant les étapes suivantes:
- la préparation des ARN cellulaires et viraux à partir d'une culture de cellules infectées depuis 16 à 20 heures par le virus de la SHV de type 1, à une multiplicité de 10 ufp par cellule, selon la technique décrite par Chomzynski et al. (Anal. Biochem 162, 156, 1987),
- la récupération et purification des ARNm cellulaires et viraux par passage des ARN cellulaires et viraux totaux par chromatographie avec un oligo(dT) immobilisé,
- la synthèse d'un brin d'ADNc à partir des ARNm purifiés selon la technique décrite dans Gene 25:263, 1983,
- le clonage des acides nucléiques ainsi obtenus dans un vecteur plasmidien approprié et la récupération de la séquence nucléique recherchée à l'aide d'une sonde d'hybridation appropriée.

15. Procédé de préparation d'un acide nucléique comprenant tout ou partie de l'enchaînement de nucléotides (1) suivant :

56

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG GTC GAA
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GGG CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG TCG GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
```

```
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

comprenant les étapes suivantes:

Lorsque l'acide nucléique à préparer comprend au maximum 200 nucléotides
- la synthèse d'ADN en utilisant la méthode automatisée des $\beta$-cyanethyl phosphoramidite décrite dans Bioorganic Chemistry 4; 274-325, 1986,
- le clonage des ADN ainsi obtenus dans un vecteur plasmidien approprié et la récupération des ADN par hybridation avec une sonde appropriée, et lorsque l'acide nucléique à préparer comprend plus de 200 nucléotides:
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Nat. Acad. Sci. USA 80; 7461-7465, 1983,
- le clonage des ADN ainsi obtenus dans un vecteur plasmidien approprié et la récupération de l'acide nucléique recherché par hybridation avec une sonde appropriée.

16. Procédé selon l'une des revendications 13, 14 ou 15 de préparation d'un acide nucléique comprenant tout ou partie de l'enchaînement de nucléotides (2) suivant :

```
                                    ATG GAA TGG AAC ACT TTT TTC TTG
GTG ATC TTG ATC ATC ATC ATA AAG AGC ACC ACA CCA CAG
ATC ACT CAA CGA CCT CCG GTC GAA AAC ATC TCG ACG TAC
CAT GCA GAC TGG GAC ACT CCG CTA TAC ACT CAT CCC TCC
AAC TGC AGG GAC GAT TCC TTT GTC CCG ATT CGA CCA GCT
CAA CTC AGG TGT CCT CAT GAA TTT GAA GAC ATA AAC AAG
GGA CTG GTT TCC GTC CCA ACC AGG ATC ATC CAT CTC CCG
CTA TCG GTC ACC AGC GTC TCC GCA GTA GCG AGT GGC CAC
TAC CTG CAC AGA GTG ACT TAT CGA GTC ACC TGT TCG ACC
AGC TTC TTT GGA GGG CAA ACC ATC GAA AAG ACC ATC TTG
GAG GCG AAA CTG TCT CGT CAG GAG GCC ACA GAC GAG GCA
AGC AAG GAT CAC GAG TAC CCG TTC TTC CCT GAA CCC TCC
TGC ATC TGG ATG AAA AAC AAT GTC CAT AAG GAC ATA ACT
CAC TAT TAC AAG ACC CCA AAA ACA GTA TCG GTG GAT CTC
TAC AGC AGG AAA TTT CTC AAC CCT GAT TTC ATA GAG GGG
GTT TGC ACA ACC TCG CCC TGT CAA ACT CAT TGG CAG GGA
GTC TAT TGG GTC GGT GCC ACA CCT AAA GCC CAT TGC CCC
ACG TCG GAA ACA CTA GAA GGA CAC CTG TTC ACC AGG ACC
CAT GAT CAC AGG GTG GTC AAG GCA ATT GTG GCA GGC CAT
CAT CCC TGG GGA CTC ACA ATG GCA TGC ACA GTG ACA TTC
TGC GGG ACA GAA TGG ATC AAG ACT GAC CTG GGG GAC CTG
ATC CAG GTG ACA GGA CCG GGG GGC ACG AGG AAA CTG ACT
CCA AAC AAG TGT GTC AAT ACC GAT ATC CAG ATG AGG GGG
GCA ACA GAC GAC TTT TCT TAT CTC AAC CAT CTC ATC ACC
AAC ATG GCT CAA AGA ACC GAG TGC CTA GAT GCC CAT AGT
GAT ATC ACC GCT TCT GGG AAA GTA TCC TCA TTT CTC CTC
TCA AAG TTT CGT CCC AGC CAC CCT GGA CCT GGC AAG GCA
CAC TAT CTT CTC GAC GGT CAA ATC ATG CGA GGT GAC TGT
GAC TAT GAG GCA GTA GTC AGC ATC AAC TAC AAT CGC GCT
CAA TAC AAG ACG ATG AAC AAC ACA TGG AAA TCA TGG AAA
```

```
CGG GTA GAC AAC AAC ACA GAC GGG TAC GAT GGG ATG ATA
TTT GGG GAC AAA TTG ATC ATC CCG GAC ATC GAA AAG TAT
CAG AGT GTC TAT GAC AGT GGA ATG CTC GTT CAA AGA AAC
CTT GTG GAA GTC CCT CAT CTG AGC ATT GTG TTT GTC TCC
AAC ACA TCT GAT CTT TCC ACT AAT CAC ATC CAC ACC AAC
CTA ATC CCT TCG GAT
```

**17.** Procédé selon l'une des revendications 13, 14 ou 15 de préparation d'un acide nucléique comprenant tout ou partie de l'enchaînement de nucléotides (3) suivant :

```
CAG ATC ACT CAA CGA CCT CCG GTC GAA AAC ATC TCG ACG
TAC CAT GCA GAC TGG GAC ACT CCG CTA TAC ACT CAT CCC
TCC AAC TGC AGG GAC GAT TCC TTT GTC CCG ATT CGA CCA
GCT CAA CTC AGG TGT CCT CAT GAA TTT GAA GAC ATA AAC
AAG GGA CTG GTT TCC GTC CCA ACC AGG ATC ATC CAT CTC
CCG CTA TCG GTC ACC AGC GTC TCC GCA GTA GCG AGT GGC
CAC TAC CTG CAC AGA GTG ACT TAT CGA GTC ACC TGT TCG
ACC AGC TTC TTT GGA GGG CAA ACC ATC GAA AAG ACC ATC
TTG GAG GCG AAA CTG TCT CGT CAG GAG GCC ACA GAC GAG
GCA AGC AAG GAT CAC CAG TAC CCG TTC TTC CCT GAA CCC
TCC TGC ATC TGG ATG AAA AAC AAT GTC CAT AAG GAC ATA
ACT CAC TAT TAC AAG ACC CCA AAA ACA GTA TCG GTG GAT
CTC TAC AGC AGG AAA TTT CTC AAC CCT GAT TTC ATA GAG
GGG GTT TGC ACA ACC TCG CCC TGT CAA ACT CAT TGG CAG
GGA GTC TAT TGG GTC GGT GCC ACA CCT AAA GCC CAT TGC
CCC ACG TCG GAA ACA CTA GAA GGA CAC CTG TTC ACC AGG
ACC CAT GAT CAC AGG GTG GTC AAG GCA ATT GTG GCA GGC
CAT CAT CCC TGG GGA CTC ACA ATG GCA TGC ACA GTG ACA
TTC TGC GGG ACA GAA TGG ATC AAG ACT GAC CTG GGG GAC
CTG ATC CAG GTG ACA GGA CCG GGG GGC ACG AGG AAA CTG
ACT CCA AAC AAG TGT GTC AAT ACC GAT ATC CAG ATG AGG
GGG GCA ACA GAC GAC TTT TCT TAT CTC AAC CAT CTC ATC
ACC AAC ATG GCT CAA AGA ACC GAG TGC CTA GAT GCC CAT
AGT GAT ATC ACC GCT TCT GGG AAA GTA TCC TCA TTT CTC
```

60

```
CTC TCA AAG TTT CGT CCC AGC CAC CCT GGA CCT GGC AAG
GCA CAC TAT CTT CTC GAC GGT CAA ATC ATG CGA GGT GAC
TGT GAC TAT GAG GCA GTA GTC AGC ATC AAC TAC AAT CGC
GCT CAA TAC AAG ACG ATG AAC AAC ACA TGG AAA TCA TGG
AAA CGG GTA GAC AAC AAC ACA GAC GGG TAC GAT GGG ATG
ATA TTT GGG GAC AAA TTG ATC ATC CCG GAC ATC GAA AAG
TAT CAG AGT GTC TAT GAC AGT GGA ATG CTC GTT CAA AGA
AAC CTT GTG GAA GTC CCT CAT CTG AGC ATT GTG TTT GTC
TCC AAC ACA TCT GAT CTT TCC ACT AAT CAC ATC CAC ACC
AAC CTA ATC CCT TCG GAT
```

**18.** Procédé selon l'une des revendications 13, 14 ou 15 de préparation d'un acide nucléique comprenant tout ou partie de l'enchaînement de nucléotides (4) suivant :

```
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT
```

**19.** Procédé selon l'une des revendications 13, 14 ou 15 de préparation d'un acide nucléique comprenant tout ou partie de l'enchaînement de nucléotides (5) suivant :

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
```

```
GTT TGA GAC CAC ACT CTC ATT AGA TAG AAA AAA ATG GCA
CAT TTG TGT ACA CAA CAA GCT AGA CCC ACA
```

**20.** Procédé de préparation selon l'une des revendications 13, 14 ou 15 de préparation d'un acide nucléique constitué par l'enchaînement (6) de nucléotides suivant :

```
        TGG TCA TTC AAC TGG AGT CTT TGG CCA TCA TTA
    TCT GGG ATG GGG GTT GTG GGA GGG GCC TTC CTT CTA CTG
    GTA CTC TGC TGT TGC TGC AAG GCG TCC CCT CCC ATT CCA
    AAT TAC GGG ATT CCG ATG CAG CAG TTC TCC AGA AGT CAG
    ACG GTC TGA GCA CAC CTG TCC GAA TGA CCA CAA TTC CTC
    TCT TAG GTA GAT AGA AAA AAA AAA AAA AAA AAA AAA AAA
    A
```

**21.** Procédé de production d'anticorps comprenant l'immunisation d'un animal à l'aide d'un polypeptide défini dans les revendications 1 à 13 et la récupération des anticorps formés.

**22.** Procédé de préparation d'une sonde nucléotidique de séquence suivante:
ACG TTG GAG GGA AAG GCC
GAC TGG GAC ACT CCG CTA
CCC TCC TGC ATC TGG ATG
AAG ACT GAC CTG GGG GAC
GAC AAC AAC ACA GAC GGG
TTC CTT CTA CTG GTA CTC
caractérisé en ce que on effectue la synthèse d'ADN en utilisant la méthode automatisée des *β*-cyanéthyl phosphoramidite décrite dans Bioorganic Chemistry 4; 274-325, 1986.

**23.** Procédé de détection d'une séquence d'acides nucléiques définie dans les revendications 13 à 19, caractérisé en ce que:
- on effectue, avec l'une des sondes de la revendications 22, une étape de préhybridation de l'ARN cible, préalablement rendu accessible, dans les conditions suivantes: traitement à 16 heures à 56°C dans le milieu de pré-hybridation contenant le tampon suivant : 3M chlorure de tétraméthylammonium, 50mM Tris- Tris-HC1, pH 8.0, 2mM EDTA, 100$\mu$g/ml DNA soniqué, dénaturé de thymus de veau, 5X Denhardt,
- on effectue une hybridation dans les conditions suivantes: traitement pendant 1 heure à la même température avec environ 100 pg d'une des sondes ou d'un mélange des sondes définies à la revendication 22,
- on effectue ensuite un lavage dans les conditions suivantes :
  . 5 minutes dans 2X SSC, 0,1% SDS à températurure ambiante,
  . 5 minutes dans 5X SSC, 0,1% SDS à 59°C,
  . deux passages dans le tampon d'hybridation ci-dessus, sans Denhardt ni ADN de veau pendant 1 h, à 59°C.

**24.** Procédé de préparation d'un polypeptide défini dans l'une quelconque des revendications 1 à 12, comprenant les étapes suivantes:
- la culture d'un hôte cellulaire compétent, auparavant transformé avec un acide nucléique contenant une séquence de nucléotides codant pour les polypeptides définis dans l'une des revendications 1 à 21 et
  dans laquelle cette séquence de nucléotides se trouve placée sous le contrôle d'éléments de régulation, dont notamment un promoteur reconnu par les polymérases de cet hôte cellulaire compétent, les éléments d'initiation et de terminaison de la traduction, et
- la récupération du polypeptide produit à partir des produits d'expression de cet hôte cellulaire.

**25.** Procédé, selon la revendication 24, de préparation d'un polypeptide recombinant déterminé, caractérisé par la mise en culture de E. Coli transformé par le vecteur contenant en l'un de ses sites non essentiels pour sa réplication, des éléments nécessaires pour promouvoir l'expression d'une séquence d'acides aminés définie dans les revendications 1 à 12 dans un hôte cellulaire, et éventuellement un promoteur reconnu par les polymérases de l'hôte cellulaire, en particulier un promoteur inductible et éventuellement une séquence signal et/ou une séquence d'ancrage,
et notamment

- les éléments permettant l'expression de tout ou partie du gène de la $\beta$-galactosidase, et par l'arrêt de la culture en fin de phase exponentielle de croissance dudit E. Coli,
- et récupération de la séquence d'acides aminés déterminée à partir du milieu de culture exempt des autres protéines et enzymes exocellulaires susceptibles d'être secrétées par E. Coli.

26. Procédé, selon la revendication 24, de préparation d'un polypeptide déterminé, caractérisé par la mise en culture de levure transformé par le vecteur contenant les éléments permettant d'insérer l'un quelconque des acides nucléiques définis dans les revendications 13 à 19 dans la levure, et l'expression de cet acide nucléique dans la levure, et par l'arrêt de la culture en fin de phase exponentielle de croissance de ladite levure, et récupération du polypeptide micro-encapsulé dans la levure.

27. Procédé, selon l'une quelconque des revendications 1, 24, 25 ou 26, de préparation d'un polypeptide constitué par la séquence d'acides aminés définie entre les résidus 111 à 302, contenue dans la séquence d'acides aminées représentée sur la figure 3.

28. Procédé, selon l'une quelconque des revendications 13, 14 ou 15, de préparation de l'acide nucléique, notamment fragment d'ADN, codant pour le polypeptide constitué par la séquence d'acides aminés définie entre les résidus 111 à 302, contenue dans la séquence d'acides aminées représentée sur la figure 3.

29. Procédé de production d'anticorps monoclonaux comportant la culture d'hybridomes préparés à partir de polypeptides obtenus par le procédé selon l'une quelconque des revendications 1 à 12 ou 24 à 27.

**Revendications pour l'Etat contractant suivant : GR**

1. Polypeptide recombinant caractérisé par la présence dans son ossature polypeptidique d'un enchaînement (I) d'acides aminés contenu dans la séquence suivante :

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-


Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

et par l'absence de contaminants naturels, issus du virus de la septicémie hémorragique virale.

2. Polypeptide selon la revendication 1, caractérisé en ce qu'il est reconnu par des anticorps neutralisants formés au préalable contre le virus de la SHV.

3. Polypeptide recombinant selon la revendication 2, caractérisé en ce qu'il est susceptible d'induire in vivo, des anticorps neutralisants contre le virus de la SHV.

4. Polypeptide recombinant selon l'une quelconque des revendications 1 à 3, caractérisé par la présence dans son ossature peptidique d'un enchaînement (II) d'acides aminés suivant :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
```

```
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

et par l'absence de contaminants naturels, issus du virus de la septicémie hémorragique virale.

**5.** Polypeptide selon l'une quelconque des revendications 1 à 3, caractérisé en ce qu'il contient tout ou partie de l'enchaînement (III) d'acides aminés suivant :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

**6.** Polypeptide selon l'une quelconque des revendications 1 à 3, caractérisé en ce qu'il contient tout ou partie de l'enchaînement d'acides aminés suivants :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
```

```
Gly-Ile-Pro-Met-Gln-Gln-Phe-Ser-Arg-Ser-Gln-Thr-Val
```

**7.** Polypeptide selon l'une quelconque des revendications 1 à 3, caractérisé en ce qu'il est constitué par l'enchaînement d'acides aminés I suivant:

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
```

```
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

8. Polypeptide selon l'une quelconque des revendications 1 à 3, caractérisé en ce qu'il est constitué par l'enchaînement (II) d'acides aminés suivant :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
```

```
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

9. Polypeptide selon l'une quelconque des revendications 1 à 3, caractérisé en ce qu'il est constitué par l'enchaînement (III) d'acides aminés suivant :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

10. Polypeptide selon l'une quelconque des revendications 1 à 3, caractérisé en ce qu'il est constitué par la séquence d'acides aminés IV suivante :

```
Met-Glu-Trp-Asn-Thr-Phe-Phe-Leu-Val-Ile-Leu-Ile-Ile-
Ile-Ile-Lys-Ser-Thr-Thr-Pro
```

11. Séquence d'acides aminés, selon les revendications 1 à 9, caractérisée en ce qu'elle est constituée par un enchaînement d'acides aminés selon les revendications 1 à 9 et une protéine ou une séquence d'acides aminés hétérologue par rapport audit enchaînement comportant d'environ 8 à environ 800 acides aminés.

12. Séquence d'acides aminés, selon la revendication 11, caractérisée en ce que la séquence d'acides aminés étrangère est la $\beta$-galactosidase.

13. Polypeptide caractérisé par la présence d'une ossature peptidique comprenant tout ou partie d'un enchaînement d'acides aminés, codé par l'acide nucléique (1) suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
```

```
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT CCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC GAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
```

70

EP 0 377 349 B1

```
ACA TGG AAA TCA TGG AAA CGG GTA GAC AAC AAC ACA GAC GGG

TAC GAT GGG ATG ATA TTT GGG GAC AAA TTG ATC ATC CCG GAC

ATC GAA AAG TAT CAG AGT GTC TAT GAC AGT GGA ATG CTC GTT

CAA AGA AAC CTT GTG GAA GTC CCT CAT CTG AGC ATT GTG TTT

GTC TCC AAC ACA TCT GAT CTT TCC ACT AAT CAC ATC CAC ACC

AAC CTA ATC CCT TCG GAT
```

14. Acide nucléique caractérisé en ce qu'il comprend un enchaînement de nucléotides codant pour les polypeptides selon les revendications 1 à 13.

15. Acide nucléique selon la revendication 14, caractérisé en ce qu'il comprend tout ou partie de l'enchaînement de nucléotides (2) suivant :

```
                                ATG GAA TGG AAC ACT TTT TTC TTG GTG

ATC TTG ATC ATC ATC ATA AAG AGC ACC ACA CCA CAG ATC ACT

CAA CGA CCT CCG GTC GAA AAC ATC TCG ACG TAC CAT GCA GAC

TGG GAC ACT CCG CTA TAC ACT CAT CCC TCC AAC TGC AGG GAC

GAT TCC TTT GTC CCG ATT CGA CCA GCT CAA CTC AGG TGT CCT

CAT GAA TTT GAA GAC ATA AAC AAG GGA CTG GTT TCC GTC CCA

ACC AGG ATC ATC CAT CTC CCG CTA TCG GTC ACC AGC GTC TCC

GCA GTA GCG AGT GGC CAC TAC CTG CAC AGA GTG ACT TAT CGA

GTC ACC TGT TCG ACC AGC TTC TTT GGA GGG CAA ACC ATC GAA

AAG ACC ATC TTG GAG GCG AAA CTG TCT CGT CAG GAG GCC ACA

GAC GAG GCA AGC AAG GAT CAC GAG TAC CCG TTC TTC CCT GAA

CCC TCC TGC ATC TGG ATG AAA AAC AAT GTC CAT AAG GAC ATA

ACT CAC TAT TAC AAG ACC CCA AAA ACA GTA TCG GTG GAT CTC

TAC AGC AGG AAA TTT CTC AAC CCT GAT TTC ATA GAG GGG GTT

TGC ACA ACC TCG CCC TGT CAA ACT CAT TGG CAG GGA GTC TAT

TGG GTC GGT GCC ACA CCT AAA GCC CAT TGC CCC ACG TCG GAA

ACA CTA GAA GGA CAC CTG TTC ACC AGG ACC CAT GAT CAC AGG

GTG GTC AAG GCA ATT GTG GCA GGC CAT CAT CCC TGG GGA CTC

ACA ATG GCA TGC ACA GTG ACA TTC TGC GGG ACA GAA TGG ATC

AAG ACT GAC CTG GGG GAC CTG ATC CAG GTG ACA GGA CCG GGG

GGC ACG AGG AAA CTG ACT CCA AAC AAG TGT GTC AAT ACC GAT

ATC CAG ATG AGG GGG GCA ACA GAC GAC TTT TCT TAT CTC AAC

CAT CTC ATC ACC AAC ATG GCT CAA AGA ACC GAG TGC CTA GAT

GCC CAT AGT GAT ATC ACC GCT TCT GGG AAA GTA TCC TCA TTT
```

71

```
CTC CTC TCA AAG TTT CGT CCC AGC CAC CCT GGA CCT GGC AAG
GCA CAC TAT CTT CTC GAC GGT CAA ATC ATG CGA GGT GAC TGT
GAC TAT GAG GCA GTA GTC AGC ATC AAC TAC AAT CGC GCT CAA
TAC AAG ACG ATG AAC AAC ACA TGG AAA TCA TGG AAA CGG GTA
GAC AAC AAC ACA GAC GGG TAC GAT GGG ATG ATA TTT GGG GAC
AAA TTG ATC ATC CCG GAC ATC GAA AAG TAT CAG AGT GTC TAT
GAC AGT GGA ATG CTC GTT CAA AGA AAC CTT GTG GAA GTC CCT
CAT CTG AGC ATT GTG TTT GTC TCC AAC ACA TCT GAT CTT TCC
ACT AAT CAC ATC CAC ACC AAC CTA ATC CCT TCG GAT
```

16. Acide nucléique caractérisé en ce qu'il comprend tout ou partie de l'enchaînement de nucléotides (3) suivant :

```
CAG ATC ACT CAA CGA CCT CCG GTC GAA AAC ATC TCG ACG TAC
CAT GCA GAC TGG GAC ACT CCG CTA TAC ACT CAT CCC TCC AAC
TGC AGG GAC GAT TCC TTT GTC CCG ATT CGA CCA GCT CAA CTC
AGG TGT CCT CAT GAA TTT GAA GAC ATA AAC AAG GGA CTG GTT
TCC GTC CCA ACC AGG ATC ATC CAT CTC CCG CTA TCG GTC ACC
AGC GTC TCC GCA GTA GCG AGT GGC CAC TAC CTG CAC AGA GTG
ACT TAT CGA GTC ACC TGT TCG ACC AGC TTC TTT GGA GGG CAA
ACC ATC GAA AAG ACC ATC TTG GAG GCG AAA CTG TCT CGT CAG
GAG GCC ACA GAC GAG GCA AGC AAG GAT CAC CAG TAC CCG TTC
TTC CCT GAA CCC TCC TGC ATC TGG ATG AAA AAC AAT GTC CAT
AAG GAC ATA ACT CAC TAT TAC AAG ACC CCA AAA ACA GTA TCG
GTG GAT CTC TAC AGC AGG AAA TTT CTC AAC CCT GAT TTC ATA
GAG GGG GTT TGC ACA ACC TCG CCC TGT CAA ACT CAT TGG CAG
GGA GTC TAT TGG GTC GGT GCC ACA CCT AAA GCC CAT TGC CCC
ACG TCG GAA ACA CTA GAA GGA CAC CTG TTC ACC AGG ACC CAT
GAT CAC AGG GTG GTC AAG GCA ATT GTG GCA GGC CAT CAT CCC
TGG GGA CTC ACA ATG GCA TGC ACA GTG ACA TTC TGC GGG ACA
GAA TGG ATC AAG ACT GAC CTG GGG GAC CTG ATC CAG GTG ACA
GGA CCG GGG GGC ACG AGG AAA CTG ACT CCA AAC AAG TGT GTC
AAT ACC GAT ATC CAG ATG AGG GGG GCA ACA GAC GAC TTT TCT
TAT CTC AAC CAT CTC ATC ACC AAC ATG GCT CAA AGA ACC GAG
TGC CTA GAT GCC CAT AGT GAT ATC ACC GCT TCT GGG AAA GTA
TCC TCA TTT CTC CTC TCA AAG TTT CGT CCC AGC CAC CCT GGA
CCT GGC AAG GCA CAC TAT CTT CTC GAC GGT CAA ATC ATG CGA
```

```
GGT GAC TGT GAC TAT GAG GCA GTA GTC AGC ATC AAC TAC AAT

CGC GCT CAA TAC AAG ACG ATG AAC AAC ACA TGG AAA TCA TGG

AAA CGG GTA GAC AAC AAC ACA GAC GGG TAC GAT GGG ATG ATA

TTT GGG GAC AAA TTG ATC ATC CCG GAC ATC GAA AAG TAT CAG

AGT GTC TAT GAC AGT GGA ATG CTC GTT CAA AGA AAC CTT GTG

GAA GTC CCT CAT CTG AGC ATT GTG TTT GTC TCC AAC ACA TCT

GAT CTT TCC ACT AAT CAC ATC CAC ACC AAC CTA ATC CCT TCG

GAT
```

17. Acide nucléique caractérisé en ce qu'il comprend tout ou partie de l'enchaînement de nucléotides (4) suivant :

```
ATG CGA GGT GAC TGT GAC TAT GAG GCA GTA GTC AGC ATC AAC

TAC AAT CGC GCT CAA TAC AAG ACG ATG AAC AAC ACA TGG AAA

TCA TGG AAA CGG GTA GAC AAC AAC ACA GAC GGG TAC GAT GGG

ATG ATA TTT GGG GAC AAA TTG ATC ATC CCG GAC ATC GAA AAG

TAT CAG AGT GTC TAT GAC AGT GGA ATG CTC GTT CAA AGA AAC

CTT GTG GAA GTC CCT CAT
```

18. Acide nucléique caractérisé en ce qu'il comprend tout ou partie de l'enchaînement de nucléotides (5) suivant :

```
TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA
```

19. Acide nucléique caractérisé en ce qu'il comprend tout ou partie de l'enchaînement de nucléotides (6) suivant :

```
        TGG TCA TTC AAC TGG AGT CTT TGG CCA TCA TTA TCT
    GGG ATG GGG GTT GTG GGA GGG GCC TTC CTT CTA CTG GTA CTC
```

```
    TGC TGT TGC TGC AAG GCG TCC CCT CCC ATT CCA AAT TAC GGG
    ATT CCG ATG CAG CAG TTC TCC AGA AGT CAG ACG GTC TGA GCA
    CAC CTG TCC GAA TGA CCA CAA TTC CTC TCT TAG GTA GAT AGA
    AAA AAA AAA AAA AAA AAA AAA AAA A
```

**20.** Acide nucléique selon la revendication 15, caractérisé en ce qu'il est précédé d'une séquence nucléotidique comprenant les éléments de régulation de l'expression de ce dernier.

**21.** Acide nucléique selon les revendications 15 et 20, caractérisé en ce qu'il comprend les éléments terminateurs de la transcription de ce dernier.

**22.** Acide nucléique caractérisé en ce qu'il comprend tout ou partie de l'enchaînement de nucléotides (1) suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
```

75

```
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**23.** Acide nucléique selon la revendication 22, caractérisé en ce qu'il est constitué par l'enchaînement (1) de nucléotides suivant :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT CCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
```

```
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**24.** Acide nucléique recombinant, caractérisé en ce qu'il contient l'acide nucléique selon l'une quelconque des revendications 14 à 23, inséré dans un acide nucléique hétérologue vis-à-vis du susdit fragment.

**25.** Vecteur recombinant, en particulier pour le clonage et/ou l'expression notamment du type plasmide, cosmide ou phage, caractérisé en ce qu'il contient un acide nucléique recombinant selon l'une des revendications 14 à 23, en l'un des sites non essentiels pour sa réplication.

26. Vecteur recombinant selon la revendication 25, caractérisé en ce qu'il s'agit du plasmide PSHV-G1 déposé à la CNCM sous le n° I 778.

27. Vecteur recombinant selon la revendication 25, caractérisé en ce qu'il contient en l'un de ses sites non essentiels pour sa réplication, des éléments nécessaires pour promouvoir l'expression d'une séquence d'acides aminés selon les revendications 1 à 13 dans un hôte cellulaire, et éventuellement un promoteur reconnu par les polymérases de l'hôte cellulaire, en particulier un promoteur inductible et éventuellement une séquence signal et/ou une séquence d'ancrage.

28. Vecteur recombinant selon la revendication 27, caractérisé en ce qu'il contient
   - les éléments permettant l'expression de cet acide nucléique selon les revendications 11 à 20 inséré dans le vecteur par E. Coli, et notamment
   - les éléments permettant l'expression de tout ou partie du gène de la β-galactosidase.

29. Vecteur recombinant selon la revendication 27, caractérisé en ce qu'il contient les éléments permetettant d'insérer l'acide nucléique selon les revendications 14 à 23 dans la levure et l'expression de cet acide nucléique dans la levure.

30. Hôte cellulaire transformé par un vecteur recombinant selon l'une quelconque des revendications 25 à 29 et comprenant les éléments de régulation permettant l'expression de la séquence nucléique codant pour le polypeptide selon l'une des revendications 1 à 13 dans cet hôte.

31. Hôte cellulaire selon la revendication 30, caractérisé en qu'il s'agit de E. Coli transformé par le vecteur selon la revendication 28.

32. Hôte cellulaire transformé selon la revendication 30, caractérisé en ce qu'il s'agit de levure transformée par le vecteur selon la revendication 29.

33. Produit d'expression d'un acide nucléique exprimé par un hôte cellulaire transformé selon les revendications 30 à 32.

34. Sonde nucléotidique caractérisée en ce qu'elle hybride avec l'un des acides nucléiques selon les revendications 14 à 23 ou avec leur séquence complémentaire,
   et, en particulier, les sondes choisies parmi les séquences nucléotidiques suivantes :
   ACG TTG GAG GGA AAG GCC
   GAC TGG GAC ACT CCG CTA
   CCC TCC TGC ATC TGG ATG
   AAG ACT GAC CTG GGG GAC
   GAC AAC AAC ACA GAC GGG
   TTC CTT CTA CTG GTA CTC
   ou leurs séquences nucléotidiques complémentaires.

35. Procédé de préparation d'un polypeptide recombinant selon l'une quelconque des revendications 1 à 13, caractérisé en ce que :
   - on cultive, dans un milieu de culture approprié, un hôte cellulaire préalablement transformé par un vecteur approprié contenant un acide nucléique selon l'une des revendications 14 à 23 et
   - on récupère à partir du susdit milieu de culture le polypeptide produit par ledit hôte cellulaire transformé.

36. Procédé, selon la revendication 35, de préparation d'un polypeptide recombinant déterminé, caractérisé par la mise en culture de E. Coli selon la revendication 31 et par l'arrêt de la culture en fin de phase exponentielle de croissance dudit E. Coli, et récupération de la séquence d'acides aminés déterminée à partir du milieu de culture exempt des autres protéines et enzymes exocellulaires susceptibles d'être secrétées par E. Coli.

**37.** Procédé, selon la revendication 35, de préparation d'un polypeptide déterminé, caractérisé par la mise en culture de levure selon la revendication 32, et par l'arrêt de la culture en fin de phase exponentielle de croissance de ladite levure, et récupération du polypeptide micro-encapsulé dans la levure.

**38.** Polypeptide selon l'une quelconque des revendications 1 à 3, caractérisé en ce qu'il est constitué par la séquence d'acides aminés définie entre les résidus 111 à 302, contenue dans la séquence d'acides aminées représentée sur la figure 3.

**39.** Acide nucléique, notamment fragment d'ADN, codant pour le polypeptide défini dans la revendication 38.

**40.** Anticorps monoclonal préparé à partir d'un polypeptide recombinant immunogène selon l'une quelconque des revendications 1 à 13, et caractérisé en ce qu'il est dirigé contre l'un quelconque de ces polypeptides.

**41.** Anticorps monoclonal selon la revendication 40, caractérisé en ce qu'il reconnaît spécifiquement le polypeptide de la revendication 38.

**Claims**

**Claims for the following Contracting States : AT, BE, CH, DE, FR, GB, IT, LI, LU, NL, SE**

**1.** Recombinant polypeptide characterized by the presence in its polypeptide backbone of an amino acid sequence (I) contained in the following sequence :

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-

Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

and by the absence of natural contaminants, derived from the virus responsible for the viral hemorrhagic septicemia.

2. Polypeptide according to Claim1, characterized in that it is recognized by neutralizing antibodies previously formed against the VHS virus.

3. Recombinant polypeptide according to Claim 2, characterized in that it is capable of inducing in vivo neutralizing antibodies against the VHS virus.

4. Recombinant polypeptide according to any one of the Claims 1 to 3, characterized by the presence in its peptide backbone of the following amino acid sequence (II) :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
```

81

```
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

and by the absence of natural contaminants, derived from the virus responsible for the viral hemorrhagic septicemia.

5. Polypeptide according to any one of the Claims 1 to 3, characterized in that it contains all or part of the following amino acid sequence (III) :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

6. Polypeptide according to any one of the Claims 1 and 3, characterized in that it contains all or part of the following amino acid sequence :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

**7.** Polypeptide according to any one of the Claims 1 to 3, characterized in that it is constituted by the following amino acid sequence I :

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-

His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

8. Polypeptide according to any one of the Claims 1 to 3, characterized in that it is constituted by the following amino acid sequence (II) :

Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-

Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

9. Polypeptide according to any one of the Claims 1 to 3, characterized in that it is constituted by the following amino acid sequence (III) :

Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His

10. Polypeptide according to any one of the Claims 1 to 3, characterized in that it is constituted by the following amino acid sequence IV :

Met-Glu-Trp-Asn-Thr-Phe-Phe-Leu-Val-Ile-Leu-Ile-Ile-
Ile-Ile-Lys-Ser-Thr-Thr-Pro

11. Amino acid sequences according to Claims 1 to 9, characterized in that it is constituted by an amino acid sequence according to Claims 1 to 9 and a protein or an amino acid sequence heterologous with respect to the said sequence comprising from about 8 to about 800 amino acids.

12. Amino acid sequence according to Claim 11, characterized in that the foreign amino acid sequence is beta-galactosidase.

13. Polypeptide characterized by the presence of a peptide backbone comprising all or part of an amino acid sequence encoded by the following nucleic acid (1) :

TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG

85

```
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC GAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
```

```
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**14.** Nucleic acid characterized in that it comprises a nucleotide sequence coding for the polypeptides according to Claims 1 to 13.

**15.** Nucleic acid according to Claim 14, characterized in that it comprises all or part of the following nucleotide sequence (2) :

```
                        ATG GAA TGG AAC ACT TTT TTC TTG GTG
ATC TTG ATC ATC ATC ATA AAG AGC ACC ACA CCA CAG ATC ACT
CAA CGA CCT CCG GTC GAA AAC ATC TCG ACG TAC CAT GCA GAC
TGG GAC ACT CCG CTA TAC ACT CAT CCC TCC AAC TGC AGG GAC
GAT TCC TTT GTC CCG ATT CGA CCA GCT CAA CTC AGG TGT CCT
CAT GAA TTT GAA GAC ATA AAC AAG GGA CTG GTT TCC GTC CCA
ACC AGG ATC ATC CAT CTC CCG CTA TCG GTC ACC AGC GTC TCC
GCA GTA GCG AGT GGC CAC TAC CTG CAC AGA GTG ACT TAT CGA
GTC ACC TGT TCG ACC AGC TTC TTT GGA GGG CAA ACC ATC GAA
AAG ACC ATC TTG GAG GCG AAA CTG TCT CGT CAG GAG GCC ACA
GAC GAG GCA AGC AAG GAT CAC GAG TAC CCG TTC TTC CCT GAA
CCC TCC TGC ATC TGG ATG AAA AAC AAT GTC CAT AAG GAC ATA
ACT CAC TAT TAC AAG ACC CCA AAA ACA GTA TCG GTG GAT CTC
TAC AGC AGG AAA TTT CTC AAC CCT GAT TTC ATA GAG GGG GTT
TGC ACA ACC TCG CCC TGT CAA ACT CAT TGG CAG GGA GTC TAT
TGG GTC GGT GCC ACA CCT AAA GCC CAT TGC CCC ACG TCG GAA
ACA CTA GAA GGA CAC CTG TTC ACC AGG ACC CAT GAT CAC AGG
GTG GTC AAG GCA ATT GTG GCA GGC CAT CAT CCC TGG GGA CTC
ACA ATG GCA TGC ACA GTG ACA TTC TGC GGG ACA GAA TGG ATC
AAG ACT GAC CTG GGG GAC CTG ATC CAG GTG ACA GGA CCG GGG
GGC ACG AGG AAA CTG ACT CCA AAC AAG TGT GTC AAT ACC GAT
ATC CAG ATG AGG GGG GCA ACA GAC GAC TTT TCT TAT CTC AAC
CAT CTC ATC ACC AAC ATG GCT CAA AGA ACC GAG TGC CTA GAT
GCC CAT AGT GAT ATC ACC GCT TCT GGG AAA GTA TCC TCA TTT
CTC CTC TCA AAG TTT CGT CCC AGC CAC CCT GGA CCT GGC AAG
```

```
GCA CAC TAT CTT CTC GAC GGT CAA ATC ATG CGA GGT GAC TGT
GAC TAT GAG GCA GTA GTC AGC ATC AAC TAC AAT CGC GCT CAA
TAC AAG ACG ATG AAC AAC ACA TGG AAA TCA TGG AAA CGG GTA
GAC AAC AAC ACA GAC GGG TAC GAT GGG ATG ATA TTT GGG GAC
AAA TTG ATC ATC CCG GAC ATC GAA AAG TAT CAG AGT GTC TAT
GAC AGT GGA ATG CTC GTT CAA AGA AAC CTT GTG GAA GTC CCT
CAT CTG AGC ATT GTG TTT GTC TCC AAC ACA TCT GAT CTT TCC
ACT AAT CAC ATC CAC ACC AAC CTA ATC CCT TCG GAT
```

16. Nucleic acid characterized in that it comprises all or part of the following nucleotide sequence (3) :

```
CAG ATC ACT CAA CGA CCT CCG GTC GAA AAC ATC TCG ACG TAC
CAT GCA GAC TGG GAC ACT CCG CTA TAC ACT CAT CCC TCC AAC
TGC AGG GAC GAT TCC TTT GTC CCG ATT CGA CCA GCT CAA CTC
AGG TGT CCT CAT GAA TTT GAA GAC ATA AAC AAG GGA CTG GTT
TCC GTC CCA ACC AGG ATC ATC CAT CTC CCG CTA TCG GTC ACC
AGC GTC TCC GCA GTA GCG AGT GGC CAC TAC CTG CAC AGA GTG
ACT TAT CGA GTC ACC TGT TCG ACC AGC TTC TTT GGA GGG CAA
ACC ATC GAA AAG ACC ATC TTG GAG GCG AAA CTG TCT CGT CAG
GAG GCC ACA GAC GAG GCA AGC AAG GAT CAC CAG TAC CCG TTC
TTC CCT GAA CCC TCC TGC ATC TGG ATG AAA AAC AAT GTC CAT
AAG GAC ATA ACT CAC TAT TAC AAG ACC CCA AAA ACA GTA TCG
GTG GAT CTC TAC AGC AGG AAA TTT CTC AAC CCT GAT TTC ATA
GAG GGG GTT TGC ACA ACC TCG CCC TGT CAA ACT CAT TGG CAG
GGA GTC TAT TGG GTC GGT GCC ACA CCT AAA GCC CAT TGC CCC
ACG TCG GAA ACA CTA GAA GGA CAC CTG TTC ACC AGG ACC CAT
GAT CAC AGG GTG GTC AAG GCA ATT GTG GCA GGC CAT CAT CCC
TGG GGA CTC ACA ATG GCA TGC ACA GTG ACA TTC TGC GGG ACA
GAA TGG ATC AAG ACT GAC CTG GGG GAC CTG ATC CAG GTG ACA
GGA CCG GGG GGC ACG AGG AAA CTG ACT CCA AAC AAG TGT GTC
AAT ACC GAT ATC CAG ATG AGG GGG GCA ACA GAC GAC TTT TCT
TAT CTC AAC CAT CTC ATC ACC AAC ATG GCT CAA AGA ACC GAG
TGC CTA GAT GCC CAT AGT GAT ATC ACC GCT TCT GGG AAA GTA
TCC TCA TTT CTC CTC TCA AAG TTT CGT CCC AGC CAC CCT GGA
CCT GGC AAG GCA CAC TAT CTT CTC GAC GGT CAA ATC ATG CGA
GGT GAC TGT GAC TAT GAG GCA GTA GTC AGC ATC AAC TAC AAT
```

88

```
CGC GCT CAA TAC AAG ACG ATG AAC AAC ACA TGG AAA TCA TGG
AAA CGG GTA GAC AAC AAC ACA GAC GGG TAC GAT GGG ATG ATA
TTT GGG GAC AAA TTG ATC ATC CCG GAC ATC GAA AAG TAT CAG
AGT GTC TAT GAC AGT GGA ATG CTC GTT CAA AGA AAC CTT GTG
GAA GTC CCT CAT CTG AGC ATT GTG TTT GTC TCC AAC ACA TCT
GAT CTT TCC ACT AAT CAC ATC CAC ACC AAC CTA ATC CCT TCG
GAT
```

**17.** Nucleic acid characterized in that it comprises all or part of the following nucleotide sequence (4) :

```
ATG CGA GGT GAC TGT GAC TAT GAG GCA GTA GTC AGC ATC AAC
TAC AAT CGC GCT CAA TAC AAG ACG ATG AAC AAC ACA TGG AAA
TCA TGG AAA CGG GTA GAC AAC AAC ACA GAC GGG TAC GAT GGG
ATG ATA TTT GGG GAC AAA TTG ATC ATC CCG GAC ATC GAA AAG
TAT CAG AGT GTC TAT GAC AGT GGA ATG CTC GTT CAA AGA AAC
CTT GTG GAA GTC CCT CAT
```

**18.** Nucleic acid characterized in that it comprises all or part of the following nucleotide sequence (5) :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA
```

89

**19.** Nucleic acid characterized in that it comprises all or part of the following nucleotide sequence (6) :

```
TGG TCA TTC AAC TGG AGT CTT TGG CCA TCA TTA TCT
GGG ATG GGG GTT GTG GGA GGG GCC TTC CTT CTA CTG GTA CTC
TGC TGT TGC TGC AAG GCG TCC CCT CCC ATT CCA AAT TAC GGG
ATT CCG ATG CAG CAG TTC TCC AGA AGT CAG ACG GTC TGA GCA
CAC CTG TCC GAA TGA CCA CAA TTC CTC TCT TAG GTA GAT AGA
AAA AAA AAA AAA AAA AAA AAA AAA A
```

**20.** Nucleic acid according to Claim 15, characterized in that it is preceded by a nucleotide sequence comprising the regulatory elements for the expression of this latter.

**21.** Nucleic acid according to Claims 15 and 20, characterized in that it comprises the termination elements for transcription of this latter.

90

22. Nucleic acid characterized in that it comprises all or part of the following nucleotide sequence (1) :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
```

91

```
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**23.** Nucleic acid according to Claim 22, characterized in that it is constituted by the following nucleotide sequence (1) :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
```

```
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
GGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**24.** Recombinant nucleic acid, characterized in that it contains the nucleic acid according to any one of the Claims 14 to 23, inserted into a nucleic acid heterologous with respect to the above-mentioned fragment.

**25.** Recombinant vector, in particular for the cloning and/or expression in particular of the plasmid, cosmid or phage, characterized in that it contains a recombinant nucleic acid according to one of the Claims 14 to 23 at one of its sites inessential for its replication.

26. Recombinant vector according to Claim 25, characterized in that it is the plasmid PSHV-G1 deposited with the CNCM under No. I-778.

27. Recombinant vector according to Claim 25, characterized in that it contains at one of its sites inessential for its replication, elements necessary for promoting the expression of an amino acid sequence according to the Claims 1 to 13 in a cell host, and optionally a promoter recognized by the polymerases of the cell host, in particular an inducible promoter and/or an anchoring sequence.

28. Recombinant vector according to Claim 27, characterized in that it contains
   - the elements allowing the expression of this nucleic acid according to the Claims 11 to 20 inserted in the vector by E. coli, and in particular
   - the elements allowing the expression of all or part of the beta-galactosidase gene.

29. Recombinant vector according to Claim 27, characterized in that it contains the elements enabling the nucleic acid according to the Claims 14 to 23 to be inserted into yeast and for this nucleic acid to be expressed in yeast.

30. Cell host transformed by a recombinant vector according to any one of the Claims 25 to 29 and comprising the regulatory elements allowing the expression of the nucleic acid sequence coding for the polypeptide according to one of the Claims 1 to 13 in this host.

31. Cell host according to Claim 30, characterized in that it is E. coli transformed by the vector according to Claim 28.

32. Transformed cell host according to Claim 30, characterized in that it is a yeast transformed by the vector according to Claim 29.

33. Expression product of a nucleic acid expressed by a cell host transformed according to the Claims 30 to 32.

34. Active ingredient of a vaccine characterized in that it comprises the expression product of Claim 33, optionally coupled to a natural protein or to a synthetic polypeptide having a molecular weight sufficiently high for the conjugate to be capable of inducing in vivo the production of neutralising anti-VHS antibodies.

35. Active ingredient of a vaccine according to Claim 34 which can be administered by the oral route, characterized in that it contains from 10 ng to 100 $\mu$g of active ingredient per gram of ingested vaccine.

36. Active ingredient of a vaccine according to Claim 34, which can be administered by the intraperitoneal route, characterized in that it contains from 1 to 1000 ng/g of fish.

37. Active principle of a vaccine according to Claim 34 which can be administered by balneotherapy in a medium containing from 10 ng to 100 $\mu$g/ml.

38. Nucleotide probe characterized in that it hybridizes with one of the nucleic acids according to the Claims 14 to 23 or with their complementary sequence, and, in particular, the probes selected from the following nucleotide sequences :

   ACG TTG GAG GGA AAG GCC
   GAC TGG GAC ACT CCG CTA
   CCC TCC TGC ATC TGG ATG
   AAG ACT GAC CTG GGG GAC
   GAC AAC AAC ACA GAC GGG
   TTC CTT CTA CTG GTA CTC

   or their complementary nucleotide sequences.

**39.** Procedure for the preparation of a recombinant polypeptide according to any one of the Claims 1 to 13, characterized in that
- a cell host previously transformed by a suitable vector containing a nucleic acid according to one of the Claims 14 to 23 is cultured in a suitable culture medium and
- the polypeptide produced by the said transformed cell host is recovered from the said culture medium.

**40.** Procedure according to Claim 39 for the preparation of specific recombinant polypeptide characterized by the placing of E. coli according to Claim 31 in culture and by interruption of the culture at the end of the exponential phase of growth of the said E. coli, and recovery of the specific amino acid sequence from the culture medium free of the other extracellular proteins and enzymes capable of being secreted by E. coli.

**41.** Procedure according to Claim 40 for the preparation of a specific polypeptide characterized by the placing of the yeast according Claim 32 in culture, and interruption of the culture at the end of the exponential phase of growth of the said yeast and recovery of the polypeptide micro-encapsulated in the yeast.

**42.** Polypeptide according to any one of the Claims 1 or 3, characterized in that it is constituted by the amino acid sequence defined between the residues 111 and 302, contained in the amino acid sequence shown in Figure 3.

**43.** Nucleic acid, in particular a DNA fragment, coding for the polypeptide defined in Claim 42.

**44.** Monoclonal antibody prepared from an immunogenic recombinant polypeptide according to any one of the Claims 1 to 13, and characterized in that it is directed against any one of these polypeptides.

**45.** Monoclonal antibody according to Claim 44, characterized in that it specifically recognizes the polypeptide of Claim 42.

**Claims for the following Contracting State : ES**

**1.** Procedure for the preparation of a polypeptide containing in its polypeptide backbone an amino acid sequence (I) contained in the following sequence

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-

Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp

characterized in that, starting preferably from the C-terminal amino acid, the successive aminoacyl residues are condensed successively with each other in the required order, or aminoacyl residues are condensed with fragments previously formed which already contain several amino acids in the correct order, or also several fragments thus prepared beforehand are condensed, it being understood that care will be taken to protect beforehand all of the reactive functions borne by these aminoacyl residues or fragments, with the exception of the amino function of the one and the carboxyl function of the other or vice versa, which are usually required to participate in the formation of the peptide bonds, in particular after activation of the carboxyl function according to the methods well known in peptide synthesis and so on, one after the other, until the N-terminal amino acid is reached.

2. Preparative procedure according to Claim 1 for a polypeptide recognized by neutralizing antibodies formed beforehand against the VHS virus.

3. Preparative procedure according to Claim 1 for a polypeptide capable of inducing in vivo neutralizing antibodies against the VHS virus.

4. Preparative procedure according to Claim 1 for a polypeptide which contains in its peptide backbone the following amino acid sequence (II):

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-

Ser-Asn-Thr-Ser-Asp-Leu-Ser-Thr-Asn-His-Ile-His-Thr-
Asn-Leu-Ile-Pro-Ser-Asp
```

5. Preparative procedure according to Claim 1 for a polypeptide containing all or part of the following amino acid sequence (III) :

```
Met Arg Gly Asp Cys Asp Tyr Glu Ala Val Val Ser Ile
Asn Tyr Asn Arg Ala Gln Tyr Lys Thr Met Asn Asn Thr
Trp Lys Ser Trp Lys Arg Val Asp Asn Asn Thr Asp Gly
Tyr Asp Gly Met Ile Phe Gly Asp Lys Leu Ile Ile Pro
Asp Ile Glu Lys Tyr Gln Ser Val Tyr Asp Ser Gly Met
Leu Val Gln Arg Asn Leu Val Glu Val Pro His
```

6. Preparative procedure according to Claim 1 for a polypeptide containing all or part of the following amino acid sequence :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

7. Preparative procedure according to Claim 1 for a polypeptide constituted by the following amino acid sequence I :

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
```

99

```
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

8.  Preparative procedure according to Claim 1 for a polypeptide constituted by the following amino acid sequence (II) :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
```

Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

**9.** Preparative procedure according to Claim 1 for a polypeptide constituted by the following amino acid sequence (III) :

Met Arg Gly Asp Cys Asp Tyr Glu Ala Val Val Ser Ile
Asn Tyr Asn Arg Ala Gln Tyr Lys Thr Met Asn Asn Thr
Trp Lys Ser Trp Lys Arg Val Asp Asn Asn Thr Asp Gly
Tyr Asp Gly Met Ile Phe Gly Asp Lys Leu Ile Ile Pro
Asp Ile Glu Lys Tyr Gln Ser Val Tyr Asp Ser Gly Met
Leu Val Gln Arg Asn Leu Val Glu Val Pro His

EP 0 377 349 B1

10. Preparative procedure according to Claim 1 for a polypeptide constituted by the following amino acid sequence IV :

```
Met-Glu-Trp-Asn-Thr-Phe-Phe-Leu-Val-Ile-Leu-Ile-Ile-
Ile-Ile-Lys-Ser-Thr-Thr-Pro
```

11. Preparative procedure according to Claim 1 for an amino acid sequence constituted by an amino acid sequence defined in the Claims 1 to 9 and a protein or amino acid sequence heterologous with respect to the said sequence comprising from about 8 to about 800 amino acids.

12. Preparative procedure according to Claim 1 of an amino acid sequence defined in claim 11 in which the foreign amino acid sequence is beta-galactosidase.

13. Preparative procedure for a nucleic acid comprising all or part of the following nucleotide sequence (1) :

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG GTC GAA
```

102

```
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GCG CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

comprising the following steps :
- the centrifugation of the supernatant of a cell culture infected with an isolate of the VHS virus type I and the recovery of the centrifugation pellet;
- optionally, the centrifugation of the recovered pellet in a sucrose gradient by the method described in the patent EP-A-0138667,

- the recovery of the portion of the gradient containing the virus and the resedimentation of the virus by centrifugation,
- the resuspension of the viral pellet and extraction of the genomic RNA according to the procedure described by Chomzynski et al. (Anal. Biochem. 162? 156, 1987).
- the synthesis of a cDNA strand in contact with the genomic RNA of the virus by a reverse transcriptase in the presence of oligomeric initiators of random sequence according to the procedure described in DNA 4 : 429-438, 1985,
- optionally, the purification of the RNA/cDNA hybrids by extraction with a phenol/chloroform mixture and precipitation with ethanol,
- the production of a second cDNA strand in the presence of DNA polymerase I, RNAse H and the 4 deoxynucleotides according to the method described in Gene, 25 / 263-269 (1983°, if necessary after extraction of the proteins present in the reaction medium,
- the cloning of the nucleic acids thus obtained in a suitable plasmid vector and the recovery of the desired nucleic acid with the aid of an appropriate probe.

**14.** Preparative procedure for a nucleic acid comprising all or part of the following nucleotide sequence (1) :

```
TCT TTG GAG AAA CCA GTG AGA AGA TTG ACT TCG GGA CTC

GCA GTA AGA CCC TGA TAA CAA GCT TCA AGA TAG CTG AAG

CAA AGG CCA TCT ACC TGG ATT CAA GTC CGG TGA GAT CTC

GCA TAG AGG CCA AGA AGT ACA CCA CTC CCA TTA GAC ATG

GGA GTG TGA CTT ACT ATG GTC CGT TCA TAT TTG CAG ATG

ACC ACG TTG GAG GGA AAG GCC ACC GCG AGA AGC TAG GGG

CAC TAT GTG GAT TCC TCC AGT CTG GAC CTT ACG GGC AGG

CGA AGG ACT ACT ACA ATC GTG CCG TCG AAG AAG AGA TAG

GCA TTC CCC CAA GGG ACC CGA AGC GCA GAT CTG GAA CCT

CCT CTG TCC GAC CTT GGT AGA CCG AAA GGA CTG ACC CAG

GTT TGA GAC CAC ACT CTC ATT AGA TAG AAA AAA ATG GCA

CAT TTG TGT ACA CAA CAA GCT AGA CCC ACA ATG GAA TGC
```

```
AAC ACT TTT TTC TTG GTG ATC TTG ATC ATC ATC ATA AAG
AGC ACC ACA CCA CAG ATC ACT CAA CGA CCT CCG GTC GAA
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GGG CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

comprising the following steps :
- the preparation of the cellular and viral RNAs from cell cultures infected for 16 to 20 hours by the VHS type I virus to a multiplicity of 10 pfu per cell according to the method described by Chomzynski et al. (Anal. Biochem. 162, 156, 1987),
- the recovery and purification of the cellular and viral RNAs by chromatography of the total cellular and viral RNAs on a column of oligo (dT),

- the synthesis of a cDNA strand from the purified mRNAs according to the procedure described in Gene 25 : 263, 1983,
- the cloning of the nucleic acids thus obtained in a suitable plasmid vector and the recovery of the desired nucleic acid sequence with the aid of a suitable hybridization probe.

**15.** Preparative procedure for a nucleic acid comprising all or part of the following nucleotide sequence (1) :

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG GTC GAA
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
```

106

```
GTA GCG AGT GGC CAC TAC CTG CAC AGA GTG ACT TAT CGA
GTC ACC TGT TCG ACC AGC TTC TTT GGA GGG CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT

ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

comprising the following steps :

When the nucleic acid to be prepared contains a maximum of 200 nucleotides
- the synthesis of DNA using the automated beta-cyanoethyl phosphoramidite method described in Bioorganic chemistry 4 ; 274-325, 1986,
- the cloning of the DNAs thus obtained in a suitable plasmid vector and the recovery of the DNAs by hybridization with a suitable probe,

and when the nucleic acid contains more than 200 nucleotides :
- the assembly of chemically synthesized oligonucleotides provided at their ends with different restriction sites, the sequences of which are compatible with the amino acid sequence of the natural polypeptide according to the principle described in Proc. Nat. Acad. Sci. USA 80; 7461-7465, 1983,
- the cloning of the DNAs thus obtained in a suitable plasmid vector and the recovery of the desired nucleic acid by hybridization with a suitable probe.

**16.** Procedure according to one of the Claims 13, 14 or 15 for the preparation of a nucleic acid containing all or part of the following nucleotide sequence (2) :

```
                            ATG GAA TGG AAC ACT TTT TTC TTG
        GTG ATC TTG ATC ATC ATC ATA AAG AGC ACC ACA CCA CAG
        ATC ACT CAA CGA CCT CCG GTC GAA AAC ATC TCG ACG TAC
        CAT GCA GAC TGG GAC ACT CCG CTA TAC ACT CAT CCC TCC
        AAC TGC AGG GAC GAT TCC TTT GTC CCG ATT CGA CCA GCT
        CAA CTC AGG TGT CCT CAT GAA TTT GAA GAC ATA AAC AAG
        GGA CTG GTT TCC GTC CCA ACC AGG ATC ATC CAT CTC CCG
        CTA TCG GTC ACC AGC GTC TCC GCA GTA GCG AGT GGC CAC
        TAC CTG CAC AGA GTG ACT TAT CGA GTC ACC TGT TCG ACC
        AGC TTC TTT GGA GGG CAA ACC ATC GAA AAG ACC ATC TTG
        GAG GCG AAA CTG TCT CGT CAG GAG GCC ACA GAC GAG GCA
        AGC AAG GAT CAC GAG TAC CCG TTC TTC CCT GAA CCC TCC
        TGC ATC TGG ATG AAA AAC AAT GTC CAT AAG GAC ATA ACT
        CAC TAT TAC AAG ACC CCA AAA ACA GTA TCG GTG GAT CTC
        TAC AGC AGG AAA TTT CTC AAC CCT GAT TTC ATA GAG GGG
        GTT TGC ACA ACC TCG CCC TGT CAA ACT CAT TGG CAG GGA
```

```
GTC TAT TGG GTC GGT GCC ACA CCT AAA GCC CAT TGC CCC
ACG TCG GAA ACA CTA GAA GGA CAC CTG TTC ACC AGG ACC
CAT GAT CAC AGG GTG GTC AAG GCA ATT GTG GCA GGC CAT
CAT CCC TGG GGA CTC ACA ATG GCA TGC ACA GTG ACA TTC
TGC GGG ACA GAA TGG ATC AAG ACT GAC CTG GGG GAC CTG
ATC CAG GTG ACA GGA CCG GGG GGC ACG AGG AAA CTG ACT
CCA AAC AAG TGT GTC AAT ACC GAT ATC CAG ATG AGG GGG
GCA ACA GAC GAC TTT TCT TAT CTC AAC CAT CTC ATC ACC
AAC ATG GCT CAA AGA ACC GAG TGC CTA GAT GCC CAT AGT
GAT ATC ACC GCT TCT GGG AAA GTA TCC TCA TTT CTC CTC
TCA AAG TTT CGT CCC AGC CAC CCT GGA CCT GGC AAG GCA
CAC TAT CTT CTC GAC GGT CAA ATC ATG CGA GGT GAC TGT
GAC TAT GAG GCA GTA GTC AGC ATC AAC TAC AAT CGC GCT
CAA TAC AAG ACG ATG AAC AAC ACA TGG AAA TCA TGG AAA

CGG GTA GAC AAC AAC ACA GAC GGG TAC GAT GGG ATG ATA
TTT GGG GAC AAA TTG ATC ATC CCG GAC ATC GAA AAG TAT
CAG AGT GTC TAT GAC AGT GGA ATG CTC GTT CAA AGA AAC
CTT GTG GAA GTC CCT CAT CTG AGC ATT GTG TTT GTC TCC
AAC ACA TCT GAT CTT TCC ACT AAT CAC ATC CAC ACC AAC
CTA ATC CCT TCG GAT
```

17. Procedure according to one of the Claims 13, 14 or 15 for the preparation of a nucleic acid containing all or part of the following nucleotide sequence (3) :

```
CAG ATC ACT CAA CGA CCT CCG GTC GAA AAC ATC TCG ACG
TAC CAT GCA GAC TGG GAC ACT CCG CTA TAC ACT CAT CCC
TCC AAC TGC AGG GAC GAT TCC TTT GTC CCG ATT CGA CCA
GCT CAA CTC AGG TGT CCT CAT GAA TTT GAA GAC ATA AAC
AAG GGA CTG GTT TCC GTC CCA ACC AGG ATC ATC CAT CTC
CCG CTA TCG GTC ACC AGC GTC TCC GCA GTA GCG AGT GGC
CAC TAC CTG CAC AGA GTG ACT TAT CGA GTC ACC TGT TCG
ACC AGC TTC TTT GGA GGG CAA ACC ATC GAA AAG ACC ATC
TTG GAG GCG AAA CTG TCT CGT CAG GAG GCC ACA GAC GAG
GCA AGC AAG GAT CAC CAG TAC CCG TTC TTC CCT GAA CCC
TCC TGC ATC TGG ATG AAA AAC AAT GTC CAT AAG GAC ATA
ACT CAC TAT TAC AAG ACC CCA AAA ACA GTA TCG GTG GAT
```

109

```
CTC TAC AGC AGG AAA TTT CTC AAC CCT GAT TTC ATA GAG
GGG GTT TGC ACA ACC TCG CCC TGT CAA ACT CAT TGG CAG
GGA GTC TAT TGG GTC GGT GCC ACA CCT AAA GCC CAT TGC
CCC ACG TCG GAA ACA CTA GAA GGA CAC CTG TTC ACC AGG
ACC CAT GAT CAC AGG GTG GTC AAG GCA ATT GTG GCA GGC
CAT CAT CCC TGG GGA CTC ACA ATG GCA TGC ACA GTG ACA
TTC TGC GGG ACA GAA TGG ATC AAG ACT GAC CTG GGG GAC
CTG ATC CAG GTG ACA GGA CCG GGG GGC ACG AGG AAA CTG
ACT CCA AAC AAG TGT GTC AAT ACC GAT ATC CAG ATG AGG
GGG GCA ACA GAC GAC TTT TCT TAT CTC AAC CAT CTC ATC
ACC AAC ATG GCT CAA AGA ACC GAG TGC CTA GAT GCC CAT
AGT GAT ATC ACC GCT TCT GGG AAA GTA TCC TCA TTT CTC
```

```
CTC TCA AAG TTT CGT CCC AGC CAC CCT GGA CCT GGC AAG
GCA CAC TAT CTT CTC GAC GGT CAA ATC ATG CGA GGT GAC
TGT GAC TAT GAG GCA GTA GTC AGC ATC AAC TAC AAT CGC
GCT CAA TAC AAG-ACG ATG AAC AAC ACA TGG AAA TCA TGG
AAA CGG GTA GAC AAC AAC ACA GAC GGG TAC GAT GGG ATG
ATA TTT GGG GAC AAA TTG ATC ATC CCG GAC ATC GAA AAG
TAT CAG AGT GTC TAT GAC AGT GGA ATG CTC GTT CAA AGA
AAC CTT GTG GAA GTC CCT CAT CTG AGC ATT GTG TTT GTC
TCC AAC ACA TCT GAT CTT TCC ACT AAT CAC ATC CAC ACC
AAC CTA ATC CCT TCG GAT
```

**18.** Procedure according to one of the Claims 13, 14 or 15 for the preparation of a nucleic acid containing all or part of the following nucleotide sequence (4) :

```
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT
```

**19.** Procedure according to one of the Claims 13, 14 or 15 for the preparation of a nucleic acid containing all or part of the following nucleotide sequence (5) :

110

EP 0 377 349 B1

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA
```

**20.** Procedure according to one of the Claims 13, 14 or 15 for the preparation of a nucleic acid constituted by the following nucleotide sequence (6) :

```
        TGG TCA TTC AAC TGG AGT CTT TGG CCA TCA TTA
TCT GGG ATG GGG GTT GTG GGA GGG GCC TTC CTT CTA CTG
GTA CTC TGC TGT TGC TGC AAG GCG TCC CCT CCC ATT CCA
AAT TAC GGG ATT CCG ATG CAG CAG TTC TCC AGA AGT CAG
ACG GTC TGA GCA CAC CTG TCC GAA TGA CCA CAA TTC CTC
TCT TAG GTA GAT AGA AAA AAA AAA AAA AAA AAA AAA AAA
```

**21.** Procedure for the production of antibodies comprising the immunization of an animal with the aid of a polypeptide defined in the claims 1 to 13 and the recovery of the antibodies formed.

**22.** Preparative procedure for a nucleotide probe of the following sequence :
ACG TTG GAG GGA AAG GCC
GAC TGG GAC ACT CCG CTA
CCC TCC TGC ATC TGG ATG
AAG ACT GAC CTG GGG GAC
GAC AAC AAC ACA GAC GGG
TTC CTT CTA CTG GTA CTC
characterized in that the synthesis of the DNA is carried out by using the automated beta-cyanoethyl phosphoramidite method described in Bioorganic Chemistry 4; 274-325, 1986.

**23.** Procedure for the detection of a nucleotide sequence defined in the Claims 13 to 19, characterized in that :
- a prehybridization step of the target RNA, previously made accessible, is carried out with one of the probes of Claim 22 under the following conditions : treatment for 16 hours at 56°C in the prehybridization medium containing the following buffer: 3M tetramethylammonium chloride, 50 mM Tris- Tris-HCl, pH 8.0, 2 mM EDTA, 100 $\mu$g/ml sonicated, denatured calf thymus DNA, 5 X Denhardt,
- hybridization is carried out under the following conditions : treatment for 1 hour at the same temperature with about 100 pg of one of the probes or a mixture of the probes defined in Claim

111

EP 0 377 349 B1

22,
- washing is then performed under the following conditions :
  ° 5 minutes in 2X SSC, 0.1% SDS at room temperature,
  ° 5 minutes in 5X SSC, 0.1% SDS at 59°C,
  ° two passages in the above hybridization buffer, without Denhardt and calf DNA for 1 h at 59°C.

24. Preparative procedure for a polypeptide defined in any one of the Claims 1 to 12, comprising the following steps :
  - the culture of a competent cell host previously transformed by a nucleic acid containing a nucleotide sequence coding for the polypeptides defined in one of the Claims 1 to 21 and in which this nucleotide sequence is placed under the control of regulatory elements, including in particular a promoter recognized by the polymerases of this competent host, the elements for the initiation and termination of translation and
  - the recovery of the polypeptide produced from the expression products of this cell host.

25. Preparative procedure according to Claim 24 for a specific recombinant polypeptide characterized by the placing in culture of E. coli transformed by the vector containing at one of its sites inessential for its replication elements necessary to promote the expression of an amino acid sequence defined in the Claims 1 to 12 in a cell host, and optionally a promoter recognized by the polymerases of the cell host, in particular an inducible promoter and optionally a signal sequence and/or an anchoring sequence, and in particular
  - the elements permitting the expression of all or part of the beta-galactosidase gene,
    and by interruption of the culture at the end of the exponential phase of growth of the said E. coli,
  - and recovery of the specific amino acid sequence from the culture medium free from the other extracellular proteins and enzymes likely to be secreted by E. coli.

26. Preparative procedure according to Claim 24 of a specific polypeptide characterized by the placing in culture of a yeast transformed by the vector containing the elements making it possible to insert any one of the nucleic acids defined in the Claims 13 to 19 into the yeast, and the expression of this nucleic acid in the yeast, and by interruption of the culture at the end of the exponential phase of growth of the said yeast, and recovery of the polypeptide micro-encapsulated in the yeast.

27. Preparative procedure according to any one of the Claims 1, 24, 25 or 26, for a polypeptide constituted by the amino acid sequence defined between the residues 111 and 302 and contained in the amino acid sequence shown in Figure 3.

28. Preparative procedure according to any one of the Claims 13, 14 or 15 for a nucleic acid, in particular DNA fragment, coding for the polypeptide constituted by the amino acid sequence defined between the residues 111 and 302 and contained in the amino acid sequence shown in Figure 3.

29. Procedure for the production of monoclonal antibodies comprising the culture of hybridomas prepared from polypeptides obtained by the procedure according to any one of the Claims 1 to 12 or 24 to 27.

**Claims for the following Contracting State : GR**

1. Recombinant polypeptide characterized by the presence in its polypeptide backbone of an amino acid sequence (I) contained in the following sequence :

112

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-

```
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

and by the absence of natural contaminants, derived from the virus responsible for the viral hemorrhagic septicemia.

2. Polypeptide according to Claim 1, characterized in that it is recognized by neutralizing antibodies previously formed against the VHS virus.

3. Recombinant polypeptide according to Claim 2, characterized in that it is capable of inducing in vivo neutralizing antibodies against the VHS virus.

4. Recombinant polypeptide according to any one of the Claims 1 to 3, characterized by the presence in its peptide backbone of the following amino acid sequence (II) :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
```

EP 0 377 349 B1

His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

and by the absence of natural contaminants, derived from the virus responsible for the viral hemorrhagic septicemia.

5. Polypeptide according to any one of the Claims 1 to 3, characterized in that it contains all or part of the following amino acids sequence (III) :

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

6. Polypeptide according to any one of the Claims 1 and 3, characterized in that it contains all or part of the following amino acid sequence :

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

115

7. Polypeptide according to any one of the Claims 1 to 3, characterized in that it is constituted by the following amino acid sequence I :

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
```

```
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

8. Polypeptide according to any one of the Claims 1 to 3, characterized in that it is constituted by the following amino acid sequence (II) :

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
```

EP 0 377 349 B1

Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

9. Polypeptide according to any one of the Claims 1 to 3, characterized in that it is constituted by the following amino acid sequence (III) :

Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His

10. Polypeptide according to any one of the Claims 1 to 3, characterized in that it is constituted by the following amino acid sequence IV :

Met-Glu-Trp-Asn-Thr-Phe-Phe-Leu-Val-Ile-Leu-Ile-Ile-
Ile-Ile-Lys-Ser-Thr-Thr-Pro

11. Amino acid sequences according to Claims 1 to 9, characterized in that it is constituted by an amino acid sequence according to Claims 1 to 9 and a protein or an amino acid sequence heterologous with respect to the said sequence comprising from about 8 to about 800 amino acids.

12. Amino acid sequence according to Claim 11, characterized in that the foreign amino acid sequence is beta-galactosidase.

13. Polypeptide characterized by the presence of a peptide backbone comprising all or part of an amino acid sequence encoded by the following nucleic acid (1) :

TCT TTG GAG AAA CCA GTG AGA AGA TTG ACT TCG GGA CTC GCA
GTA AGA CCC TGA TAA CAA GCT TCA AGA TAG CTG AAG CAA AGG

118

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA ATG

GAA TGG AAC ACT TTT TTC TTG GTG ATC TTG ATC ATC ATC ATA

AAG AGC ACC ACA CCA CAG ATC ACT CAA CGA CCT CCG GTC GAA

AAC ATC TCG ACG TAC CAT GCA GAC TGG GAC ACT CCG CTA TAC

ACT CAT CCC TCC AAC TGC AGG GAC GAT TCC TTT GTC CCG ATT

CGA CCA GCT CAA CTC AGG TGT CCT CAT GAA TTT GAA GAC ATA

AAC AAG GGA CTG GTT TCC GTC CCA ACC AGG ATC ATC CAT CTC

CCG CTA TCG GTC ACC AGC GTC TCC GCA GTA GCG AGT GGC CAC

TAC CTG CAC AGA GTG ACT TAT CGA GTC ACC TGT TCG ACC AGC

TTC TTT GGA GGG CAA ACC ATC GAA AAG ACC ATC TTG GAG GCG

AAA CTG TCT CGT CAG GAG GCC ACA GAC GAG GCA AGC AAG GAT

CAC GAG TAC CCG TTC TTC CCT GAA CCC TCC TGC ATC TGG ATG

AAA AAC AAT GTC CAT AAG GAC ATA ACT CAC TAT TAC AAG ACC

CCA AAA ACA GTA TCG GTG GAT CTC TAC AGC AGG AAA TTT CTC

AAC CCT GAT TTC ATA GAG GGG GTT TGC ACA ACC TCG CCC TGT

CAA ACT CAT TGG CAG GGA GTC TAT TGG GTC GGT GCC ACA CCT

AAA GCC CAT TGC CCC ACG TCG GAA ACA CTA GAA GGA CAC CTG

TTC ACC AGG ACC CAT GAT CAC AGG GTG GTC AAG GCA ATT GTG

GCA GGC CAT CAT CCC TGG GGA CTC ACA ATG GCA TGC ACA GTG

ACA TTC TGC GGG ACA GAA TGG ATC AAG ACT GAC CTC GGG GAC

CTG ATC CAG GTG ACA GGA CCG GGG GGC ACG AGG AAA CTG ACT

CCA AAC AAG TGT GTC AAT ACC GAT ATC CAG ATG AGG GGG GCA

ACA GAC GAC TTT TCT TAT CTC AAC CAT CTC ATC ACC AAC ATG

GCT CAA AGA ACC GAG TGC CTA GAT GCC CAT AGT GAT ATC ACC

GCT TCT GGG AAA GTA TCC TCA TTT CTC CTC TCA AAG TTT CGT

CCC AGC CAC CCT GGA CCT GGC AAG GCA CAC TAT CTT CTC GAC

GGT CAA ATC ATG CGA GGT GAC TGT GAC TAT GAG GCA GTA GTC

AGC ATC AAC TAC AAT CGC GCT CAA TAC AAG ACG ATG AAC AAC

119

```
ACA TGG AAA TCA TGG AAA CGG GTA GAC AAC AAC ACA GAC GGG

TAC GAT GGG ATG ATA TTT GGG GAC AAA TTG ATC ATC CCG GAC

ATC GAA AAG TAT CAG AGT GTC TAT GAC AGT GGA ATG CTC GTT

CAA AGA AAC CTT GTG GAA GTC CCT CAT CTG AGC ATT GTG TTT

GTC TCC AAC ACA TCT GAT CTT TCC ACT AAT CAC ATC CAC ACC

AAC CTA ATC CCT TCG GAT
```

14. Nucleic acid characterized in that it comprises a nucleotide sequence coding for the polypeptides according to Claims 1 to 13.

15. Nucleic acid according to Claim 14, characterized in that it comprises all or part of the following nucleotide sequence (2) :

```
                                ATG GAA TGG AAC ACT TTT TTC TTG GTG

ATC TTG ATC ATC ATC ATA AAG AGC ACC ACA CCA CAG ATC ACT

CAA CGA CCT CCG GTC GAA AAC ATC TCG ACG TAC CAT GCA GAC

TGG GAC ACT CCG CTA TAC ACT CAT CCC TCC AAC TGC AGG GAC

GAT TCC TTT GTC CCG ATT CGA CCA GCT CAA CTC AGG TGT CCT

CAT GAA TTT GAA GAC ATA AAC AAG GGA CTG GTT CCG TCC CCA

ACC AGG ATC ATC CAT CTC CCG CTA TCG GTC ACC AGC GTC TCC

GCA GTA GCG AGT GGC CAC TAC CTG CAC AGA GTG ACT TAT CGA

GTC ACC TGT TCG ACC AGC TTC TTT GGA GGG CAA ACC ATC GAA

AAG ACC ATC TTG GAG GCG AAA CTG TCT CGT CAG GAG GCC ACA

GAC GAG GCA AGC AAG GAT CAC GAG TAC CCG TTC TTC CCT GAA

CCC TCC TGC ATC TGG ATG AAA AAC AAT GTC CAT AAG GAC ATA

ACT CAC TAT TAC AAG ACC CCA AAA ACA GTA TCG GTG GAT CTC

TAC AGC AGG AAA TTT CTC AAC CCT GAT TTC ATA GAG GGG GTT

TGC ACA ACC TCG CCC TGT CAA ACT CAT TGG CAG GGA GTC TAT

TGG GTC GGT GCC ACA CCT AAA GCC CAT TGC CCC ACG TCG GAA

ACA CTA GAA GGA CAC CTG TTC ACC AGG ACC CAT GAT CAC AGG

GTG GTC AAG GCA ATT GTG GCA GGC CAT CAT CCC TGG GGA CTC

ACA ATG GCA TGC ACA GTG ACA TTC TGC GGG ACA GAA TGG ATC

AAG ACT GAC CTG GGG GAC CTG ATC CAG GTG ACA GGA CCG GGG

GGC ACG AGG AAA CTG ACT CCA AAC AAG TGT GTC AAT ACC GAT

ATC CAG ATG AGG GGG GCA ACA GAC GAC TTT TCT TAT CTC AAC

CAT CTC ATC ACC AAC ATG GCT CAA AGA ACC GAG TGC CTA GAT

GCC CAT AGT GAT ATC ACC GCT TCT GGG AAA GTA TCC TCA TTT
```

120

```
CTC CTC TCA AAG TTT CGT CCC AGC CAC CCT GGA CCT GGC AAG
GCA CAC TAT CTT CTC GAC GGT CAA ATC ATG CGA GGT GAC TGT
GAC TAT GAG GCA GTA GTC AGC ATC AAC TAC AAT CGC GCT CAA
TAC AAG ACG ATG AAC AAC ACA TGG AAA TCA TGG AAA CGG GTA
GAC AAC AAC ACA GAC GGG TAC GAT GGG ATG ATA TTT GGG GAC
AAA TTG ATC ATC CCG GAC ATC GAA AAG TAT CAG AGT GTC TAT
GAC AGT GGA ATG CTC GTT CAA AGA AAC CTT GTG GAA GTC CCT
CAT CTG AGC ATT GTG TTT GTC TCC AAC ACA TCT GAT CTT TCC
ACT AAT CAC ATC CAC ACC AAC CTA ATC CCT TCG GAT
```

16. Nucleic acid characterized in that it comprises all or part of the following nucleotide sequence (3) :

```
CAG ATC ACT CAA CGA CCT CCG GTC GAA AAC ATC TCG ACG TAC
CAT GCA GAC TGG GAC ACT CCG CTA TAC ACT CAT CCC TCC AAC
TGC AGG GAC GAT TCC TTT GTC CCG ATT CGA CCA GCT CAA CTC
AGG TGT CCT CAT GAA TTT GAA GAC ATA AAC AAG GGA CTG GTT
TCC GTC CCA ACC AGG ATC ATC CAT CTC CCG CTA TCG GTC ACC
AGC GTC TCC GCA GTA GCG AGT GGC CAC TAC CTG CAC AGA GTG
ACT TAT CGA GTC ACC TGT TCG ACC AGC TTC TTT GGA GGG CAA
ACC ATC GAA AAG ACC ATC TTG GAG GCG AAA CTG TCT CGT CAG
GAG GCC ACA GAC GAG GCA AGC AAG GAT CAC CAG TAC CCG TTC
TTC CCT GAA CCC TCC TGC ATC TGG ATG AAA AAC AAT GTC CAT
AAG GAC ATA ACT CAC TAT TAC AAG ACC CCA AAA ACA GTA TCG
GTG GAT CTC TAC AGC AGG AAA TTT CTC AAC CCT GAT TTC ATA
GAG GGG GTT TGC ACA ACC TCG CCC TGT CAA ACT CAT TGG CAG
GGA GTC TAT TGG GTC GGT GCC ACA CCT AAA GCC CAT TGC CCC
ACG TCG GAA ACA CTA GAA GGA CAC CTG TTC ACC AGG ACC CAT
GAT CAC AGG GTG GTC AAG GCA ATT GTG GCA GGC CAT CAT CCC
TGG GGA CTC ACA ATG GCA TGC ACA GTG ACA TTC TGC GGG ACA
GAA TGG ATC AAG ACT GAC CTG GGG GAC CTG ATC CAG GTG ACA
GGA CCG GGG GGC ACG AGG AAA CTG ACT CCA AAC AAG TGT GTC
AAT ACC GAT ATC CAG ATG AGG GGG GCA ACA GAC GAC TTT TCT
TAT CTC AAC CAT CTC ATC ACC AAC ATG GCT CAA AGA ACC GAG
TGC CTA GAT GCC CAT AGT GAT ATC ACC GCT TCT GGG AAA GTA
TCC TCA TTT CTC CTC TCA AAG TTT CGT CCC AGC CAC CCT GGA
CCT GGC AAG GCA CAC TAT CTT CTC GAC GGT CAA ATC ATG CGA
```

121

```
GGT GAC TGT GAC TAT GAG GCA GTA GTC AGC ATC AAC TAC AAT
CGC GCT CAA TAC AAG ACG ATG AAC AAC ACA TGG AAA TCA TGG
AAA CGG GTA GAC AAC AAC ACA GAC GGG TAC GAT GGG ATG ATA
TTT GGG GAC AAA TTG ATC ATC CCG GAC ATC GAA AAG TAT CAG.
AGT GTC TAT GAC AGT GGA ATG CTC GTT CAA AGA AAC CTT GTG
GAA GTC CCT CAT CTG AGC ATT GTG TTT GTC TCC AAC ACA TCT
GAT CTT TCC ACT AAT CAC ATC CAC ACC AAC CTA ATC CCT TCG
GAT
```

17. Nucleic acid characterized in that it comprises all or part of the following nucleotide sequence (4) :

```
ATG CGA GGT GAC TGT GAC TAT GAG GCA GTA GTC AGC ATC AAC
TAC AAT CGC GCT CAA TAC AAG ACG ATG AAC AAC ACA TGG AAA
TCA TGG AAA CGG GTA GAC AAC AAC ACA GAC GGG TAC GAT GGG
ATG ATA TTT GGG GAC AAA TTG ATC ATC CCG GAC ATC GAA AAG
TAT CAG AGT GTC TAT GAC AGT GGA ATG CTC GTT CAA AGA AAC
CTT GTG GAA GTC CCT CAT
```

18. Nucleic acid characterized in that it comprises all or part of the following nucleotide sequence (5) :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA
```

**19.** Nucleic acid characterized in that it comprises all or part of the following nucleotide sequence (6) :

```
                ' TGG TCA TTC AAC TGG AGT CTT TGG CCA TCA TTA TCT
GGG ATG GGG GTT GTG GGA GGG GCC TTC CTT CTA CTG GTA CTC

TGC TGT TGC TGC AAG GCG TCC CCT CCC ATT CCA AAT TAC GGG
ATT CCG ATG CAG CAG TTC TCC AGA AGT CAG ACG GTC TGA GCA
CAC CTG TCC GAA TGA CCA CAA TTC CTC TCT TAG GTA GAT AGA
AAA AAA AAA AAA AAA AAA AAA AAA A
```

**20.** Nucleic acid according to Claim 15, characterized in that it is preceded by a nucleotide sequence comprising the regulatory elements for the expression of this latter.

**21.** Nucleic acid according to Claims 15 and 20, characterized in that it comprises the termination elements for transcription of this latter.

**22.** Nucleic acid characterized in that it comprises all or part of the following nucleotide sequence (1) :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
```

```
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**23.** Nucleic acid according to Claim 22, characterized in that it is constituted by the following nucleotide sequence (1) :

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA·AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
```

124

```
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

24. Recombinant nucleic acid, characterized in that it contains the nucleic acid according to any one of the Claims 14 to 23, inserted into a nucleic acid heterologous with respect to the above-mentioned fragment.

**25.** Recombinant vector, in particular for the cloning and/or expression in particular of the plasmid, cosmid or phage, characterized in that it contains a recombinant nucleic acid according to one of the Claims 14 to 23 at one of its sites inessential for its replication.

**26.** Recombinant vector according to Claim 25, characterized in that it is the plasmid PSHV-G1 deposited with the CNCM under No. I-778.

**27.** Recombinant vector according to Claim 25, characterized in that it contains at one of its sites inessential for its replication, elements necessary for promoting the expression of an amino acid sequence according to the Claims 1 to 13 in a cell host, and optionally a promoter recognized by the polymerases of the cell host, in particular an inducible promoter and/or an anchoring sequence.

**28.** Recombinant vector according to Claim 27, characterized in that it contains
- the elements allowing the expression of this nucleic acid according to the Claims 11 to 20 inserted in the vector by E. coli, and in particular
- the elements allowing the expression of all or part of the beta-galactosidase gene.

**29.** Recombinant vector according to Claim 27, characterized in that it contains the elements enabling the nucleic acid according to the Claims 14 to 23 to be inserted into yeast and for this nucleic acid to be expressed in yeast.

**30.** Cell host transformed by a recombinant vector according to any one of the Claims 25 to 29 and comprising the regulatory elements allowing the expression of the nucleic acid sequence coding for the polypeptide according to one of the Claims 1 to 13 in this host.

**31.** Cell host according to Claim 30, characterized in that it is E. coli transformed by the vector according to Claim 28.

**32.** Transformed cell host according to Claim 30, characterized in that it is a yeast transformed by the vector according to Claim 29.

**33.** Expression product of a nucleic acid expressed by a cell host transformed according to the Claims 30 to 32.

**34.** Nucleotide probe characterized in that it hybridizes with one of the nucleic acids according to the Claims 14 to 23 or with their complementary sequence, and, in particular, the probes selected from the following nucleotide sequences :
ACG TTG GAG GGA AAG GCC
GAC TGG GAC ACT CCG CTA
CCC TCC TGC ATC TGG ATG
AAG ACT GAC CTG GGG GAC
GAC AAC AAC ACA GAC GGG
TTC CTT CTA CTG GTA CTC
or their complementary nucleotide sequences.

**35.** Procedure for the preparation of a recombinant polypeptide according to any one of the Claims 1 to 13, characterized in that
- a cell host previously transformed by a suitable vector containing a nucleic acid according to one of the Claims 14 to 23 is cultured in a suitable culture medium and
- the polypeptide produced by the said transformed cell host is recovered from the said culture medium.

**36.** Procedure according to Claim 35 for the preparation of specific recombinant polypeptide characterized by the placing of E. coli according to Claim 31 in culture and by interruption of the culture at the end of the exponential phase of growth of the said E. coli, and recovery of the specific amino acid sequence from the culture medium free of the other extracellular proteins and enzymes capable of being secreted by E. coli.

37. Procedure according to Claim 36 for the preparation of a specific polypeptide characterized by the placing of the yeast according Claim 32 in culture, and interruption of the culture at the end of the exponential phase of growth of the said yeast and recovery of the polypeptide micro-encapsulated in the yeast.

38. Polypeptide according to any one of the Claims 1 or 3, characterized in that it is constituted by the amino acid sequence defined between the residues 111 and 302, contained in the amino acid sequence shown in Figure 3.

39. Nucleic acid, in particular a DNA fragment, coding for the polypeptide defined in Claim 38.

40. Monoclonal antibody prepared from an immunogenic recombinant polypeptide according to any one of the Claims 1 to 13, and characterized in that it is directed against any one of these polypeptides.

41. Monoclonal antibody according to Claim 40, characterized in that it specifically recognizes the polypeptide of Claim 38.

**Patentansprüche**
**Patentansprüche für folgende Vertragsstaaten : AT, BE, CH, DE, FR, GB, IT, LI, LU, NL, SE**

1. Rekombinantes Polypeptid, dadurch gekennzeichnet, daß in seinem Polypeptidgerüst eine fortlaufende Abfolge (I) von Aminosäuren mit der nachstehenden Sequenz vorhanden ist:

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-

128

```
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

und daß natürliche Verunreinigungen, die vom Virus der viralen hämorrhagischen Sepsis stammen, fehlen.

2. Polypeptid nach Anspruch 1, dadurch gekennzeichnet, daß es durch neutralisierende Antikörper erkannt wird, die zuvor gegen das Virus der SHV gebildet wurden.

3. Rekombinantes Polypeptid nach Anspruch 2, dadurch gekennzeichnet, daß es geeignet ist, in vivo neutralisierende Antikörper gegen das Virus der SHV zu induzieren.

4. Rekombinantes Polypeptid nach einem der Ansprüche 1 bis 3, dadurch gekennzeichnet, daß in seinem Peptidgerüst die nachstehende Abfolge (II) von Aminosäuren vorhanden ist:

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
```

```
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

und daß natürliche Verunreinigungen, die vom Virus der viralen hämorrhagischen Sepsis stammen, fehlen.

**5.** Polypeptid nach einem der Ansprüche 1 bis 3, dadurch gekennzeichnet, daß es die gesamte nachstehende Abfolge (III) von Aminosäuren oder einen Teil der Abfolge enthält:

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

**6.** Polypeptid nach einem der Ansprüche 1 bis 3, dadurch gekennzeichnet, daß es die gesamte Abfolge der nachstehenden Aminosäuren oder einen Teil der Abfolge enthält:

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

7. Polypeptid nach einem der Ansprüche 1 bis 3, dadurch gekennzeichnet, daß es aus der nachstehenden Abfolge von Aminosäuren I aufgebaut ist:

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
```

```
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

8. Polypeptid nach einem der Ansprüche 1 bis 3, dadurch gekennzeichnet, daß es aus der nachstehenden Abfolge (II) von Aminosäuren aufgebaut ist:

Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-


Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

9. Polypeptid nach einem der Ansprüche 1 bis 3, dadurch gekennzeichnet, daß es aus der nachstehenden Abfolge (III) von Aminosäuren aufgebaut ist:

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

10. Polypeptid nach einem der Ansprüche 1 bis 3, dadurch gekennzeichnet, daß es aus der nachstehenden Sequenz von Aminosäuren IV aufgebaut ist:

```
Met-Glu-Trp-Asn-Thr-Phe-Phe-Leu-Val-Ile-Leu-Ile-Ile-
Ile-Ile-Lys-Ser-Thr-Thr-Pro
```

11. Aminosäuresequenz nach einem der Ansprüche 1 bis 9, dadurch gekennzeichnet, daß sie aus einer Abfolge von Aminosäuren gemäß einem der Ansprüche 1 bis 9 und einem Protein oder einer Aminosäuresequenz, die heterolog in bezug auf die Abfolge sind und etwa 8 bis etwa 800 Aminosäuren enthalten, aufgebaut ist.

12. Aminosäuresequenz nach Anspruch 11, dadurch gekennzeichnet, daß es sich bei der fremden Aminosäuresequenz um $\beta$-Galactosidase handelt.

13. Polypeptid, dadurch gekennzeichnet, daß ein Peptidgerüst vorhanden ist, das die gesamte Abfolge der Aminosäuren, die durch die nachstehende Nucleinsäure (1) codiert wird, oder einen Teil der Abfolge umfaßt:

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
```

134

```
CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA ATG

GAA TGG AAC ACT TTT TTC TTG GTG ATC TTG ATC ATC ATC ATA

AAG AGC ACC ACA CCA CAG ATC ACT CAA CGA CCT CCG GTC GAA

AAC ATC TCG ACG TAC CAT GCA GAC TGG GAC ACT CCG CTA TAC

ACT CAT CCC TCC AAC TGC AGG GAC GAT TCC TTT GTC CCG ATT

CGA CCA GCT CAA CTC AGG TGT CCT CAT GAA TTT GAA GAC ATA

AAC AAG GGA CTG GTT TCC GTC CCA ACC AGG ATC ATC CAT CTC

CCG CTA TCG GTC ACC AGC GTC TCC GCA GTA GCG AGT GGC CAC

TAC CTG CAC AGA GTG ACT TAT CGA GTC ACC TGT TCG ACC AGC

TTC TTT GGA GGG CAA ACC ATC GAA AAG ACC ATC TTG GAG GCG

AAA CTG TCT CGT CAG GAG GCC ACA GAC GAG GCA AGC AAG GAT

CAC GAG TAC CCG TTC TTC CCT GAA CCC TCC TGC ATC TGG ATG

AAA AAC AAT GTC CAT AAG GAC ATA ACT CAC TAT TAC AAG ACC

CCA AAA ACA GTA TCG GTG GAT CTC TAC AGC AGG AAA TTT CTC

AAC CCT GAT TTC ATA GAG GGG GTT TGC ACA ACC TCG CCC TGT

CAA ACT CAT TGG CAG GGA GTC TAT TGG GTC GGT GCC ACA CCT

AAA GCC CAT TGC CCC ACG TCG GAA ACA CTA GAA GGA CAC CTG

TTC ACC AGG ACC CAT GAT CAC AGG GTG GTC AAG GCA ATT GTG

GCA GGC CAT CAT CCC TGG GGA CTC ACA ATG GCA TGC ACA GTG

ACA TTC TGC GGG ACA GAA TGG ATC AAG ACT GAC CTG GGG GAC

CTG ATC CAG GTG ACA GGA CCG GGG GGC ACG AGG AAA CTG ACT

CCA AAC AAG TGT GTC AAT ACC GAT ATC CAG ATG AGG GGG GCA

ACA GAC GAC TTT TCT TAT CTC AAC CAT CTC ATC ACC AAC ATG

GCT CAA AGA ACC GAG TGC CTA GAT GCC CAT AGT GAT ATC ACC

GCT TCT GGG AAA GTA TCC TCA TTT CTC CTC TCA AAG TTT CGT

CCC AGC CAC CCT GGA CCT GGC AAG GCA CAC TAT CTT CTC GAC

GGT CAA ATC ATG CGA GGT GAC TGT GAC TAT GAG GCA GTA GTC

AGC ATC AAC TAC AAT CGC GCT CAA TAC AAG ACG ATG AAC AAC

ACA TGG AAA TCA TGG AAA CGG GTA GAC AAC AAC ACA GAC GGG
```

```
TAC GAT GGG ATG ATA TTT GGG GAC AAA TTG ATC ATC CCG GAC

ATC GAA AAG TAT CAG AGT GTC TAT GAC AGT GGA ATG CTC GTT

CAA AGA AAC CTT GTG GAA GTC CCT CAT CTG AGC ATT GTG TTT

GTC TCC AAC ACA TCT GAT CTT TCC ACT AAT CAC ATC CAC ACC

AAC CTA ATC CCT TCG GAT
```

**14.** Nucleinsäure, dadurch gekennzeichnet, daß sie eine Abfolge von Nucleotiden umfaßt, die für die Polypeptide nach einem der Ansprüche 1 bis 13 codiert.

**15.** Nucleinsäure nach Anspruch 14, dadurch gekennzeichnet, daß sie die gesamte nachstehende Abfolge von Nucleotiden (2) oder einen Teil der Abfolge umfaßt:

```
                                ATG GAA TGG AAC ACT TTT TTC TTG GTG

ATC TTG ATC ATC ATC ATA AAG AGC ACC ACA CCA CAG ATC ACT

CAA CGA CCT CCG GTC GAA AAC ATC TCG ACG TAC CAT GCA GAC

TGG GAC ACT CCG CTA TAC ACT CAT CCC TCC AAC TGC AGG GAC

GAT TCC TTT GTC CCG ATT CGA CCA GCT CAA CTC AGG TGT CCT

CAT GAA TTT GAA GAC ATA AAC AAG GGA CTG GTT TCC GTC CCA

ACC AGG ATC ATC CAT CTC CCG CTA TCG GTC ACC AGC GTC TCC

GCA GTA GCG AGT GGC CAC TAC CTG CAC AGA GTG ACT TAT CGA

GTC ACC TGT TCG ACC AGC TTC TTT GGA GGG CAA ACC ATC GAA

AAG ACC ATC TTG GAG GCG AAA CTG TCT CGT CAG GAG GCC ACA

GAC GAG GCA AGC AAG GAT CAC GAG TAC CCG TTC TTC CCT GAA

CCC TCC TGC ATC TGG ATG AAA AAC AAT GTC CAT AAG GAC ATA

ACT CAC TAT TAC AAG ACC CCA AAA ACA GTA TCG GTG GAT CTC

TAC AGC AGG AAA TTT CTC AAC CCT GAT TTC ATA GAG GGG GTT

TGC ACA ACC TCG CCC TGT CAA ACT CAT TGG CAG GGA GTC TAT

TGG GTC GGT GCC ACA CCT AAA GCC CAT TGC CCC ACG TCG GAA

ACA CTA GAA GGA CAC CTG TTC ACC AGG ACC CAT GAT CAC AGG

GTG GTC AAG GCA ATT GTG GCA GGC CAT CAT CCC TGG GGA CTC

ACA ATG GCA TGC ACA GTG ACA TTC TGC GGG ACA GAA TGG ATC

AAG ACT GAC CTG GGG GAC CTG ATC CAG GTG ACA GGA CCG GGG

GGC ACG AGG AAA CTG ACT CCA AAC AAG TGT GTC AAT ACC GAT

ATC CAG ATG AGG GGG GCA ACA GAC GAC TTT TCT TAT CTC AAC

CAT CTC ATC ACC AAC ATG GCT CAA AGA ACC GAG TGC CTA GAT

GCC CAT AGT GAT ATC ACC GCT TCT GGG AAA GTA TCC TCA TTT

CTC CTC TCA AAG TTT CGT CCC AGC CAC CCT GGA CCT GGC AAG
```

```
GCA CAC TAT CTT CTC GAC GGT CAA ATC ATG CGA GGT GAC TGT
GAC TAT GAG GCA GTA GTC AGC ATC AAC TAC AAT CGC GCT CAA
TAC AAG ACG ATG AAC AAC ACA TGG AAA TCA TGG AAA CGG GTA
GAC AAC AAC ACA GAC GGG TAC GAT GGG ATG ATA TTT GGG GAC
AAA TTG ATC ATC CCG GAC ATC GAA AAG TAT CAG AGT GTC TAT
GAC AGT GGA ATG CTC GTT CAA AGA AAC CTT GTG GAA GTC CCT
CAT CTG AGC ATT GTG TTT GTC TCC AAC ACA TCT GAT CTT TCC
ACT AAT CAC ATC CAC ACC AAC CTA ATC CCT TCG GAT
```

16. Nucleinsäure, dadurch gekennzeichnet, daß sie die gesamte nachstehende Abfolge von Nucleotiden (3) oder einen Teil der Abfolge umfaßt:

```
CAG ATC ACT CAA CGA CCT CCG GTC GAA AAC ATC TCG ACG TAC
CAT GCA GAC TGG GAC ACT CCG CTA TAC ACT CAT CCC TCC AAC
TGC AGG GAC GAT TCC TTT GTC CCG ATT CGA CCA GCT CAA CTC
AGG TGT CCT CAT GAA TTT GAA GAC ATA AAC AAG GGA CTG GTT
TCC GTC CCA ACC AGG ATC ATC CAT CTC CCG CTA TCG GTC ACC
AGC GTC TCC GCA GTA GCG AGT GGC CAC TAC CTG CAC AGA GTG
ACT TAT CGA GTC ACC TGT TCG ACC AGC TTC TTT GGA GGG CAA
ACC ATC GAA AAG ACC ATC TTG GAG GCG AAA CTG TCT CGT CAG
GAG GCC ACA GAC GAG GCA AGC AAG GAT CAC CAG TAC CCG TTC
TTC CCT GAA CCC TCC TGC ATC TGG ATG AAA AAC AAT GTC CAT
AAG GAC ATA ACT CAC TAT TAC AAG ACC CCA AAA ACA GTA TCG
GTG GAT CTC TAC AGC AGG AAA TTT CTC AAC CCT GAT TTC ATA
GAG GGG GTT TGC ACA ACC TCG CCC TGT CAA ACT CAT TGG CAG
GGA GTC TAT TGG GTC GGT GCC ACA CCT AAA GCC CAT TGC CCC
ACG TCG GAA ACA CTA GAA GGA CAC CTG TTC ACC AGG ACC CAT
GAT CAC AGG GTG GTC AAG GCA ATT GTG GCA GGC CAT CAT CCC
TGG GGA CTC ACA ATG GCA TGC ACA GTG ACA TTC TGC GGG ACA
GAA TGG ATC AAG ACT GAC CTG GGG GAC CTG ATC CAG GTG ACA
GGA CCG GGG GGC ACG AGG AAA CTG ACT CCA AAC AAG TGT GTC
AAT ACC GAT ATC CAG ATG AGG GGG GCA ACA GAC GAC TTT TCT
TAT CTC AAC CAT CTC ATC ACC AAC ATG GCT CAA AGA ACC GAG
TGC CTA GAT GCC CAT AGT GAT ATC ACC GCT TCT GGG AAA GTA
TCC TCA TTT CTC CTC TCA AAG TTT CGT CCC AGC CAC CCT GGA
CCT GGC AAG GCA CAC TAT CTT CTC GAC GGT CAA ATC ATG CGA
GGT GAC TGT GAC TAT GAG GCA GTA GTC AGC ATC AAC TAC AAT
```

```
CGC GCT CAA TAC AAG ACG ATG AAC AAC ACA TGG AAA TCA TGG

AAA CGG GTA GAC AAC AAC ACA GAC GGG TAC GAT GGG ATG ATA

TTT GGG GAC AAA TTG ATC ATC CCG GAC ATC GAA AAG TAT CAG

AGT GTC TAT GAC AGT GGA ATG CTC GTT CAA AGA AAC CTT GTG

GAA GTC CCT CAT CTG AGC ATT GTG TTT GTC TCC AAC ACA TCT

GAT CTT TCC ACT AAT CAC ATC CAC ACC AAC CTA ATC CCT TCG

GAT
```

17. Nucleinsäure, dadurch gekennzeichnet, daß sie die gesamte nachstehende Abfolge von Nucleotiden (4) oder einen Teil der Abfolge umfaßt:

```
ATG CGA GGT GAC TGT GAC TAT GAG GCA GTA GTC AGC ATC AAC

TAC AAT CGC GCT CAA TAC AAG ACG ATG AAC AAC ACA TGG AAA

TCA TGG AAA CGG GTA GAC AAC AAC ACA GAC GGG TAC GAT GGG

ATG ATA TTT GGG GAC AAA TTG ATC ATC CCG GAC ATC GAA AAG

TAT CAG AGT GTC TAT GAC AGT GGA ATG CTC GTT CAA AGA AAC

CTT GTG GAA GTC CCT CAT
```

18. Nucleinsäure, dadurch gekennzeichnet, daß sie die gesamte nachstehende Abfolge von Nucleotiden (5) oder einen Teil der Abfolge umfaßt:

```
TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA
```

19. Nucleinsäure, dadurch gekennzeichnet, daß sie die gesamte nachstehende Abfolge von Nucleotiden (6) oder einen Teil der Abfolge umfaßt:

```
        TGG TCA TTC AAC TGG AGT CTT TGG CCA TCA TTA TCT
    GGG ATG GGG GTT GTG GGA GGG GCC TTC CTT CTA CTG GTA CTC
    TGC TGT TGC TGC AAG GCG TCC CCT CCC ATT CCA AAT TAC GGG


    ATT CCG ATG CAG CAG TTC TCC AGA AGT CAG ACG GTC TGA GCA
    CAC CTG TCC GAA TGA CCA CAA TTC CTC TCT TAG GTA GAT AGA
    AAA AAA AAA AAA AAA AAA AAA AAA A
```

**20.** Nucleinsäure nach Anspruch 15, dadurch gekennzeichnet, daß ihr eine Nucleotidsequenz vorangeht, die Regulationselemente für die Expression der Nucleinsäure umfaßt.

**21.** Nucleinsäure nach einem der Ansprüche 15 oder 20, dadurch gekennzeichnet, daß sie Terminationselemente für die Transkription der Nucleinsäure umfaßt.

**22.** Nucleinsäure, dadurch gekennzeichnet, daß sie die gesamte nachstehende Abfolge von Nucleotiden (1) oder einen Teil der Abfolge umfaßt:

```
TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT CCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA ATG

GAA TGG AAC ACT TTT TTC TTG GTG ATC TTG ATC ATC ATC ATA

AAG AGC ACC ACA CCA CAG ATC ACT CAA CGA CCT CCG GTC GAA

AAC ATC TCG ACG TAC CAT GCA GAC TGG GAC ACT CCG CTA TAC

ACT CAT CCC TCC AAC TGC AGG GAC GAT TCC TTT GTC CCG ATT

CGA CCA GCT CAA CTC AGG TGT CCT CAT GAA TTT GAA GAC ATA

AAC AAG GGA CTG GTT TCC GTC CCA ACC AGG ATC ATC CAT CTC

CCG CTA TCG GTC ACC AGC GTC TCC GCA GTA GCG AGT GGC CAC

TAC CTG CAC AGA GTG ACT TAT CGA GTC ACC TGT TCG ACC AGC

TTC TTT GGA GGG CAA ACC ATC GAA AAG ACC ATC TTG GAG GCG

AAA CTG TCT CGT CAG GAG GCC ACA GAC GAG GCA AGC AAG GAT

CAC CAG TAC CCG TTC TTC CCT GAA CCC TCC TGC ATC TGG ATG

AAA AAC AAT GTC CAT AAG GAC ATA ACT CAC TAT TAC AAG ACC
```

140

```
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**23.** Nucleinsäure nach Anspruch 22, dadurch gekennzeichnet, daß sie aus der nachstehenden Abfolge (1) von Nucleotiden aufgebaut ist:

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT CCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
```

142

```
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**24.** Rekombinante Nucleinsäure, dadurch gekennzeichnet, daß sie die Nucleinsäure nach einem der Ansprüche 14 bis 23 inseriert in eine gegenüber dem vorstehenden Fragment heterologe Nucleinsäure enthält.

**25.** Rekombinanter Vektor, insbesondere für die Clonierung und/oder die Expression, insbesondere vom Typ eines Plasmids, eines Cosmids oder eines Phagen, dadurch gekennzeichnet, daß er eine rekombinante Nucleinsäure nach einem der Ansprüche 14 bis 23 an einer der für seine Replikation nicht essentiellen Stellen enthält.

26. Rekombinanter Vektor nach Anspruch 25, dadurch gekennzeichnet, daß es sich um das Plasmid PSHV-G1 handelt, das bei der CNCM unter der Nummer I 778 hinterlegt wurde.

27. Rekombinanter Vektor nach Anspruch 25, dadurch gekennzeichnet, daß er in einer der für seine Replikation nicht essentiellen Stellen die zur Förderung der Expression einer Aminosäuresequenz nach einem der Ansprüche 1 bis 13 in einem zellulären Wirt erforderlichen Elemente und gegebenenfalls einen Promotor, der von den Polymerasen des zellulären Wirts erkannt wird, und insbesondere einen induzierbaren Promotor sowie gegebenenfalls eine Signalsequenz und/oder eine Ankersequenz enthält.

28. Rekombinanter Vektor nach Anspruch 27, dadurch gekennzeichnet, daß er
   - die Elemente, die die Expression der Nucleinsäuresequenz nach einem der Ansprüche 11 bis 20, die in den Vektor für E. coli inseriert ist, erlauben und insbesondere
   - die Elemente, die die Expression des gesamten Gens für $\beta$-Galactosidaseoder eines Teils davon erlauben, enthält.

29. Rekombinanter Vektor nach Anspruch 27, dadurch gekennzeichnet, daß er die Elemente, die das Einführen der Nucleinsäure nach einem der Ansprüche 14 bis 23 in Hefe und die Expression der Nucleinsäure in der Hefe erlauben, enthält.

30. Durch einen rekombinanten Vektor nach einem der Ansprüche 25 bis 29 transformierter und die Regulationselemente, die die Expression der Nucleinsäuresequenz, die für das Polypeptid nach einem der Ansprüche 1 bis 13 codiert, in dem Wirt erlauben, umfassender zellulärer Wirt.

31. Zellulärer Wirt nach Anspruch 30, dadurch gekennzeichnet, daß es sich um den Wirt E. coli handelt, der durch den Vektor nach Anspruch 28 transformiert ist.

32. Zellulärer Wirt nach Anspruch 30, dadurch gekennzeichnet, daß es sich um Hefe handelt, die mit dem Vektor nach Anspruch 29 transformiert ist.

33. Expressionsprodukt einer durch den transformierten zellulären Wirt nach einem der Ansprüche 30 bis 32 exprimierten Nucleinsäure.

34. Impfwirkstoff, dadurch gekennzeichnet, daß er das Expressionsprodukt nach Anspruch 33, gegebenenfalls gekoppelt an ein natürliches Protein oder ein synthetisches Polypeptid mit einem ausreichenden Molekulargewicht, so daß das Konjugat imstande ist, in vivo die Bildung neutralisierender Antikörper gegen SHV zu induzieren, umfaßt.

35. Impfwirkstoff nach Anspruch 34, auf oralem Weg verabreichbar, dadurch gekennzeichnet, daß er 10 ng bis 100 $\mu$g des Wirkstoffs pro g Impf-Futter enthält.

36. Impfwirkstoff nach Anspruch 34, auf intraperitonealem Weg verabreichbar, dadurch gekennzeichnet, daß er 1 bis 1000 ng/g Fisch enthält.

37. Impfwirkstoff nach Anspruch 34, verabreichbar durch Behandlung durch Bäder in einem Medium mit einem Gehalt an 10 ng/ml bis 100 $\mu$g/ml.

38. Nucleinsäuresonde, dadurch gekennzeichnet, daß sie mit den Nucleinsäuren nach einem der Ansprüche 14 bis 23 oder mit einer dazu komplementären Sequenz hybridisiert, und insbesondere Sonden, die unter den nachstehenden Nucleotidsequenzen oder den dazu komplementären Nucleotidsequenzen ausgewählt sind:
   ACG TTG GAG GGA AAG GCC
   GAC TGG GAC ACT CCG CTA
   CCC TCC TGC ATC TGG ATG
   AAG ACT GAC CTG GGG GAC
   GAC AAC AAC ACA GAC GGG

TTC CTT CTA CTG GTA CTC

39. Verfahren zur Herstellung eines rekombinanten Polypeptids nach einem der Ansprüche 1 bis 13, dadurch gekennzeichnet, daß

- man in einem geeigneten Kulturmedium einen zuvor mit einem geeigneten Vektor, der eine Nucleinsäure gemäß einem der Ansprüche 14 bis 23 enthält, transformierten Wirt züchtet und
- ausgehend von dem vorstehend genannten Kulturmedium das Polypeptidprodukt durch den transformierten zellulären Wirt gewinnt.

40. Verfahren nach Anspruch 39 zur Herstellung eines bestimmten rekombinanten Polypeptids, dadurch gekennzeichnet, daß man E. coli nach Anspruch 31 züchtet und die Kultur am Ende der exponentiellen Wachstumsphase der E. coli arretiert und die bestimmte Aminosäuresequenz ausgehend vom Kulturmedium frei von anderen extrazellulären Proteinen und Enzymen, die von E. coli angesondert werden können, gewinnt.

41. Verfahren nach Anspruch 40 zur Herstellung eines bestimmten Polypeptids, dadurch gekennzeichnet, daß man die Hefe nach Anspruch 32 züchtet und die Kultur am Ende der exponentiellen Wachstumsphase der Hefe arretiert und das in der Hefe in Mikroeinschlüssen vorliegende Polypeptid gewinnt.

42. Polypeptid nach einem der Ansprüche 1 bis 3, dadurch gekennzeichnet, daß es aus der zwischen den Resten 111 bis 302 definierten Aminosäuresequenz, die in der in Fig. 3 dargestellten Aminosäuresequenz enthalten ist, aufgebaut ist.

43. Nucleinsäure, insbesondere Fragment von DNA, codierend für ein Polypeptid, wie es in Anspruch 42 definiert ist.

44. Monoclonaler Antikörper, hergestellt ausgehend von einem immunogenen rekombinanten Polypeptid nach einem der Ansprüche 1 bis 13 und dadurch gekennzeichnet, daß er gegen eines dieser Polypeptide gerichtet ist.

45. Monoclonaler Antikörper nach Anspruch 44, dadurch gekennzeichnet, daß er spezifisch das Polypeptid von Anspruch 42 erkennt.

**Patentansprüche für folgenden Vertragsstaat : ES**

1. Verfahren zur Herstellung eines rekombinanten Polypeptids, das in seinem Polypeptidgerüst eine fortlaufende Abfolge (I) von Aminosäuren mit der nachstehenden Sequenz enthält:

Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-

```
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

dadurch gekennzeichnet, daß man, vorzugsweise ausgehend von C-terminalen Aminosäure, nacheinander paarweise die Aminoacylverbindungen in der erforderlichen Reihenfolge oder Aminoacylverbindungen und zuvor gebildete und bereits mehrere Reste Aminoacylreste in der richtigen Reihenfolge enthaltende Fragmente oder auch mehrere ebenfalls zuvor hergestellte Fragmente, wobei es sich versteht, daß Sorge getragen ist, daß alle reaktiven funktionellen Gruppen, die die Aminoacylverbindungen oder die Fragmente tragen, mit Ausnahme der Aminfunktion der einen und der Carboxylfunktion der anderen oder umgekehrt, die normalerweise die Bildung der Peptidverknüpfungen stören, insbesondere nach Aktivierung der Carboxylfunktion, vorher geschützt worden sind, gemäß bekannten Verfahren der Peptidsynthese kondensiert und so allmählich bis zur N-terminalen Aminosäure fortfährt.

2. Verfahren nach Anspruch 1 zur Herstellung eines Polypeptids, das durch neutralisierende Antikörper erkannt wird, die zuvor gegen das Virus der SHV gebildet wurden.

3. Verfahren nach Anspruch 1 zur Herstellung eines Polypeptids, das geeignet ist, in vivo neutralisierende Antikörper gegen das Virus der SHV zu induzieren.

4. Verfahren nach Anspruch 1 zur Herstellung eines Polypeptids, das in seinem Peptidgerüst die nachstehende Abfolge (II) von Aminosäuren enthält:

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
```

Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

5. Verfahren nach Anspruch 1 zur Herstellung eines Polypeptids, das die gesamte nachstehende Abfolge (III) von Aminosäuren oder einen Teil der Abfolge enthält:

Met Arg Gly Asp Cys Asp Tyr Glu Ala Val Val Ser Ile
Asn Tyr Asn Arg Ala Gln Tyr Lys Thr Met Asn Asn Thr
Trp Lys Ser Trp Lys Arg Val Asp Asn Asn Thr Asp Gly
Tyr Asp Gly Met Ile Phe Gly Asp Lys Leu Ile Ile Pro
Asp Ile Glu Lys Tyr Gln Ser Val Tyr Asp Ser Gly Met
Leu Val Gln Arg Asn Leu Val Glu Val Pro His

148

6. Verfahren nach Anspruch 1 zur Herstellung eines Polypeptids, das die gesamte Abfolge der nachstehenden Aminosäuren oder einen Teil der Abfolge enthält:

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

7. Verfahren nach Anspruch 1 zur Herstellung eines Polypeptids, das die gesamte nachstehende Abfolge der Aminosäuren I oder einen Teil der Abfolge enthält:

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
```

```
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

**8.** Verfahren nach Anspruch 1 zur Herstellung eines Polypeptids, das aus der nachstehenden Abfolge (II) von Aminosäuren aufgebaut ist:

Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-

Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

9. Verfahren nach Anspruch 1 zur Herstellung eines Polypeptids, das aus der nachstehenden Abfolge (III) von Aminosäuren aufgebaut ist:

```
Met Arg Gly Asp Cys Asp Tyr Glu Ala Val Val Ser Ile
Asn Tyr Asn Arg Ala Gln Tyr Lys Thr Met Asn Asn Thr
Trp Lys Ser Trp Lys Arg Val Asp Asn Asn Thr Asp Gly
Tyr Asp Gly Met Ile Phe Gly Asp Lys Leu Ile Ile Pro
Asp Ile Glu Lys Tyr Gln Ser Val Tyr Asp Ser Gly Met
Leu Val Gln Arg Asn Leu Val Glu Val Pro His
```

10. Verfahren nach Anspruch 1 zur Herstellung eines Polypeptids, das aus der nachstehenden Sequenz von Aminosäuren IV aufgebaut ist:

11. Verfahren nach Anspruch 1 zur Herstellung einer Aminosäuresequenz, die aus einer Abfolge von Aminosäuren, wie sie in einem der Ansprüche 1 bis 9 definiert ist, und einem Protein oder einer Aminosäuresequenz, die heterolog in bezug auf die Abfolge sind und etwa 8 bis etwa 800 Aminosäuren enthalten, aufgebaut ist.

12. Verfahren nach Anspruch 1 zur Herstellung einer Aminosäuresequenz, wie sie in Anspruch 11 definiert ist, wobei es sich bei der fremden Aminosäuresequenz um $\beta$-Galactosidase handelt.

13. Verfahren zur Herstellung einer Nucleinsäure, die die gesamte nachstehende Abfolge von Nucleotiden (1) oder einen Teil der Abfolge umfaßt:

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
```

152

```
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG GTC GAA
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GGC CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
```

```
AAC TAC AAT CGC GCT CAA TAC AAG ACG ATG AAC AAC ACA

TGG AAA TCA TGG AAA CGG GTA GAC AAC AAC ACA GAC GGG

TAC GAT GGG ATG ATA TTT GGG GAC AAA TTG ATC ATC CCG

GAC ATC GAA AAG TAT CAG AGT GTC TAT GAC AGT GGA ATG

CTC GTT CAA AGA AAC CTT GTG GAA GTC CCT CAT CTG AGC

ATT GTG TTT GTC TCC AAC ACA TCT GAT CTT TCC ACT AAT

CAC ATC CAC ACC AAC CTA ATC CCT TCG GAT
```

wobei das Verfahren die folgenden Stufen umfaßt:

- Zentrifugation des Kulturüberstands von Zellen, die durch eine Isolierung von SHV Typ I infiziert sind, und Gewinnung des Bodensatzes der Zentrifugation;
- gegebenenfalls Zentrifugation des gewonnenen Bodensatzes über einen Saccharose-Gradienten gemäß dem in EP-A-0 138 667 beschriebenen Verfahren;
- Gewinnung des Anteils des Gradienten, der das Virus enthält, und erneute Sedimentation des Virus durch Zentrifugation;
- Resuspendieren des Bodensatzes des Virus und Extraktion der genomischen RNA gemäß der von Chomzynski et al. (Anal. Biochem., Bd. 162 (1987), S. 156) beschriebenen Technik;
- Synthese eines cDNA-Strangs im Kontakt mit der genomischen RNA des Virus durch eine reverse Transkriptase in Gegenwart von oligomeren Initiatoren, deren Sequenzen zufallsbedingt sind, gemäß der Technik, die in DNA, Bd. 4 (1985), S. 429-438, beschrieben wird;
- gegebenenfalls Reinigung der RNA/cDNA-Hybride durch Extraktion mit einem Phenol/Chloroform-Gemisch und Fällung mit Ethanol;
- Herstellung eines zweiten cDNA-Strangs in Gegenwart von DNA-Polymerase I, RNAse H und 4 Desoxynucleotiden gemäß dem Verfahren, das in Gene, Bd. 25 (1983), S. 263-269, beschrieben wird, bei Bedarf anschließend Extraktion der im Reaktionsmedium vorhandenen Proteine;
- Clonierung der auf diese Weise erhaltenen Nucleinsäuren in einem geeigneten Plasmidvektor und Gewinnung der mit Hilfe einer geeigneten Sonde untersuchten Nucleinsäure.

**14.** Verfahren zur Herstellung einer Nucleinsäure, die die gesamte nachstehend Abfolge von Nucleotiden (1) oder einen Teil der Abfolge umfaßt:

EP 0 377 349 B1

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG GTC GAA
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GGG CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
```

155

```
ATC CAG ATG AGG GGG GCA ACA GAC GAC TTT TCT TAT CTC

AAC CAT CTC ATC ACC AAC ATG GCT CAA AGA ACC GAG TGC

CTA GAT GCC CAT AGT GAT ATC ACC GCT TCT GGG AAA GTA

TCC TCA TTT CTC CTC TCA AAG TTT CGT CCC AGC CAC CCT

GGA CCT GGC AAG GCA CAC TAT CTT CTC GAC GGT CAA ATC

ATG CGA GGT GAC TGT GAC TAT GAG GCA GTA GTC AGC ATC

AAC TAC AAT CGC GCT CAA TAC AAG ACG ATG. AAC AAC ACA

TGG AAA TCA TGG AAA CGG GTA GAC AAC AAC ACA GAC GGG

TAC GAT GGG ATG ATA TTT GGG GAC AAA TTG ATC ATC CCG

GAC ATC GAA AAG TAT CAG AGT GTC TAT GAC AGT GGA ATG

CTC GTT CAA AGA AAC CTT GTG GAA GTC CCT CAT CTG AGC

ATT GTG TTT GTC TCC AAC ACA TCT GAT CTT TCC ACT AAT

CAC ATC CAC ACC AAC CTA ATC CCT TCG GAT
```

wobei das Verfahren folgende Stufen umfaßt:
- Herstellung der zellulären und der viralen RNA ausgehend von einer Kultur seit 16 bis 20 Stunden durch das Virus vom SHV-Typ 1 mit einer Vielfachheit von 10 ufp pro Zelle infizierter Zellen gemäß der von Chomzynski et al. (Anal. Biochem., Bd. 162 (1987), S. 156) beschriebenen Technik;
- Gewinnung und Reinigung der zellulären und viralen mRNA, indem die gesamte zelluläre und virale RNA einer chromatographischen Trennung mit immobilisiertem Oligo(dT) unterworfen wird;
- Synthese eines cDNA-Strangs ausgehend von der gereinigten mRNA gemäß der Technik, die in Gene, Bd. 25 (1983), S. 263, beschrieben wird;
- Clonierung der auf diese Weise erhaltenen Nucleinsäuren in einen geeigneten Plasmidvektor und Gewinnung der mit Hilfe einer geeigneten Hybridisierungssonde untersuchten Nucleinsäuresequenz.

**15.** Verfahren zur Herstellung einer Nucleinsäure, die die gesamte nachstehende Abfolge von Nucleotiden (1) oder einen Teil der Abfolge umfaßt:

```
TCT TTG GAG AAA CCA GTG AGA AGA TTG ACT TCG GGA CTC

GCA GTA AGA CCC TGA TAA CAA GCT TCA AGA TAG CTG AAG

CAA AGG CCA TCT ACC TGG ATT CAA GTC CGG TGA GAT CTC

GCA TAG AGG CCA AGA AGT ACA CCA CTC CCA TTA GAC ATG

GGA GTG TGA CTT ACT ATG GTC CGT TCA TAT TTG CAG ATG

ACC ACG TTG GAG GGA AAG GCC ACC GCG AGA AGC TAG GGG

CAC TAT GTG GAT TCC TCC AGT CTG GAC CTT ACG GGC AGG
```

```
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA ATG GAA TGG
AAC ACT TTT TTC TTG GTG ATC TTG ATC ATC ATC ATA AAG
AGC ACC ACA CCA CAG ATC ACT CAA CGA CCT CCG TCG GAA
AAC ATC TCG ACG TAC CAT GCA GAC TGG GAC ACT CCG CTA
TAC ACT CAT CCC TCC AAC TGC AGG GAC GAT TCC TTT GTC
CCG ATT CGA CCA GCT CAA CTC AGG TGT CCT CAT GAA TTT
GAA GAC ATA AAC AAG GGA CTG GTT TCC GTC CCA ACC AGG
ATC ATC CAT CTC CCG CTA TCG GTC ACC AGC GTC TCC GCA
GTA GCG AGT GGC CAC TAC CTG CAC AGA GTG ACT TAT CGA
GTC ACC TGT TCG ACC AGC TTC TTT GGA GGG CAA ACC ATC
GAA AAG ACC ATC TTG GAG GCG AAA CTG TCT CGT CAG GAG
GCC ACA GAC GAG GCA AGC AAG GAT CAC CAG TAC CCG TTC
TTC CCT GAA CCC TCC TGC ATC TGG ATG AAA AAC AAT GTC
CAT AAG GAC ATA ACT CAC TAT TAC AAG ACC CCA AAA ACA
GTA TCG GTG GAT CTC TAC AGC AGG AAA TTT CTC AAC CCT
GAT TTC ATA GAG GGG GTT TGC ACA ACC TCG CCC TGT CAA
ACT CAT TGG CAG GGA GTC TAT TGG GTC GGT GCC ACA CCT
AAA GCC CAT TGC CCC ACG TCG GAA ACA CTA GAA GGA CAC
CTG TTC ACC AGG ACC CAT GAT CAC AGG GTG GTC AAG GCA
ATT GTG GCA GGC CAT CAT CCC TGG GGA CTC ACA ATG GCA
TGC ACA GTG ACA TTC TGC GGG ACA GAA TGG ATC AAG ACT
GAC CTG GGG GAC CTG ATC CAG GTG ACA GGA CCG GGG GGC
ACG AGG AAA CTG ACT CCA AAC AAG TGT GTC AAT ACC GAT
ATC CAG ATG AGG GGG GCA ACA GAC GAC TTT TCT TAT CTC
AAC CAT CTC ATC ACC AAC ATG GCT CAA AGA ACC GAG TGC
CTA GAT GCC CAT AGT GAT ATC ACC GCT TCT GGG AAA GTA
TCC TCA TTT CTC CTC TCA AAG TTT CGT CCC AGC CAC CCT
GGA CCT GGC AAG GCA CAC TAT CTT CTC GAC GGT CAA ATC
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
```

```
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT CTG AGC
ATT GTG TTT GTC TCC AAC ACA TCT GAT CTT TCC ACT AAT
CAC ATC CAC ACC AAC CTA ATC CCT TCG GAT
```

wobei das Verfahren,
wenn die herzustellende Nucleinsäure maximal 200 Nucleotide umfaßt, folgende Stufen umfaßt:
- die Synthese von DNA unter Anwendung des automatisierten Verfahrens mit $\beta$-Cyanoethylphosphoramidit, das in Bioorganic Chemistry, Bd. 4 (1986), S. 274-325, beschrieben wird;
- die Clonierung der auf diese Weise erhaltenen DNA in einen geeigneten Plasmidvektor und die Gewinnung der DNA durch Hybridisierung mit einer geeigneten Sonde;
     und wenn die herzustellende Nucleinsäure mehr als 200 Nucleotide umfaßt, die folgenden Stufen umfaßt:
- Verknüpfung chemisch synthetisierter Oligonucleotide, an deren Enden unterschiedliche Restriktionsstellen vorgesehen sind, wobei die Sequenzen mit der Abfolge der Aminosäuren des natürlichen Polypeptids übereinstimmen, gemäß den Prinzipien, die in Proc. Natl. Acad. Sci. USA, Bd. 80 (1983), S. 7461-7465, beschrieben werden;
- Clonierung der auf diese Weise erhaltenen DNA in einen geeigneten Plasmidvektor und Gewinnung der durch Hybridisierung mit einer geeigneten Sonde untersuchten Nucleinsäure.

16. Verfahren nach einem der Ansprüche 13, 14 oder 15 zur Herstellung einer Nucleinsäure, die die gesamte nachstehende Abfolge von Nucleotiden (2) oder einen Teil der Abfolge umfaßt:

```
                        ATG GAA TGG AAC ACT TTT TTC TTG
GTG ATC TTG ATC ATC ATC ATA AAG AGC ACC ACA CCA CAG
ATC ACT CAA CGA CCT CCG GTC GAA AAC ATC TCG ACG TAC
CAT GCA GAC TGG GAC ACT CCG CTA TAC ACT CAT CCC TCC
AAC TGC AGG GAC GAT TCC TTT GTC CCG ATT CGA CCA GCT
CAA CTC AGG TGT CCT CAT GAA TTT GAA GAC ATA AAC AAG
GGA CTG GTT TCC GTC CCA ACC AGG ATC ATC CAT CTC CCG
CTA TCG GTC ACC AGC GTC TCC GCA GTA GCG AGT GGC CAC
TAC CTG CAC AGA GTG ACT TAT CGA GTC ACC TGT TCG ACC
```

```
AGC TTC TTT GGA GGG CAA ACC ATC GAA AAG ACC ATC TTG

GAG GCG AAA CTG TCT CGT CAG GAG GCC ACA GAC GAG GCA

AGC AAG GAT CAC GAG TAC CCG TTC TTC CCT GAA CCC TCC

TGC ATC TGG ATG AAA AAC AAT GTC CAT AAG GAC ATA ACT

CAC TAT TAC AAG ACC CCA AAA ACA GTA TCG GTG GAT CTC

TAC AGC AGG AAA TTT CTC AAC CCT GAT TTC ATA GAG GGG

GTT TGC ACA ACC TCG CCC TGT CAA ACT CAT TGG CAG GGA

GTC TAT TGG GTC GGT GCC ACA CCT AAA GCC CAT TGC CCC

ACG TCG GAA ACA CTA GAA GGA CAC CTG TTC ACC AGG ACC

CAT GAT CAC AGG GTG GTC AAG GCA ATT GTG GCA GGC CAT

CAT CCC TGG GGA CTC ACA ATG GCA TGC ACA GTG ACA TTC

TGC GGG ACA GAA TGG ATC AAG ACT GAC CTG GGG GAC CTG

ATC CAG GTG ACA GGA CCG GGG GGC ACG AGG AAA CTG ACT

CCA AAC AAG TGT GTC AAT ACC GAT ATC CAG ATG AGG GGG

GCA ACA GAC GAC TTT TCT TAT CTC AAC CAT CTC ATC ACC

AAC ATG GCT CAA AGA ACC GAG TGC CTA GAT GCC CAT AGT

GAT ATC ACC GCT TCT GGG AAA GTA TCC TCA TTT CTC CTC

TCA AAG TTT CGT CCC AGC CAC CCT GGA CCT GGC AAG GCA

CAC TAT CTT CTC GAC GGT CAA ATC ATG CGA GGT GAC TGT

GAC TAT GAG GCA GTA GTC AGC ATC AAC TAC AAT CGC GCT

CAA TAC AAG ACG ATG AAC AAC ACA TGG AAA TCA TGG AAA

CGG GTA GAC AAC AAC ACA GAC GGG TAC GAT GGG ATG ATA

TTT GGG GAC AAA TTG ATC ATC CCG GAC ATC GAA AAG TAT

CAG AGT GTC TAT GAC AGT GGA ATG CTC GTT CAA AGA AAC

CTT GTG GAA GTC CCT CAT CTG AGC ATT GTG TTT GTC TCC

AAC ACA TCT GAT CTT TCC ACT AAT CAC ATC CAC ACC AAC

CTA ATC CCT TCG GAT
```

**17.** Verfahren nach einem der Ansprüche 13, 14 oder 15 zur Herstellung einer Nucleinsäure, die die gesamte nachstehende Abfolge von Nucleotiden (3) oder einen Teil der Abfolge umfaßt:

```
CAG ATC ACT CAA CGA CCT CCG GTC GAA AAC ATC TCG ACG

TAC CAT GCA GAC TGG GAC ACT CCG CTA TAC ACT CAT CCC

TCC AAC TGC AGG GAC GAT TCC TTT GTC CCG ATT CGA CCA

GCT CAA CTC AGG TGT CCT CAT GAA TTT GAA GAC ATA AAC
```

```
AAG GGA CTG GTT TCC GTC CCA ACC AGG ATC ATC CAT CTC
CCG CTA TCG GTC ACC AGC GTC TCC GCA GTA GCG AGT GGC
CAC TAC CTG CAC AGA GTG ACT TAT CGA GTC ACC TGT TCG
ACC AGC TTC TTT GGA GGG CAA ACC ATC GAA AAG ACC ATC
TTG GAG GCG AAA CTG TCT CGT CAG GAG GCC ACA GAC GAG
GCA AGC AAG GAT CAC CAG TAC CCG TTC TTC CCT GAA CCC
TCC TGC ATC TGG ATG AAA AAC AAT GTC CAT AAG GAC ATA
ACT CAC TAT TAC AAG ACC CCA AAA ACA GTA TCG GTG GAT
CTC TAC AGC AGG AAA TTT CTC AAC CCT GAT TTC ATA GAG
GGG GTT TGC ACA ACC TCG CCC TGT CAA ACT CAT TGG CAG
GGA GTC TAT TGG GTC GGT GCC ACA CCT AAA GCC CAT TGC
CCC ACG TCG GAA ACA CTA GAA GGA CAC CTG TTC ACC AGG
ACC CAT GAT CAC AGG GTG GTC AAG GCA ATT GTG GCA GGC
CAT CAT CCC TGG GGA CTC ACA ATG GCA TGC ACA GTG ACA
TTC TGC GGG ACA GAA TGG ATC AAG ACT GAC CTG GGG GAC
CTG ATC CAG GTG ACA GGA CCG GGG GGC ACG AGG AAA CTG
ACT CCA AAC AAG TGT GTC AAT ACC GAT ATC CAG ATG AGG
GGG GCA ACA GAC GAC TTT TCT TAT CTC AAC CAT CTC ATC
ACC AAC ATG GCT CAA AGA ACC GAG TGC CTA GAT GCC CAT
AGT GAT ATC ACC GCT TCT GGG AAA GTA TCC TCA TTT CTC
CTC TCA AAG TTT CGT CCC AGC CAC CCT GGA CCT GGC AAG
GCA CAC TAT CTT CTC GAC GGT CAA ATC ATG CGA GGT GAC
TGT GAC TAT GAG GCA GTA GTC AGC ATC AAC TAC AAT CGC
GCT CAA TAC AAG ACG ATG AAC AAC ACA TGG AAA TCA TGG
AAA CGG GTA GAC AAC AAC ACA GAC GGG TAC GAT GGG ATG
ATA TTT GGG GAC AAA TTG ATC ATC CCG GAC ATC GAA AAG
TAT CAG AGT GTC TAT GAC AGT GGA ATG CTC GTT CAA AGA
AAC CTT GTG GAA GTC CCT CAT CTG AGC ATT GTG TTT GTC
TCC AAC ACA TCT GAT CTT TCC ACT AAT CAC ATC CAC ACC
AAC CTA ATC CCT TCG GAT
```

18. Verfahren nach einem der Ansprüche 13, 14 oder 15 zur Herstellung einer Nucleinsäure, die die gesamte nachstehende Abfolge von Nucleotiden (4) oder einen Teil der Abfolge umfaßt:

```
ATG CGA GGT GAC TGT GAC TAT GAG GCA GTA GTC AGC ATC
AAC TAC AAT CGC GCT CAA TAC AAG ACG ATG AAC AAC ACA
TGG AAA TCA TGG AAA CGG GTA GAC AAC AAC ACA GAC GGG
TAC GAT GGG ATG ATA TTT GGG GAC AAA TTG ATC ATC CCG
GAC ATC GAA AAG TAT CAG AGT GTC TAT GAC AGT GGA ATG
CTC GTT CAA AGA AAC CTT GTG GAA GTC CCT CAT
```

19. Verfahren nach einem der Ansprüche 13, 14 oder 15 zur Herstellung einer Nucleinsäure, die die gesamte nachstehende Abfolge von Nucleotiden (5) oder einen Teil der Abfolge umfaßt:

```
TCT TTG GAG AAA CCA GTG AGA AGA TTG ACT TCG GGA CTC
GCA GTA AGA CCC TGA TAA CAA GCT TCA AGA TAG CTG AAG
CAA AGG CCA TCT ACC TGG ATT CAA GTC CGG TGA GAT CTC
GCA TAG AGG CCA AGA AGT ACA CCA CTC CCA TTA GAC ATG
GGA GTG TGA CTT ACT ATG GTC CGT TCA TAT TTG CAG ATG
ACC ACG TTG GAG GGA AAG GCC ACC GCG AGA AGC TAG GGG
CAC TAT GTG GAT TCC TCC AGT CTG GAC CTT ACG GGC AGG
CGA AGG ACT ACT ACA ATC GTG CCG TCG AAG AAG AGA TAG
GCA TTC CCC CAA GGG ACC CGA AGC GCA GAT CTG GAA CCT
CCT CTG TCC GAC CTT GGT AGA CCG AAA GGA CTG ACC CAG
GTT TGA GAC CAC ACT CTC ATT AGA TAG AAA AAA ATG GCA
CAT TTG TGT ACA CAA CAA GCT AGA CCC ACA
```

20. Verfahren nach einem der Ansprüche 13, 14 oder 15 zur Herstellung einer Nucleinsäure, die die gesamte nachstehende Abfolge (6) von Nucleotiden oder einen Teil der Abfolge umfaßt:

```
                TGG TCA TTC AAC TGG AGT CTT TGG CCA TCA TTA
TCT GGG ATG GGG GTT GTG GGA GGG GCC TTC CTT CTA CTG
GTA CTC TGC TGT TGC TGC AAG GCG TCC CCT CCC ATT CCA
AAT TAC GGG ATT CCG ATG CAG CAG TTC TCC AGA AGT CAG
ACG GTC TGA GCA CAC CTG TCC GAA TGA CCA CAA TTC CTC
TCT TAG GTA GAT AGA AAA AAA AAA AAA AAA AAA AAA AAA
A
```

21. Verfahren zur Herstellung von Antikörpern, umfassend die Immunisierung eines Tiers mit Hilfe eines Polypeptids, das in einem der Ansprüche 1 bis 13 definiert ist, und die Gewinnung der gebildeten Antikörper.

**22.** Verfahren zur Herstellung einer Nucleinsäuresonde mit der nachstehenden Sequenz:

ACG TTG GAG GGA AAG GCC
GAC TGG GAC ACT CCG CTA
CCC TCC TGC ATC TGG ATG
AAG ACT GAC CTG GGG GAC
GAC AAC AAC ACA GAC GGG
TTC CTT CTA CTG GTA CTC

dadurch gekennzeichnet, daß man die Synthese von DNA unter Anwendung der automatisierten β-Cyanoethylphosphoramidit-Methode, die in Bioorganic Chemistry, Bd. 4 (1986), S. 274-325, beschrieben ist, durchführt.

**23.** Verfahren zum Nachweis einer Nucleinsäuresequenz, die in einem der Ansprüche 13 bis 19 definiert ist, dadurch gekennzeichnet, daß man

- mit einer der Sonden nach Anspruch 22 eine Stufe der Vorhybridisierung der gewünschten RNA, die zuvor zugänglich gemacht worden ist, unter den folgenden Bedingungen durchführt: eine Behandlung von 16 Stunden bei 56°C in einem Medium zur Vorhybridisierung, das den folgenden Puffer enthält: 3 m Tetramethylammoniumchlorid, 50 millimolar Tris-HCl, pH-Wert: 8,0, 2 millimolar EDTA, 100 μg/ml beschallte denaturierte Kalbsthymus-DNA, 5X Denhardt-Lösung;
- eine Hybridisierung unter den folgenden Bedingungen durchführt: 1-stündige Behandlung bei der gleichen Temperatur mit etwa 100 pg einer der Sonden oder eines Gemisches der Sonden, die in Anspruch 22 definiert sind,
- anschließend unter den folgenden Bedingungen wäscht:
  . 5 Minuten in 5X SSC, 0,1 % SDS bei Umgebungstemperatur;
  . 5 Minuten in 5X SSC, 0,1 % SDS bei 59°C;
  . 2 Durchgänge im vorstehend angegebenen Hybridisierungspuffer ohne Denhardt-Lösung und ohne Kalbsthymus-DNA für 1 Stunde bei 59°C.

**24.** Verfahren zur Herstellung eines Polypeptids, das in einem der Ansprüche 1 bis 12 definiert ist, wobei das Verfahren folgende Stufen umfaßt:

- Kultur eines kompetenten zellulären Wirts, der vorher mit einer Nucleinsäure transformiert wurde, die eine Nucleotidsequenz enthält, die für die in einem der Ansprüche 1 bis 21 definierten Polypeptide codiert und in der diese Nucleotidsequenz sich unter der Steuerung von Regulationselementen, insbesondere von einem Promotor, der von den Polymerasen des kompetenten zellulären Wirts erkannt wird, sowie von Initiations-und Terminationselementen der Transkription, befindet, und
- Gewinnung des Polypeptidprodukts ausgehend von Expressionsprodukten des zellulären Wirts.

**25.** Verfahren nach Anspruch 24 zur Herstellung eines bestimmten rekombinanten Polypeptids, dadurch gekennzeichnet, daß man mit dem Vektor, der in einer der für seine Replikation nicht essentiellen Stellen die erforderlichen Elemente zur Förderung der Expression einer Aminosäuresequenz, die in einem der Ansprüche 1 bis 12 definiert ist, in dem zellulären Wirt und gegebenenfalls einen Promotor, der von den Polymerasen des zellulären Wirts erkannt wird, insbesondere einen induzierbaren Promotor, und gegebenenfalls eine Signalsequenz und/oder eine Ankersequenz und insbesondere

- die Elemente, die Expression des gesamten Gens für β-Galactosidase oder eines Teils davon erlauben,

enthält, transformierte E. coli züchtet und
die Kultur am Ende der exponentiellen Wachstumsphase der E. coli arretiert

- und die bestimmte Aminosäuresequenz ausgehend von dem Kulturmedium frei von anderen extrazellulären Proteinen und Enzymen, die von E. coli abgesondert werden können, gewinnt.

**26.** Verfahren nach Anspruch 24 zur Herstellung eines bestimmten Polypeptids, dadurch gekennzeichnet, daß man durch den Vektor, der die Elemente enthält, die das Einführen von beliebigen Nucleinsäuren, wie sie in den Ansprüchen 13 bis 19 definiert sind, in Hefe und die Expression dieser Nucleinsäure in der Hefe erlauben, transformierte Hefe züchtet und die Kultur am Ende der exponentiellen Wachstumsphase der Hefe arretiert und das in der Hefe in Mikroeinschlüssen vorliegende Polypeptid gewinnt.

**27.** Verfahren nach einem der Ansprüche 1, 24, 25 oder 26 zur Herstellung eines Polypeptids, das aus der zwischen den Resten 111 bis 302 definierten Aminosäuresequenz aufgebaut ist, die in der in Fig. 3 gezeigten Aminosäuresequenz enthalten ist.

28. Verfahren nach einem der Ansprüche 13, 14 oder 15 zur Herstellung einer Nucleinsäure, insbesondere eines DNA-Fragments, die für das Polypeptid codiert, das aus der zwischen den Resten 111 bis 302 definierten Aminosäuresequenz aufgebaut ist, die in der in Fig. 3 gezeigten Aminosäuresequenz enthalten ist.

29. Verfahren zur Herstellung von monoclonalen Antikörpern, umfassend das Züchten von Hybridomen, die ausgehend von den Polypeptiden hergestellt wurden, die durch das Verfahren nach einem der Ansprüche 1 bis 12 oder 24 bis 27 erhalten wurden.

**Patentansprüche für folgenden Vertragsstaat : GR**

1. Rekombinantes Polypeptid, dadurch gekennzeichnet, daß in seinem Polypeptidgerüst eine fortlaufende Abfolge (I) von Aminosäuren mit der nachstehenden Sequenz vorhanden ist:

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-.
```

163

```
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

und daß natürliche Verunreinigungen, die vom Virus der viralen hämorrhagischen Sepsis stammen, fehlen.

**2.** Polypeptid nach Anspruch 1, dadurch gekennzeichnet, daß es durch neutralisierende Antikörper erkannt wird, die zuvor gegen das Virus der SHV gebildet wurden.

**3.** Rekombinantes Polypeptid nach Anspruch 2, dadurch gekennzeichnet, daß es geeignet ist, in vivo neutralisierende Antikörper gegen das Virus der SHV zu induzieren.

**4.** Rekombinantes Polypeptid nach einem der Ansprüche 1 bis 3, dadurch gekennzeichnet, daß in seinem Peptidgerüst die nachstehende Abfolge (II) von Aminosäuren vorhanden ist:

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
```

Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp

und daß natürliche Verunreinigungen, die vom Virus der viralen hämorrhagischen Sepsis stammen, fehlen.

5. Polypeptid nach einem der Ansprüche 1 bis 3, dadurch gekennzeichnet, daß es die gesamte nachstehende Abfolge (III) von Aminosäuren oder einen Teil der Abfolge enthält:

Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His

6. Polypeptid nach einem der Ansprüche 1 bis 3, dadurch gekennzeichnet, daß es die gesamte Abfolge der nachstehenden Aminosäuren oder einen Teil der Abfolge enthält:

```
Trp-Ser-Phe-Asn-Trp-Ser-Leu-Trp-Pro-Ser-Leu-Ser-Gly-
Met-Gly-Val-Val-Gly-Gly-Ala-Phe-Leu-Leu-Leu-Val-Leu-
Cys-Cys-Cys-Cys-Lys-Ala-Ser-Pro-Pro-Ile-Pro-Asn-Tyr-
Gly-Ile-Pro-Met-Gln-Gln-Phe-Ser-Arg-Ser-Gln-Thr-Val
```

7. Polypeptid nach einem der Ansprüche 1 bis 3, dadurch gekennzeichnet, daß es aus der nachstehenden Abfolge von Aminosäuren I aufgebaut ist:

```
Met-Glu-Trp-Asn-Thr-Phe-Phe-Leu-Val-Ile-Leu-Ile-Ile-
Ile-Ile-Lys-Ser-Thr-Thr-Pro-Gln-Ile-Thr-Gln-Arg-Pro-
Pro-Val-Glu-Asn-Ile-Ser-Thr-Tyr-His-Ala-Asp-Trp-Asp-
Thr-Pro-Leu-Tyr-Thr-His-Pro-Ser-Asn-Cys-Arg-Asp-Asp-
Ser-Phe-Val-Pro-Ile-Arg-Pro-Ala-Gln-Leu-Arg-Cys-Pro-
His-Glu-Phe-Glu-Asp-Ile-Asn-Lys-Gly-Leu-Val-Ser-Val-
Pro-Thr-Arg-Ile-Ile-His-Leu-Pro-Leu-Ser-Val-Thr-Ser-
Val-Ser-Ala-Val-Ala-Ser-Gly-His-Tyr-Leu-His-Arg-Val-
Thr-Tyr-Arg-Val-Thr-Cys-Ser-Thr-Ser-Phe-Phe-Gly-Gly-
Gln-Thr-Ile-Glu-Lys-Thr-Ile-Leu-Glu-Ala-Lys-Leu-Ser-
Arg-Gln-Glu-Ala-Thr-Asp-Glu-Ala-Ser-Lys-Asp-His-Glu-
Tyr-Pro-Phe-Phe-Pro-Glu-Pro-Ser-Cys-Ile-Trp-Met-Lys-
Asn-Asn-Val-His-Lys-Asp-Ile-Thr-His-Tyr-Tyr-Lys-Thr-
Pro-Lys-Thr-Val-Ser-Val-Asp-Leu-Tyr-Ser-Arg-Lys-Phe-
Leu-Asn-Pro-Asp-Phe-Ile-Glu-Gly-Val-Cys-Thr-Thr-Ser-
Pro-Cys-Gln-Thr-His-Trp-Gln-Gly-Val-Tyr-Trp-Val-Gly-
Ala-Thr-Pro-Lys-Ala-His-Cys-Pro-Thr-Ser-Glu-Thr-Leu-
Glu-Gly-His-Leu-Phe-Thr-Arg-Thr-His-Asp-His-Arg-Val-
Val-Lys-Ala-Ile-Val-Ala-Gly-His-His-Pro-Trp-Gly-Leu-
Thr-Met-Ala-Cys-Thr-Val-Thr-Phe-Cys-Gly-Thr-Glu-Trp-
Ile-Lys-Thr-Asp-Leu-Gly-Asp-Leu-Ile-Gln-Val-Thr-Gly-
Pro-Gly-Gly-Thr-Arg-Lys-Leu-Thr-Pro-Asn-Lys-Cys-Val-
Asn-Thr-Asp-Ile-Gln-Met-Arg-Gly-Ala-Thr-Asp-Asp-Phe-
Ser-Tyr-Leu-Asn-His-Leu-Ile-Thr-Asn-Met-Ala-Gln-Arg-
Thr-Glu-Cys-Leu-Asp-Ala-His-Ser-Asp-Ile-Thr-Ala-Ser-
Gly-Lys-Val-Ser-Ser-Phe-Leu-Leu-Ser-Lys-Phe-Arg-Pro-
Ser-His-Pro-Gly-Pro-Gly-Lys-Ala-His-Tyr-Leu-Leu-Asp-
Gly-Gln-Ile-Met-Arg-Gly-Asp-Cys-Asp-Tyr-Glu-Ala-Val-
Val-Ser-Ile-Asn-Tyr-Asn-Arg-Ala-Gln-Tyr-Lys-Thr-Met-
```

```
Asn-Asn-Thr-Trp-Lys-Ser-Trp-Lys-Arg-Val-Asp-Asn-Asn-
Thr-Asp-Gly-Tyr-Asp-Gly-Met-Ile-Phe-Gly-Asp-Lys-Leu-
Ile-Ile-Pro-Asp-Ile-Glu-Lys-Tyr-Gln-Ser-Val-Tyr-Asp-
Ser-Gly-Met-Leu-Val-Gln-Arg-Asn-Leu-Val-Glu-Val-Pro-
His-Leu-Ser-Ile-Val-Phe-Val-Ser-Asn-Thr-Ser-Asp-Leu-
Ser-Thr-Asn-His-Ile-His-Thr-Asn-Leu-Ile-Pro-Ser-Asp
```

8. Polypeptid nach einem der Ansprüche 1 bis 3, dadurch gekennzeichnet, daß es aus der nachstehenden Abfolge (II) von Aminosäuren aufgebaut ist:

```
Gln-Ile-Thr-Gln-Arg-Pro-Pro-Val-Glu-Asn-Ile-Ser-Thr-
Tyr-His-Ala-Asp-Trp-Asp-Thr-Pro-Leu-Tyr-Thr-His-Pro-
Ser-Asn-Cys-Arg-Asp-Asp-Ser-Phe-Val-Pro-Ile-Arg-Pro-
Ala-Gln-Leu-Arg-Cys-Pro-His-Glu-Phe-Glu-Asp-Ile-Asn-
Lys-Gly-Leu-Val-Ser-Val-Pro-Thr-Arg-Ile-Ile-His-Leu-
Pro-Leu-Ser-Val-Thr-Ser-Val-Ser-Ala-Val-Ala-Ser-Gly-
His-Tyr-Leu-His-Arg-Val-Thr-Tyr-Arg-Val-Thr-Cys-Ser-
Thr-Ser-Phe-Phe-Gly-Gly-Gln-Thr-Ile-Glu-Lys-Thr-Ile-
Leu-Glu-Ala-Lys-Leu-Ser-Arg-Gln-Glu-Ala-Thr-Asp-Glu-
Ala-Ser-Lys-Asp-His-Glu-Tyr-Pro-Phe-Phe-Pro-Glu-Pro-
Ser-Cys-Ile-Trp-Met-Lys-Asn-Asn-Val-His-Lys-Asp-Ile-
Thr-His-Tyr-Tyr-Lys-Thr-Pro-Lys-Thr-Val-Ser-Val-Asp-
Leu-Tyr-Ser-Arg-Lys-Phe-Leu-Asn-Pro-Asp-Phe-Ile-Glu-
Gly-Val-Cys-Thr-Thr-Ser-Pro-Cys-Gln-Thr-His-Trp-Gln-
Gly-Val-Tyr-Trp-Val-Gly-Ala-Thr-Pro-Lys-Ala-His-Cys-
Pro-Thr-Ser-Glu-Thr-Leu-Glu-Gly-His-Leu-Phe-Thr-Arg-
Thr-His-Asp-His-Arg-Val-Val-Lys-Ala-Ile-Val-Ala-Gly-
His-His-Pro-Trp-Gly-Leu-Thr-Met-Ala-Cys-Thr-Val-Thr-
Phe-Cys-Gly-Thr-Glu-Trp-Ile-Lys-Thr-Asp-Leu-Gly-Asp-
Leu-Ile-Gln-Val-Thr-Gly-Pro-Gly-Gly-Thr-Arg-Lys-Leu-
Thr-Pro-Asn-Lys-Cys-Val-Asn-Thr-Asp-Ile-Gln-Met-Arg-
Gly-Ala-Thr-Asp-Asp-Phe-Ser-Tyr-Leu-Asn-His-Leu-Ile-
Thr-Asn-Met-Ala-Gln-Arg-Thr-Glu-Cys-Leu-Asp-Ala-His-
Ser-Asp-Ile-Thr-Ala-Ser-Gly-Lys-Val-Ser-Ser-Phe-Leu-
Leu-Ser-Lys-Phe-Arg-Pro-Ser-His-Pro-Gly-Pro-Gly-Lys-
Ala-His-Tyr-Leu-Leu-Asp-Gly-Gln-Ile-Met-Arg-Gly-Asp-
Cys-Asp-Tyr-Glu-Ala-Val-Val-Ser-Ile-Asn-Tyr-Asn-Arg-
```

167

```
Ala-Gln-Tyr-Lys-Thr-Met-Asn-Asn-Thr-Trp-Lys-Ser-Trp-
Lys-Arg-Val-Asp-Asn-Asn-Thr-Asp-Gly-Tyr-Asp-Gly-Met-
Ile-Phe-Gly-Asp-Lys-Leu-Ile-Ile-Pro-Asp-Ile-Glu-Lys-
Tyr-Gln-Ser-Val-Tyr-Asp-Ser-Gly-Met-Leu-Val-Gln-Arg-
Asn-Leu-Val-Glu-Val-Pro-His-Leu-Ser-Ile-Val-Phe-Val-
Ser-Asn-Thr-Ser-Asp-Leu-Ser-Thr-Asn-His-Ile-His-Thr-
Asn-Leu-Ile-Pro-Ser-Asp
```

**9.** Polypeptid nach einem der Ansprüche 1 bis 3, dadurch gekennzeichnet, daß es aus der nachstehenden Abfolge (III) von Aminosäuren aufgebaut ist:

```
Met Arg Gly Asp Cys Asp Tyr Glu Ala Val Val Ser Ile Asn
Tyr Asn Arg Ala Gln Tyr Lys Thr Met Asn Asn Thr Trp Lys
Ser Trp Lys Arg Val Asp Asn Asn Thr Asp Gly Tyr Asp Gly
Met Ile Phe Gly Asp Lys Leu Ile Ile Pro Asp Ile Glu Lys
Tyr Gln Ser Val Tyr Asp Ser Gly Met Leu Val Gln Arg Asn
Leu Val Glu Val Pro His
```

**10.** Polypeptid nach einem der Ansprüche 1 bis 3, dadurch gekennzeichnet, daß es aus der nachstehenden Sequenz von Aminosäuren IV aufgebaut ist:

```
Met-Glu-Trp-Asn-Thr-Phe-Phe-Leu-Val-Ile-Leu-Ile-Ile-
Ile-Ile-Lys-Ser-Thr-Thr-Pro
```

**11.** Aminosäuresequenz nach einem der Ansprüche 1 bis 9, dadurch gekennzeichnet, daß sie aus einer Abfolge von Aminosäuren gemäß einem der Ansprüche 1 bis 9 und einem Protein oder einer Aminosäuresequenz, die heterolog in bezug auf die Abfolge sind und etwa 8 bis etwa 800 Aminosäuren enthalten, aufgebaut ist.

**12.** Aminosäuresequenz nach Anspruch 11, dadurch gekennzeichnet, daß es sich bei der fremden Amino-säuresequenz um $\beta$-Galactosidase handelt.

**13.** Polypeptid, dadurch gekennzeichnet, daß ein Peptidgerüst vorhanden ist, das die gesamte Abfolge der Aminosäuren, die durch die nachstehende Nucleinsäure (1) codiert wird, oder einen Teil der Abfolge umfaßt:

```
TCT TTG GAG AAA CCA GTG AGA AGA TTG ACT TCG GGA CTC GCA
GTA AGA CCC TGA TAA CAA GCT TCA AGA TAG CTG AAG CAA AGG
```

168

```
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC GAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
```

```
ACA TGG AAA TCA TGG AAA CGG GTA GAC AAC AAC ACA GAC GGG

TAC GAT GGG ATG ATA TTT GGG GAC AAA TTG ATC ATC CCG GAC

ATC GAA AAG TAT CAG AGT GTC TAT GAC AGT GGA ATG CTC GTT

CAA AGA AAC CTT GTG GAA GTC CCT CAT CTG AGC ATT GTG TTT

GTC TCC AAC ACA TCT GAT CTT TCC ACT AAT CAC ATC CAC ACC

AAC CTA ATC CCT TCG GAT
```

**14.** Nucleinsäure, dadurch gekennzeichnet, daß sie eine Abfolge von Nucleotiden umfaßt, die für die Polypeptide nach einem der Ansprüche 1 bis 13 codiert.

**15.** Nucleinsäure nach Anspruch 14, dadurch gekennzeichnet, daß sie die gesamte nachstehende Abfolge von Nucleotiden (2) oder einen Teil der Abfolge umfaßt:

```
                                    ATG GAA TGG AAC ACT TTT TTC TTG GTG

ATC TTG ATC ATC ATC ATA AAG AGC ACC ACA CCA CAG ATC ACT

CAA CGA CCT CCG GTC GAA AAC ATC TCG ACG TAC CAT GCA GAC

TGG GAC ACT CCG CTA TAC ACT CAT CCC TCC AAC TGC AGG GAC

GAT TCC TTT GTC CCG ATT CGA CCA GCT CAA CTC AGG TGT CCT

CAT GAA TTT GAA GAC ATA AAC AAG GGA CTG GTT TCC GTC CCA

ACC AGG ATC ATC CAT CTC CCG CTA TCG GTC ACC AGC GTC TCC

GCA GTA GCG AGT GGC CAC TAC CTG CAC AGA GTG ACT TAT CGA

GTC ACC TGT TCG ACC AGC TTC TTT GGA GGG CAA ACC ATC GAA

AAG ACC ATC TTG GAG GCG AAA CTG TCT CGT CAG GAG GCC ACA

GAC GAG GCA AGC AAG GAT CAC GAG TAC CCG TTC TTC CCT GAA

CCC TCC TGC ATC TGG ATG AAA AAC AAT GTC CAT AAG GAC ATA

ACT CAC TAT TAC AAG ACC CCA AAA ACA GTA TCG GTG GAT CTC

TAC AGC AGG AAA TTT CTC AAC CCT GAT TTC ATA GAG GGG GTT

TGC ACA ACC TCG CCC TGT CAA ACT CAT TGG CAG GGA GTC TAT

TGG GTC GGT GCC ACA CCT AAA GCC CAT TGC CCC ACG TCG GAA

ACA CTA GAA GGA CAC CTG TTC ACC AGG ACC CAT GAT CAC AGG

GTG GTC AAG GCA ATT GTG GCA GGC CAT CAT CCC TGG GGA CTC

ACA ATG GCA TGC ACA GTG ACA TTC TGC GGG ACA GAA TGG ATC

AAG ACT GAC CTG GGG GAC CTG ATC CAG GTG ACA GGA CCG GGG

GGC ACG AGG AAA CTG ACT CCA AAC AAG TGT GTC AAT ACC GAT

ATC CAG ATG AGG GGG GCA ACA GAC GAC TTT TCT TAT CTC AAC

CAT CTC ATC ACC AAC ATG GCT CAA AGA ACC GAG TGC CTA GAT

GCC CAT AGT GAT ATC ACC GCT TCT GGG AAA GTA TCC TCA TTT
```

170

```
CTC CTC TCA AAG TTT CGT CCC AGC CAC CCT GGA CCT GGC AAG
GCA CAC TAT CTT CTC GAC GGT CAA ATC ATG CGA GGT GAC TGT
GAC TAT GAG GCA GTA GTC AGC ATC AAC TAC AAT CGC GCT CAA
TAC AAG ACG ATG AAC AAC ACA TGG AAA TCA TGG AAA CGG GTA
GAC AAC AAC ACA GAC GGG TAC GAT GGG ATG ATA TTT GGG GAC
AAA TTG ATC ATC CCG GAC ATC GAA AAG TAT CAG AGT GTC TAT
GAC AGT GGA ATG CTC GTT CAA AGA AAC CTT GTG GAA GTC CCT
CAT CTG AGC ATT GTG TTT GTC TCC AAC ACA TCT GAT CTT TCC
ACT AAT CAC ATC CAC ACC AAC CTA ATC CCT TCG GAT
```

16. Nucleinsäure, dadurch gekennzeichnet, daß sie die gesamte nachstehende Abfolge von Nucleotiden (3) oder einen Teil der Abfolge umfaßt:

CAG ATC ACT CAA CGA CCT CCG GTC GAA AAC ATC TCG ACG TAC

CAT GCA GAC TGG GAC ACT CCG CTA TAC ACT CAT CCC TCC AAC

TGC AGG GAC GAT TCC TTT GTC CCG ATT CGA CCA GCT CAA CTC

AGG TGT CCT CAT GAA TTT GAA GAC ATA AAC AAG GGA CTG GTT

TCC GTC CCA ACC AGG ATC ATC CAT CTC CCG CTA TCG GTC ACC

AGC GTC TCC GCA GTA GCG AGT GGC CAC TAC CTG CAC AGA GTG

ACT TAT CGA GTC ACC TGT TCG ACC AGC TTC TTT GGA GGG CAA

ACC ATC GAA AAG ACC ATC TTG GAG GCG AAA CTG TCT CGT CAG

GAG GCC ACA GAC GAG GCA AGC AAG GAT CAC CAG TAC CCG TTC

TTC CCT GAA CCC TCC TGC ATC TGG ATG AAA AAC AAT GTC CAT

AAG GAC ATA ACT CAC TAT TAC AAG ACC CCA AAA ACA GTA TCG

GTG GAT CTC TAC AGC AGG AAA TTT CTC AAC CCT GAT TTC ATA

GAG GGG GTT TGC ACA ACC TCG CCC TGT CAA ACT CAT TGG CAG

GGA GTC TAT TGG GTC GGT GCC ACA CCT AAA GCC CAT TGC CCC

ACG TCG GAA ACA CTA GAA GGA CAC CTG TTC ACC AGG ACC CAT

GAT CAC AGG GTG GTC AAG GCA ATT GTG GCA GGC CAT CAT CCC

TGG GGA CTC ACA ATG GCA TGC ACA GTG ACA TTC TGC GGG ACA

GAA TGG ATC AAG ACT GAC CTG GGG GAC CTG ATC CAG GTG ACA

GGA CCG GGG GGC ACG AGG AAA CTG ACT CCA AAC AAG TGT GTC

AAT ACC GAT ATC CAG ATG AGG GGG GCA ACA GAC GAC TTT TCT

TAT CTC AAC CAT CTC ATC ACC AAC ATG GCT CAA AGA ACC GAG

TGC CTA GAT GCC CAT AGT GAT ATC ACC GCT TCT GGG AAA GTA

TCC TCA TTT CTC CTC TCA AAG TTT CGT CCC AGC CAC CCT GGA

CCT GGC AAG GCA CAC TAT CTT CTC GAC GGT CAA ATC ATG CGA

GGT GAC TGT GAC TAT GAG GCA GTA GTC AGC ATC AAC TAC AAT


CGC GCT CAA TAC AAG ACG ATG AAC AAC ACA TGG AAA TCA TGG

AAA CGG GTA GAC AAC AAC ACA GAC GGG TAC GAT GGG ATG ATA

TTT GGG GAC AAA TTG ATC ATC CCG GAC ATC GAA AAG TAT CAG

AGT GTC TAT GAC AGT GGA ATG CTC GTT CAA AGA AAC CTT GTG

GAA GTC CCT CAT CTG AGC ATT GTG TTT GTC TCC AAC ACA TCT

GAT CTT TCC ACT AAT CAC ATC CAC ACC AAC CTA ATC CCT TCG

GAT

**17.** Nucleinsäure, dadurch gekennzeichnet, daß sie die gesamte nachstehende Abfolge von Nucleotiden (4) oder einen Teil der Abfolge umfaßt:

```
ATG CGA GGT GAC TGT GAC TAT GAG GCA GTA GTC AGC ATC AAC

TAC AAT CGC GCT CAA TAC AAG ACG ATG AAC AAC ACA TGG AAA

TCA TGG AAA CGG GTA GAC AAC AAC ACA GAC GGG TAC GAT GGG

ATG ATA TTT GGG GAC AAA TTG ATC ATC CCG GAC ATC GAA AAG

TAT CAG AGT GTC TAT GAC AGT GGA ATG CTC GTT CAA AGA AAC

CTT GTG GAA GTC CCT CAT
```

**18.** Nucleinsäure, dadurch gekennzeichnet, daß sie die gesamte nachstehende Abfolge von Nucleotiden (5) oder einen Teil der Abfolge umfaßt:

```
TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA
```

**19.** Nucleinsäure, dadurch gekennzeichnet, daß sie die gesamte nachstehende Abfolge von Nucleotiden (6) oder einen Teil der Abfolge umfaßt:

```
    TGG TCA TTC AAC TGG AGT CTT TGG CCA TCA TTA TCT

GGG ATG GGG GTT GTG GGA GGG GCC TTC CTT CTA CTG GTA CTC


TGC TGT TGC TGC AAG GCG TCC CCT CCC ATT CCA AAT TAC GGG

ATT CCG ATG CAG CAG TTC TCC AGA AGT CAG ACG GTC TGA GCA

CAC CTG TCC GAA TGA CCA CAA TTC CTC TCT TAG GTA GAT AGA

AAA AAA AAA AAA AAA AAA AAA AAA A
```

**20.** Nucleinsäure nach Anspruch 15, dadurch gekennzeichnet, daß ihr eine Nucleotidsequenz vorangeht, die Regulationselemente für die Expression der Nucleinsäure umfaßt.

**21.** Nucleinsäure nach einem der Ansprüche 15 oder 20, dadurch gekennzeichnet, daß sie Terminationselemente für die Transkription der Nucleinsäure umfaßt.

**22.** Nucleinsäure, dadurch gekennzeichnet, daß sie die gesamte nachstehende Abfolge von Nucleotiden (1) oder einen Teil der Abfolge umfaßt:

```
TCT TTG GAG AAA CCA GTG AGA AGA TTG ACT TCG GGA CTC GCA

GTA AGA CCC TGA TAA CAA GCT TCA AGA TAG CTG AAG CAA AGG

CCA TCT ACC TGG ATT CAA GTC CGG TGA GAT CTC GCA TAG AGG

CCA AGA AGT ACA CCA CTC CCA TTA GAC ATG GGA GTG TGA CTT

ACT ATG GTC CGT TCA TAT TTG CAG ATG ACC ACG TTG GAG GGA

AAG GCC ACC GCG AGA AGC TAG GGG CAC TAT GTG GAT TCC TCC

AGT CTG GAC CTT ACG GGC AGG CGA AGG ACT ACT ACA ATC GTG

CCG TCG AAG AAG AGA TAG GCA TTC CCC CAA GGG ACC CGA AGC

GCA GAT CTG GAA CCT CCT CTG TCC GAC CTT GGT AGA CCG AAA

GGA CTG ACC CAG GTT TGA GAC CAC ACT CTC ATT AGA TAG AAA

AAA ATG GCA CAT TTG TGT ACA CAA CAA GCT AGA CCC ACA ATG

GAA TGG AAC ACT TTT TTC TTG GTG ATC TTG ATC ATC ATC ATA

AAG AGC ACC ACA CCA CAG ATC ACT CAA CGA CCT CCG GTC GAA

AAC ATC TCG ACG TAC CAT GCA GAC TGG GAC ACT CCG CTA TAC

ACT CAT CCC TCC AAC TGC AGG GAC GAT TCC TTT GTC CCG ATT

CGA CCA GCT CAA CTC AGG TGT CCT CAT GAA TTT GAA GAC ATA

AAC AAG GGA CTG GTT TCC GTC CCA ACC AGG ATC ATC CAT CTC

CCG CTA TCG GTC ACC AGC GTC TCC GCA GTA GCG AGT GGC CAC

TAC CTG CAC AGA GTG ACT TAT CGA GTC ACC TGT TCG ACC AGC

TTC TTT GGA GGG CAA ACC ATC GAA AAG ACC ATC TTG GAG GCG

AAA CTG TCT CGT CAG GAG GCC ACA GAC GAG GCA AGC AAG GAT

CAC CAG TAC CCG TTC TTC CCT GAA CCC TCC TGC ATC TGG ATG
```

174

```
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

23. Nucleinsäure nach Anspruch 22, dadurch gekennzeichnet, daß sie aus der nachstehenden Abfolge (1) von Nucleotiden aufgebaut ist:

```
TCT TTG GAG AAA CCA GTG AGA AGA TTG ACT TCG GGA CTC GCA
GTA AGA CCC TGA TAA CAA GCT TCA AGA TAG CTG AAG CAA AGG
CCA TCT ACC TGG ATT CAA GTC CGG TGA GAT CTC GCA TAG AGG
CCA AGA AGT ACA CCA CTC CCA TTA GAC ATG GGA GTG TGA CTT
ACT ATG GTC CGT TCA TAT TTG CAG ATG ACC ACG TTG GAG GGA
AAG GCC ACC GCG AGA AGC TAG GGG CAC TAT GTG GAT TCC TCC
AGT CTG GAC CTT ACG GGC AGG CGA AGG ACT ACT ACA ATC GTG
CCG TCG AAG AAG AGA TAG GCA TTC CCC CAA GGG ACC CGA AGC
GCA GAT CTG GAA CCT CCT CTG TCC GAC CTT GGT AGA CCG AAA
GGA CTG ACC CAG GTT TGA GAC CAC ACT CTC ATT AGA TAG AAA
AAA ATG GCA CAT TTG TGT ACA CAA CAA GCT AGA CCC ACA ATG
GAA TGG AAC ACT TTT TTC TTG GTG ATC TTG ATC ATC ATC ATA
AAG AGC ACC ACA CCA CAG ATC ACT CAA CGA CCT CCG GTC GAA
```

```
AAC ATC TCG ACG TAC CAT GCA GAC TGG GAC ACT CCG CTA TAC
ACT CAT CCC TCC AAC TGC AGG GAC GAT TCC TTT GTC CCG ATT
CGA CCA GCT CAA CTC AGG TGT CCT CAT GAA TTT GAA GAC ATA
AAC AAG GGA CTG GTT TCC GTC CCA ACC AGG ATC ATC CAT CTC
CCG CTA TCG GTC ACC AGC GTC TCC GCA GTA GCG AGT GGC CAC
TAC CTG CAC AGA GTG ACT TAT CGA GTC ACC TGT TCG ACC AGC
TTC TTT GGA GGG CAA ACC ATC GAA AAG ACC ATC TTG GAG GCG
AAA CTG TCT CGT CAG GAG GCC ACA GAC GAG GCA AGC AAG GAT
CAC CAG TAC CCG TTC TTC CCT GAA CCC TCC TGC ATC TGG ATG
AAA AAC AAT GTC CAT AAG GAC ATA ACT CAC TAT TAC AAG ACC
CCA AAA ACA GTA TCG GTG GAT CTC TAC AGC AGG AAA TTT CTC
AAC CCT GAT TTC ATA GAG GGG GTT TGC ACA ACC TCG CCC TGT
CAA ACT CAT TGG CAG GGA GTC TAT TGG GTC GGT GCC ACA CCT
AAA GCC CAT TGC CCC ACG TCG GAA ACA CTA GAA GGA CAC CTG
TTC ACC AGG ACC CAT GAT CAC AGG GTG GTC AAG GCA ATT GTG
GCA GGC CAT CAT CCC TGG GGA CTC ACA ATG GCA TGC ACA GTG
ACA TTC TGC GGG ACA GAA TGG ATC AAG ACT GAC CTG GGG GAC
CTG ATC CAG GTG ACA GGA CCG GGG GGC ACG AGG AAA CTG ACT
CCA AAC AAG TGT GTC AAT ACC GAT ATC CAG ATG AGG GGG GCA
ACA GAC GAC TTT TCT TAT CTC AAC CAT CTC ATC ACC AAC ATG
GCT CAA AGA ACC GAG TGC CTA GAT GCC CAT AGT GAT ATC ACC
GCT TCT GGG AAA GTA TCC TCA TTT CTC CTC TCA AAG TTT CGT
CCC AGC CAC CCT GGA CCT GGC AAG GCA CAC TAT CTT CTC GAC
GGT CAA ATC ATG CGA GGT GAC TGT GAC TAT GAG GCA GTA GTC
AGC ATC AAC TAC AAT CGC GCT CAA TAC AAG ACG ATG AAC AAC
ACA TGG AAA TCA TGG AAA CGG GTA GAC AAC AAC ACA GAC GGG
TAC GAT GGG ATG ATA TTT GGG GAC AAA TTG ATC ATC CCG GAC
ATC GAA AAG TAT CAG AGT GTC TAT GAC AGT GGA ATG CTC GTT
CAA AGA AAC CTT GTG GAA GTC CCT CAT CTG AGC ATT GTG TTT
GTC TCC AAC ACA TCT GAT CTT TCC ACT AAT CAC ATC CAC ACC
AAC CTA ATC CCT TCG GAT
```

**24.** Rekombinante Nucleinsäure, dadurch gekennzeichnet, daß sie die Nucleinsäure nach einem der Ansprüche 14 bis 23 inseriert in eine gegenüber dem vorstehenden Fragment heterologe Nucleinsäure enthält.

**25.** Rekombinanter Vektor, insbesondere für die Clonierung und/oder die Expression, insbesondere vom Typ eines Plasmids, eines Cosmids oder eines Phagen, dadurch gekennzeichnet, daß er eine rekombinante Nucleinsäure nach einem der Ansprüche 14 bis 23 an einer der für seine Replikation

177

nicht essentiellen Stellen enthält.

26. Rekombinanter Vektor nach Anspruch 25, dadurch gekennzeichnet, daß es sich um das Plasmid PSHV-G1 handelt, das bei der CNCM unter der Nummer I 778 hinterlegt wurde.

27. Rekombinanter Vektor nach Anspruch 25, dadurch gekennzeichnet, daß er in einer der für seine Replikation nicht essentiellen Stellen die zur Förderung der Expression einer Aminosäuresequenz nach einem der Ansprüche 1 bis 13 in einem zellulären Wirt erforderlichen Elemente und gegebenenfalls einen Promotor, der von den Polymerasen des zellulären Wirts erkannt wird, und insbesondere einen induzierbaren Promotor sowie gegebenenfalls eine Signalsequenz und/oder eine Ankersequenz enthält.

28. Rekombinanter Vektor nach Anspruch 27, dadurch gekennzeichnet, daß er
   - die Elemente, die die Expression der Nucleinsäuresequenz nach einem der Ansprüche 11 bis 20, die in den Vektor für E. coli inseriert ist, erlauben und insbesondere
   - die Elemente, die die Expression des gesamten Gens für $\beta$-Galactosidaseoder eines Teils davon erlauben, enthält.

29. Rekombinanter Vektor nach Anspruch 27, dadurch gekennzeichnet, daß er die Elemente, die das Einführen der Nucleinsäure nach einem der Ansprüche 14 bis 23 in Hefe und die Expression der Nucleinsäure in der Hefe erlauben, enthält.

30. Durch einen rekombinanten Vektor nach einem der Ansprüche 25 bis 29 transformierter und die Regulationselemente, die die Expression der Nucleinsäuresequenz, die für das Polypeptid nach einem der Ansprüche 1 bis 13 codiert, in dem Wirt erlauben, umfassender zellulärer Wirt.

31. Zellulärer Wirt nach Anspruch 30, dadurch gekennzeichnet, daß es sich um den Wirt E. coli handelt, der durch den Vektor nach Anspruch 28 transformiert ist.

32. Zellulärer Wirt nach Anspruch 30, dadurch gekennzeichnet, daß es sich um Hefe handelt, die mit dem Vektor nach Anspruch 29 transformiert ist.

33. Expressionsprodukt einer durch den transformierten zellulären Wirt nach einem der Ansprüche 30 bis 32 exprimierten Nucleinsäure.

34. Nucleinsäuresonde, dadurch gekennzeichnet, daß sie mit den Nucleinsäuren nach einem der Ansprüche 14 bis 23 oder mit einer dazu komplementären Sequenz hybridisiert, und insbesondere Sonden, die unter den nachstehenden Nucleotidsequenzen oder den dazu komplementären Nucleotidsequenzen ausgewählt sind:
   ACG TTG GAG GGA AAG GCC
   GAC TGG GAC ACT CCG CTA
   CCC TCC TGC ATC TGG ATG
   AAG ACT GAC CTG GGG GAC
   GAC AAC AAC ACA GAC GGG
   TTC CTT CTA CTG GTA CTC

35. Verfahren zur Herstellung eines rekombinanten Polypeptids nach einem der Ansprüche 1 bis 13, dadurch gekennzeichnet, daß
   - man in einem geeigneten Kulturmedium einen zuvor mit einem geeigneten Vektor, der eine Nucleinsäure gemäß einem der Ansprüche 14 bis 23 enthält, transformierten Wirt züchtet und
   - ausgehend von dem vorstehend genannten Kulturmedium das Polypeptidprodukt durch den transformierten zellulären Wirt gewinnt.

36. Verfahren nach Anspruch 35 zur Herstellung eines bestimmten rekombinanten Polypeptids, dadurch gekennzeichnet, daß man E. coli nach Anspruch 31 züchtet und die Kultur am Ende der exponentiellen Wachstumsphase der E. coli arretiert und die bestimmte Aminesäuresequenz ausgehend vom Kulturmedium frei von anderen extrazellulären Proteinen und Enzymen, die von E. coli angesondert werden können, gewinnt.

**37.** Verfahren nach Anspruch 35 zur Herstellung eines bestimmten Polypeptids, dadurch gekennzeichnet, daß man die Hefe nach Anspruch 32 züchtet und die Kultur am Ende der exponentiellen Wachstumsphase der Hefe arretiert und das in der Hefe in Mikroeinschlüssen vorliegende Polypeptid gewinnt.

**38.** Polypeptid nach einem der Ansprüche 1 bis 3, dadurch gekennzeichnet, daß es aus der zwischen den Resten 111 bis 302 definierten Aminosäuresequenz, die in der in Fig. 3 dargestellten Aminosäuresequenz enthalten ist, aufgebaut ist.

**39.** Nucleinsäure, insbesondere Fragment von DNA, codierend für ein Polypeptid, wie es in Anspruch 38 definiert ist.

**40.** Monoclonaler Antikörper, hergestellt ausgehend von einem immunogenen rekombinanten Polypeptid nach einem der Ansprüche 1 bis 13 und dadurch gekennzeichnet, daß er gegen eines dieser Polypeptide gerichtet ist.

**41.** Monoclonaler Antikörper nach Anspruch 40, dadurch gekennzeichnet, daß er spezifisch das Polypeptid von Anspruch 38 erkennt.

FIG. I

Figure II

```
 1  Met Ala His Leu Cys Thr Gln Gln Ala Arg Pro Tyr Met Glu Trp Asn   16
 1  ATG GCA CAT TTG TGT ACA CAA CAA GCT AGA CCC ACA ATG GAA TGG AAC    48

17  Thr Phe Phe Leu Val Ile Leu Ile Ile Ile Ile Lys Ser Thr Thr Pro   32
49  ACT TTT TTC TTG GTG ATC TTG ATC ATC ATC ATA AAG AGC ACC ACA CCA    96

33  Gln Ile Thr Gln Arg Pro Pro Val Glu Asn Ile Ser Thr Tyr His Ala   48
97  CAG ATC ACT CAA CGA CCT CCG GTC GAA AAC ATC TCG ACG TAC CAT GCA   144

                                                     61
49  Asp Trp Asp Thr Pro Leu Tyr Thr His Pro Ser Asn Cys Arg Asp Asp   64
145 GAC TGG GAC ACT CCG CTA TAC ACT CAT CCC TCC AAC TGC AGG GAC GAT   192

65  Ser Phe Val Pro Asn Arg Pro Ala Gln Leu Arg Cys Pro His Glu Phe   80
193 TCC TTT GTC CCG AAT CGA CCA GCT CAA CTC AGG TGT CCT CAT GAA TTT   240

81  Glu Asp Ile Asn Lys Gly Leu Val Ser Val Pro Thr Arg Ile Ile His   96
241 GAA GAC ATA AAC AAG GGA CTG GTT TCC GTC CCA ACC AGG ATC ATC CAT   288

97  Leu Pro Leu Ser Val Thr Ser Val Ser Ala Val Ala Ser Gly His Tyr  112
289 CTC CCG CTA TCG GTC ACC AGC GTC TCC GCA GTA GCG AGT GGC CAC TAC   336

113 Leu His Arg Val Thr Tyr Arg Val Thr Cys Ser Thr Ser Phe Phe Gly  128
337 CTG CAC AGA GTG ACT TAT CGA GTC ACC TGT TCG ACC AGC TTC TTT GGA   384

129 Gly Gln Thr Ile Glu Lys Thr Ile Leu Glu Ala Lys Leu Ser Arg Gln  144
385 GGG CAA ACC ATC GAA AAG ACC ATC TTG GAG GCG AAA CTG TCT CGT CAG   432

145 Glu Ala Thr Asp Glu Ala Ser Lys Asp His Glu Tyr Pro Phe Phe Pro  160
433 GAG GCC ACA GAC GAG GCA AGC AAG GAT CAC GAG TAC CCG TTC TTC CCT   480

161 Glu Pro Ser Cys Ile Trp Met Lys Asn Asn Val His Lys Asp Ile Thr  176
481 GAA CCC TCC TGC ATC TGG ATG AAA AAC AAT GTC CAT AAG GAC ATA ACT   528

177 His Tyr Tyr Lys Thr Pro Lys Thr Val Ser Val Asp Leu Tyr Ser Arg  192
529 CAC TAT TAC AAG ACC CCA AAA ACA GTA TCG GTG GAT CTC TAC AGC AGG   576

193 Lys Phe Leu Asn Pro Asp Phe Ile Glu Gly Val Cys Thr Thr Ser Pro  208
577 AAA TTT CTC AAC CCT GAT TTC ATA GAG GGG GTT TGC ACA ACC TCG CCC   624

209 Cys Gln Thr His Trp Gln Gly Val Tyr Trp Val Gly Ala Thr Pro Lys  224
625 TGT CAA ACT CAT TGG CAG GGA GTC TAT TGG GTC GGT GCC ACA CCT AAA   672

225 Ala His Cys Pro Thr Ser Glu Thr Leu Glu Gly His Leu Phe Thr Arg  240
673 GCC CAT TGC CCC ACG TCG GAA ACA CTA GAA GGA CAC CTG TTC ACC AGG   720

241 Thr His Asp His Arg Val Val Lys Ala Ile Val Ala Gly His His Pro  256
721 ACC CAT GAT CAC AGG GTG GTC AAG GCA ATT GTG GCA GGC CAT CAT CCC   768
```

Figure III

182

```
257  Trp Gly Leu Thr Met Ala Cys Thr Val Thr Phe Cys Gly Thr Glu Trp   272
769  TGG GGA CTC ACA ATG GCA TGC ACA GTG ACA TTC TGC GGG ACA GAA TGG    816

273  Ile Lys Thr Asp Leu Gly Asp Leu Ile Gln Val Thr Gly Pro Gly Gly   288
817  ATC AAG ACT GAC CTG GGG GAC CTG ATC CAG GTG ACA GGA CCG GGG GGC    864

289  Thr Arg Lys Leu Thr Pro Asn Lys Cys Val Asn Thr Asp Ile Gln Met   304
865  ACG AGG AAA CTG ACT CCA AAC AAG TGT GTC AAT ACC GAT ATC CAG ATG    912

305  Arg Gly Ala Thr Asp Asp Phe Ser Tyr Leu Asn His Leu Ile Thr Asn   320
913  AGG GGG GCA ACA GAC GAC TTT TCT TAT CTC AAC CAT CTC ATC ACC AAC    960

321  Met Ala Gln Arg Thr Glu Cys Leu Asp Ala His Ser Asp Ile Thr Ala   336
961  ATG GCT CAA AGA ACC GAG TGC CTA GAT GCC CAT AGT GAT ATC ACC GCT   1008

337  Ser Gly Lys Val Ser Ser Phe Leu Leu Ser Lys Phe Arg Pro Ser His   352
1009 TCT GGG AAA GTA TCC TCA TTT CTC CTC TCA AAG TTT CGT CCC AGC CAC   1056

353  Pro Gly Pro Gly Lys Ala His Tyr Leu Leu Asp Gly Gln Ile Met Arg   368
1057 CCT GGA CCT GGC AAG GCA CAC TAT CTT CTC GAC GGT CAA ATC ATG CGA   1104

369  Gly Asp Cys Asp Tyr Glu Ala Val Val Ser Ile Asn Tyr Asn Arg Ala   384
1105 GGT GAC TGT GAC TAT GAG GCA GTA GTC AGC ATC AAC TAC AAT CGC GCT   1152

385  Gln Tyr Lys Thr Met Asn Asn Thr Trp Lys Ser Trp Lys Arg Val Asp   400
1153 CAA TAC AAG ACG ATG AAC AAC ACA TGG AAA TCA TGG AAA CGG GTA GAC   1200

401  Asn Asn Thr Asp Gly Tyr Asp Gly Met Ile Phe Gly Asp Lys Leu Ile   416
1201 AAC AAC ACA GAC GGG TAC GAT GGG ATG ATA TTT GGG GAC AAA TTG ATC   1248

417  Ile Pro Asp Ile Glu Lys Tyr Gln Ser Val Tyr Asp Ser Gly Met Leu   432
1249 ATC CCG GAC ATC GAA AAG TAT CAG AGT GTC TAT GAC AGT GGA ATG CTC   1296

433  Val Gln Arg Asn Leu Val Glu Val Pro His Leu Ser Ile Val Phe Val   448
1297 GTT CAA AGA AAC CTT GTG GAA GTC CCT CAT CTG AGC ATT GTG TTT GTC   1344

449  Ser Asn Thr Ser Asp Leu Ser Thr Asn His Ile His Thr Asn Leu Ile   464
1345 TCC AAC ACA TCT GAT CTT TCC ACT AAT CAC ATC CAC ACC AAC CTA ATC   1392

465  Pro Ser Asp Trp Ser Phe Asn Trp Ser Leu Trp Pro Ser Leu Ser Gly   480
1393 CCT TCG GAT TGG TCA TTC AAC TGG AGT CTT TGG CCA TCA TTA TCT GGG   1440

481  Met Gly Val Val Gly Gly Ala Phe Leu Leu Leu Val Leu Cys Cys Cys   496
1441 ATG GGG GTT GTG GGA GGG GCC TTC CTT CTA CTG GTA CTC TGC TGT TGC   1488

497  Cys Lys Ala Ser Pro Pro Ile Pro Asn Tyr Gly Ile Pro Met Gln Gln   512
1489 TGC AAG GCG TCC CCT CCC ATT CCA AAT TAC GGG ATT CCG ATG CAG CAG   1536

513  Phe Ser Arg Ser Gln Thr Val ***
1537 TTC TCC AGA AGT CAG ACG GTC TGA
```

FIGURE III SUITE

183

Figure IV

FIGURE 6

Figure V

FIG.VI

Figure VII

Figure VIII

## Elisa

**Figure IXa**

**Figure IXb**

# Epreuve par le virus de la SHV

jours apres l'epreuve

—□— PBS      —✕— SHVG27      —◇— virus tué

**solutions injectées intraperitonealement aux poissons**

Figure X

Figure XI

ELISA

anticorps monoclonal IIO

Figure XIIa

anticorps monoclonal ClO

Figure XIIb